# EUROPEAN PATENT APPLICATION

(11) **EP 4 752 140 A1**
(43) Date of publication of application: **03.06.2026**
(21) Application number: 24844844.1
(22) Date of filing: 25.07.2024
(51) Int. Cl.: C07D 401/06, C07D 403/06, C07D 487/10, C07D 401/14, A61K 31/454, A61K 31/444, A61K 31/404, A61P 13/12, A61P 27/02, A61P 7/06, A61P 19/02, A61P 9/00, A61P 11/00

(54) **SPIROCYCLICALKENYL OR AZAALKENYL COMPOUND, AND PHARMACEUTICAL COMPOSITION THEREOF, PREPARATION METHOD THEREFOR AND USE THEREOF**

(30) Priority: 26.07.2023 CN 202310925729; 06.11.2023 CN 202311463438; 27.06.2024 CN 202410847520; 18.07.2024 CN 202410971035
(71) Applicant: Shanghai Meiyue Biotech Development Co., Ltd., Shanghai 200120 (CN)
(72) Inventor: LUAN, Linbo, Shanghai 200120 (CN); HUANG, Nianfeng, Shanghai 200120 (CN); WANG, Chaodong, Shanghai 200120 (CN)
(74) Representative: IK-IP LTD
(86) International application number: PCT/CN2024/107570
(87) International publication number: WO 2025/021158

(57) **Abstract**

A spirocyclicalkenyl or azaalkenyl compound, and a pharmaceutical composition thereof, a preparation method therefor and the use thereof. Specifically, provided are a spirocyclicalkenyl or azaalkenyl compound as shown in formula (I), which can be used for preparing a drug, particularly for preparing a drug for preventing and/or treating a disease or condition by means of the alternative complement pathway. Each group in formula (I) is as defined in the description.

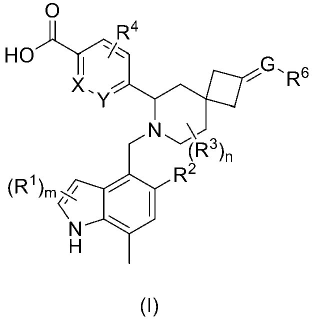

## Description

The present invention claims:
the priority of the prior application filed with the China National Intellectual Property Administration on July 26, 2023, with the patent application number of 202310925729.9, entitled "SPIROCYCLICALKENYL OR AZAALKENYL COMPOUND, AND PHARMACEUTICAL COMPOSITION THEREOF, PREPARATION METHOD THEREFOR AND USE THEREOF";
the priority of the prior application filed with the China National Intellectual Property Administration on November 06, 2023, with the patent application number of 202311463438.9, entitled "SPIROCYCLICALKENYL OR AZAALKENYL COMPOUND, AND PHARMACEUTICAL COMPOSITION THEREOF, PREPARATION METHOD THEREFOR AND USE THEREOF";
the priority of the prior application filed with the China National Intellectual Property Administration on June 27, 2024, with the patent application number of 202410847520.X, entitled "SPIROCYCLICALKENYL OR AZAALKENYL COMPOUND, AND PHARMACEUTICAL COMPOSITION THEREOF, PREPARATION METHOD THEREFOR AND USE THEREOF";
the priority of the prior application filed with the China National Intellectual Property Administration on July 18, 2024, with the patent application number of 202410971035.3, entitled "SPIROCYCLICALKENYL OR AZAALKENYL COMPOUND, AND PHARMACEUTICAL COMPOSITION THEREOF, PREPARATION METHOD THEREFOR AND USE THEREOF";
the contents of the above prior applications are incorporated herein by reference in their entireties.

### TECHNICAL FIELD

The present disclosure belongs to the field of medicine, and specifically relates to a spirocyclicalkenyl or azaalkenyl compound, a pharmaceutical composition thereof, a preparation method therefor, and a use thereof, which can be used as a complement factor B inhibitor.

### BACKGROUND

Complement is a class of soluble pattern recognition molecules in the immune system, capable of performing various effector functions. Under natural conditions, complement components exist as inactive zymogens. Various specific and non-specific immunological mechanisms cause these inactive zymogens to cleave, generating active large fragments and small fragments. Specifically, the large fragments typically remain on the surface of pathogens or cells, leading to their lysis or accelerating their clearance; the small fragments leave the cell surface and mediate various inflammatory responses. Complement activation consists of two closely linked processes, thereby forming a cascade of complement activation. Currently known complement activation pathways mainly include three types: the classical pathway, the lectin pathway, and the alternative pathway. Although the three complement activation pathways differ in initiation mechanisms and activation sequences, they share a common terminal pathway. The activation of the alternative pathway does not rely on antigen-antibody complexes. Typically, C3b deposited on the cell surface binds to factor B, becoming susceptible to cleavage by factor D in the serum. During the process, factor B is cleaved into Ba and Bb. Then, C3b and Bb form a complex, becoming the C3 convertase C3bBb of the alternative pathway. During the process, complement factor B plays an early and central role in the activation of the alternative pathway in the complement cascade. Here, C3b is both a product generated after C3 cleavage by C3 convertase and a component of C3 convertase in the alternative pathway, thereby forming a feedback amplification mechanism in which the classical and alternative pathways influence each other. Current research has found that various diseases, including hematological, autoimmune, inflammatory, and neurodegenerative diseases, are associated with complement system dysfunction.

Paroxysmal nocturnal hemoglobinuria (PNH) is a chronic disease characterized by persistent hemolysis. It is a non-malignant clonal disease caused by acquired somatic PIG-A gene mutations in one or several hematopoietic stem cells, and is classified as an extremely rare blood disorder (Medicine (Baltimore) 1997, 76(2): 63-93). The course of the disease can manifest as varying degrees of aggravated hemolysis (paroxysmal), chronic or recurrent acute intravascular hemolysis or subsequent venous/arterial thrombosis, ultimately leading to progressive end-organ damage and death. Typical PNH is characterized by chronic intravascular hemolysis, hemoglobinuria, and hemosiderinuria as the main manifestations. However, most patients often present atypically, with insidious onset, prolonged course, and varying degrees of severity.

There are more than ten proteins on the surface of red blood cells that inhibit complement pathway activation, all anchored to their cell membranes via glycosylphosphatidylinositol (GPI), collectively known as GPI-anchored proteins (APs). It is currently believed that the pathogenesis of PNH first involves hematopoietic stem cells undergoing mutations under certain conditions, producing GPI-deficient PNH clones. Then, due to certain factors (now mostly considered to be immune factors), hematopoietic function is impaired or fails, allowing the PNH clones to gain a proliferative advantage and outgrow the normal clones. The multiple GPI-linked antigens also contribute to the complexity of explaining the biological behavior of PNH cells. The most important proteins that inhibit complement pathway activation, CD55 (C3 convertase decay-accelerating factor) and CD59 (an inhibitor of the membrane attack complex (MAC)), are closely related to PNH in terms of pathogenesis, clinical manifestations, diagnosis, and treatment (Frontiers in Immunology 2019, 10, 1157). CD59 can prevent the incorporation of C9 into the C5b-8 complex, thereby preventing the formation of the membrane attack unit and inhibiting the terminal complement attack response. The typical manifestations of PNH - intravascular hemolysis and thrombosis - are currently believed to be caused by CD59 deficiency. Patients with congenital CD59 deficiency have been reported to exhibit many of the typical symptoms of PNH, such as intravascular hemolysis, hemoglobinuria, and venous thrombosis. In patients with PNH, due to GPI synthesis defects, CD59 cannot bind to the cell membrane of red blood cells, leading to the loss of its function to inhibit complement pathway activation. Consequently, abnormal activation of the complement pathway occurs and attacks red blood cells, resulting in various clinical manifestations such as intravascular hemolysis, hemoglobinuria, and smooth muscle dysfunction. Currently, apart from hematopoietic stem cell transplantation to restore normal hematopoietic function as a curative treatment for PNH, there are no other effective curative methods in clinical practice. Since hematopoietic stem cell transplantation carries certain risks and PNH is a benign clonal disease, controlling hemolytic episodes remains the main strategy for clinical treatment of the disease. Currently, only Eculizumab is approved for the treatment of PNH. However, many patients still develop anemia after treatment with Eculizumab, and many patients still require continuous blood transfusions. Furthermore, Eculizumab requires intravenous injection as a route of administration. Therefore, developing novel inhibitors of the complement pathway for the treatment of PNH is of great significance.

IgAN is one of the most common primary glomerulonephritides, characterized by mesangial IgA deposition as shown by immunofluorescence. Its clinical manifestations are diverse, typically presenting as recurrent microscopic or gross hematuria. Existing data indicate that the occurrence of IgAN is related to congenital or acquired immune dysregulation. Due to stimulation of the respiratory or digestive tract by viruses, bacteria, and food proteins, mucosal IgA1 synthesis increases, or IgA1-containing immune complexes are deposited in the mesangium, activating the alternative complement pathway and causing glomerular injury. Human IgA molecules are divided into two subtypes: IgA1 and IgA2, with IgA1 being the main form in the blood circulation of healthy individuals (accounting for approximately 85%) and also the main component deposited in the glomerular mesangium of patients with IgAN. IgA molecules can exist in two forms: monomeric and polymeric. The IgA1 molecule has a unique heavy chain hinge region between the first and second constant regions, which can serve as a domain for O-linked glycan attachment sites. Recent studies have found that the IgA molecules deposited in the serum and glomerular mesangium of patients with IgAN are mainly galactose-deficient IgA1 (gd-IgAl). The pathogenesis of IgAN is currently believed to be initiated by an abnormal increase in the production of gd-IgA1.

Over 90% of patients with IgAN have complement C3 deposition in the glomerular mesangium. 75% to 100% of patients with IgAN have co-deposition of properdin, IgA, and C3 in renal tissue, while 30% to 90% of patients with IgAN have co-deposition of complement factor H, IgA, and C3 in renal tissue. In addition to deposition in renal tissue, some studies have also found that plasma levels of markers of the alternative complement pathway in patients with IgAN are also related to the activity of IgAN (J Nephrol 2013, 26(4): 708-715). Studies have confirmed that C3a in renal tissue and urine, as well as C3a receptors in renal tissue, are significantly associated with the activity and severity of renal damage (J Clin Immunol 2014, 34(2): 224-232). Other studies have confirmed that IgA can activate the alternative complement pathway under *in vitro* conditions. During the process, abnormalities in the IgA hinge region do not play a decisive role, while the formation of IgA polymers is the key step (Eur J Immunol 1987, 17(3): 321-326). Currently, complement C3 deposition in the glomerular mesangium has become an auxiliary diagnostic marker for IgAN. A study performing immunofluorescence assays of C3c and C3d on renal tissues from 163 patients with IgAN showed that patients with higher C3c deposition intensity than C3d deposition intensity exhibited lower glomerular filtration rates, higher incidence of glomerular endocapillary proliferation, and more severe hematuria, indicating that glomerular C3c deposition is associated with active lesions in IgAN (Am J Nephrol. 2000, 20(2): 122-128). Currently, there are no specific drugs for the treatment of IgAN in clinical practice. The main treatments involve general-purpose drugs such as renin-angiotensin inhibitors (ACEI or ARB), glucocorticoids, and various immunosuppressants. Furthermore, the safety of such drugs is also an issue that cannot be ignored. For example, although glucocorticoids have the effect of reducing proteinuria, the STOP-IgAN and TESTING-I trials have clearly demonstrated the potential side effects of glucocorticoids (IgA nephropathy 2019, 95, 4, 750-756).

Arthritis is a common chronic disease, which is an inflammatory disorder caused by inflammation, infection, degeneration, trauma, or other factors. Its clinical manifestations include redness, swelling, heat, pain, dysfunction, and deformity of the joints. It often causes severe pain, limited mobility, and physical deformity, and can lead to disability in severe cases, affecting the quality of life in patients. Studies have found that serum from K/BxN mice could not induce arthritis in complement factor B-deficient mice, whereas wild-type mice developed arthritic when induced by serum from K/BxN mice (Immunity, 2002, 16, 157-168), indicating that the complement system plays an important pathogenic role in the K/BxN mouse serum-induced arthritis model, and complement factor B is a potential target for treating arthritis.

Novartis AG has developed small molecule complement factor B inhibitors for the treatment of diseases such as age-related macular degeneration (AMD). Related patents include: WO2013164802A1, WO2013192345Al, WO2014143638A1, WO2015009616A1, WO2015066241A1, and WO2019043609A1.

Inflammatory and immune-related diseases are diverse and difficult to cure. There are unmet clinical needs in these areas, necessitating the development of new small molecule drugs for medical treatment.

### SUMMARY

The present disclosure provides a compound represented by formula (IE) or a pharmaceutically acceptable salt thereof, 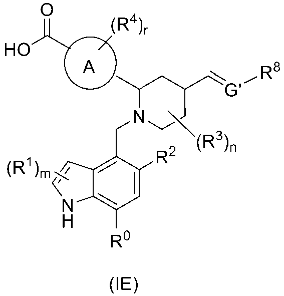 wherein
ring A is selected from the group consisting of phenyl, 5- to 10-membered heteroaryl, and 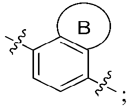
ring B is selected from the group consisting of 3- to 8-membered cycloalkyl, 3- to 8-membered heterocycloalkyl, phenyl, and 5- to 6-membered heteroaryl;
R⁰ is selected from the group consisting of H, halogen, CN, C₁₋₆ alkyl, C₁₋₆ haloalkyl, C₁₋₆ deuteroalkyl, C₁₋₆ hydroxyalkyl, and 3- to 8-membered cycloalkyl;
G' is CR⁷ or N;
each R¹ is the same or different, and independently selected from the group consisting of H, D, halogen, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
R² is selected from the group consisting of H, D, halogen, CN, C₁₋₆ alkyl, C₁₋₆ alkoxy, C₁₋₆ deuteroalkoxy, C₂₋₆ alkynyl, and 3- to 8-membered cycloalkyl;
each R³ is the same or different, and independently selected from the group consisting of H, D, halogen, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
each R⁴ is the same or different, and independently selected from the group consisting of H, halogen, OH, C₁₋₆ alkyl, C₁₋₆ haloalkyl, C₁₋₆ alkoxy, C₁₋₆ haloalkoxy, C₂₋₆ alkynyl, and 3- to 8-membered cycloalkyl;
R⁷ and R⁸ are the same or different, and each independently selected from the group consisting of H, halogen, CN, C₁₋₆ alkyl, C₁₋₆ haloalkyl, C₁₋₆ alkoxy, and C₁₋₆ haloalkoxy;
alternatively, R⁷ and R⁸ together with the carbon atom to which they are attached form a 3- to 8-membered cycloalkyl or 3- to 8-membered heterocycloalkyl ring, wherein the 3- to 8-membered cycloalkyl or 3- to 8-membered heterocycloalkyl is optionally substituted by one or more substituents selected from the group consisting of halogen, C₁₋₆ alkyl, C₁₋₆ haloalkyl, C₁₋₆ alkoxy, and C₁₋₆ haloalkoxy;
m is 0, 1, or 2;
n is 0, 1, 2, or 3;
r is 0, 1, 2, 3, or 4.

In some embodiments, in the compound represented by formula (IE) or the pharmaceutically acceptable salt thereof, R⁰ is selected from the group consisting of H, halogen, and C₁₋₆ alkyl.

In some embodiments, in the compound represented by formula (IE) or the pharmaceutically acceptable salt thereof, R⁰ is Cl or methyl.

In some embodiments, in the compound represented by formula (IE) or the pharmaceutically acceptable salt thereof, the compound represented by formula (IE) has a structure represented by formula (IG), 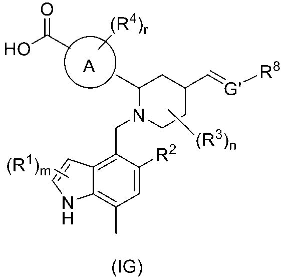 wherein ring A, G', R¹, R², R³, R⁴, R⁸, r, m, and n are as defined in formula (IE).

In some embodiments, in the compound represented by formula (IE) or formula (IG) or the pharmaceutically acceptable salt thereof, 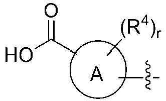 is selected from the group consisting of: 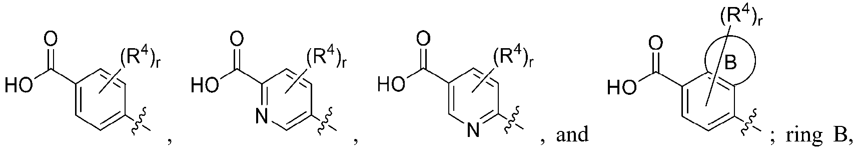 R⁴, and r are as defined in formula (IE) or formula (IG) described above.

In some embodiments, in the compound represented by formula (IE) or formula (IG) or the pharmaceutically acceptable salt thereof, 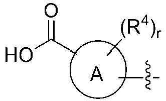 is selected from the group consisting of:

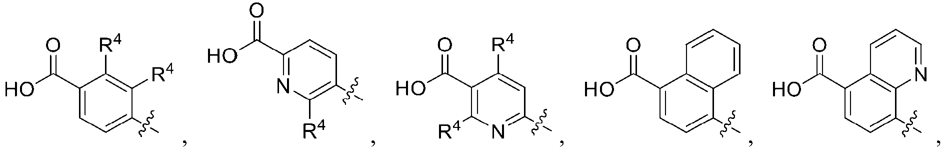

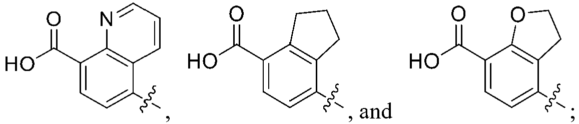

In some embodiments, in the compound represented by formula (IE) or formula (IG) or the pharmaceutically acceptable salt thereof, ring B is selected from the group consisting of 5- to 6-membered cycloalkyl, 5- to 6-membered heterocycloalkyl, phenyl, and 6-membered heteroaryl;

In some embodiments, in the compound represented by formula (IE) or formula (IG) or the pharmaceutically acceptable salt thereof, ring B is selected from the group consisting of phenyl, pyridinyl, cyclopentenyl, and dihydrofuranyl.

In some embodiments, in the compound represented by formula (IE) or formula (IG) or the pharmaceutically acceptable salt thereof, r is 0, 1, or 2.

In some embodiments, the compound represented by formula (IE) or formula (IG) has a structure represented by formula (IIG), 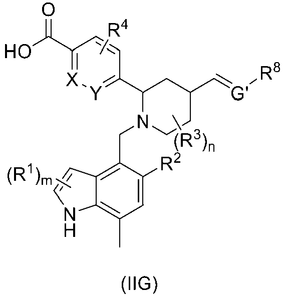 wherein
X and Y are the same or different, and each independently selected from the group consisting of CH and N;
G', R¹, R², R³, R⁴, R⁸, m, and n are as defined in formula (IE) or formula (IG).

In some embodiments, in the compound represented by formula (IIG) of the present disclosure or the pharmaceutically acceptable salt thereof,
G' is CR⁷ or N;
R⁷ and R⁸ are the same or different, and each independently selected from the group consisting of H, halogen, CN, C₁₋₆ alkyl, C₁₋₆ haloalkyl, C₁₋₆ alkoxy, and C₁₋₆ haloalkoxy;
X and Y are the same or different, and each independently selected from the group consisting of CH and N;
each R¹ is the same or different, and independently selected from the group consisting of H, D, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
R² is selected from the group consisting of C₁₋₆ alkyl, C₁₋₆ alkoxy, C₁₋₆ deuteroalkoxy, and 3-to 8-membered cycloalkyl;
each R³ is the same or different, and independently selected from the group consisting of H, D, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
each R⁴ is the same or different, and independently selected from the group consisting of H, halogen, C₁₋₆ alkyl, C₁₋₆ haloalkyl, C₁₋₆ alkoxy, and C₁₋₆ haloalkoxy;
m is 0, 1, or 2;
n is 0, 1, 2, or 3.

In some embodiments, in the compound represented by formula (IE), formula (IG), or formula (IIG) of the present disclosure or the pharmaceutically acceptable salt thereof, G' is CR⁷; R⁷ is as defined above.

In some embodiments, the compound represented by formula (IE), formula (IG), or formula (IIG) satisfies one or more of the following conditions:
(1) the C₁₋₆ alkyl is methyl, ethyl, n-propyl, isopropyl, n-butyl, isobutyl, sec-butyl, or *tert-*butyl;
(2) the halogen is fluorine, chlorine, bromine, or iodine, for example, fluorine;
(3) the 3- to 8-membered cycloalkyl is 3- to 6-membered cycloalkyl, for example, cyclopropyl, cyclobutyl, or cyclopentyl; for another example, cyclopentyl or cyclopropyl;
(4) the heteroatom in the 3- to 8-membered heterocycloalkyl is N, O, or S, for example, N or O; the number of heteroatoms may be 1, 2, or 3, for example, 1;
(5) the heteroatom in the 5- to 6-membered heteroaryl is N, O, or S, for example, N; the number of heteroatoms may be 1, 2, or 3, for example, 1;
(6) the C₁₋₆ alkoxy is C₁₋₄ alkoxy, for example, methoxy, ethoxy, n-propoxy, isopropoxy, n-butoxy, isobutoxy, sec-butoxy, or tert-butoxy; for another example, methoxy;
(7) the C₂₋₆ alkynyl is C₂₋₄ alkynyl, for example, ethynyl, propynyl, or butynyl.

The present disclosure also provides a compound represented by formula (I) or a pharmaceutically acceptable salt thereof, 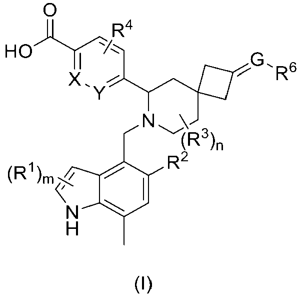 wherein
X and Y are the same or different, and each independently selected from the group consisting of CH and N;
G is CR⁵ or N;
R⁵ and R⁶ are the same or different, and each independently selected from the group consisting of H, halogen, CN, C₁₋₆ alkyl, C₁₋₆ haloalkyl, C₁₋₆ alkoxy, and C₁₋₆ haloalkoxy;
each R³ is the same or different, and independently selected from the group consisting of H, D, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, two R³ together with the carbon atom to which they are attached form a C₃₋₈ cycloalkyl or 3- to 8-membered heterocycloalkyl ring, wherein the C₃₋₈ cycloalkyl or 3- to 8-membered heterocycloalkyl is optionally substituted by halogen, C₁₋₆ alkyl, C₁₋₆ haloalkyl, C₁₋₆ alkoxy, and C₁₋₆ haloalkoxy;
R¹, R², R⁴, m, and n are as defined in any one of the schemes described above.

In some embodiments, in the compound represented by formula (I) or the pharmaceutically acceptable salt thereof, X and Y are the same or different, and each independently selected from the group consisting of CH and N; G is CR⁵ or N; each R¹ is the same or different, and independently selected from the group consisting of H, D, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl; R² is selected from the group consisting of C₁₋₆ alkyl, C₁₋₆ alkoxy, C₁₋₆ deuteroalkoxy, and 3- to 8-membered cycloalkyl; R³ is selected from the group consisting of H, D, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl; alternatively, two R³ together with the carbon atom to which they are attached form a C₃₋₈ cycloalkyl or 3- to 8-membered heterocycloalkyl ring, wherein the C₃₋₈ cycloalkyl or 3- to 8-membered heterocycloalkyl is optionally substituted by halogen, C₁₋₆ alkyl, C₁₋₆ haloalkyl, C₁₋₆ alkoxy, and C₁₋₆ haloalkoxy; R⁴ is selected from the group consisting of H, halogen, C₁₋₆ alkyl, C₁₋₆ haloalkyl, C₁₋₆ alkoxy, and C₁₋₆ haloalkoxy; R⁵ and R⁶ are the same or different, and each independently selected from the group consisting of H, halogen, CN, C₁₋₆ alkyl, C₁₋₆ haloalkyl, C₁₋₆ alkoxy, and C₁₋₆ haloalkoxy; m is 0, 1, or 2; n is 0, 1, 2, or 3.

In some embodiments, in the compound represented by formula (I) or the pharmaceutically acceptable salt thereof, G is CR⁵.

In some embodiments, in the compound represented by formula (I) or the pharmaceutically acceptable salt thereof, G is N.

In some embodiments, in the compound represented by formula (I) or the pharmaceutically acceptable salt thereof, both X and Y are CH.

In some embodiments, in the compound represented by formula (I) or the pharmaceutically acceptable salt thereof, X is N and Y is CH.

In some embodiments, in the compound represented by formula (I) or the pharmaceutically acceptable salt thereof, n is 0.

In some embodiments, in the compound represented by formula (I) or the pharmaceutically acceptable salt thereof, m is 1.

In some embodiments, in the compound represented by formula (I) or the pharmaceutically acceptable salt thereof, two R³ together with the carbon atom to which they are attached form a C₃₋₈ cycloalkyl or 3- to 8-membered heterocycloalkyl ring, wherein the C₃₋₈ cycloalkyl or 3- to 8-membered heterocycloalkyl is optionally substituted by halogen, C₁₋₆ alkyl, C₁₋₆ haloalkyl, C₁₋₆ alkoxy, and C₁₋₆ haloalkoxy.

In some embodiments, in the compound represented by formula (I) or the pharmaceutically acceptable salt thereof, two R³ together with the carbon atom to which they are attached form a C₃₋₈ cycloalkyl ring.

In some embodiments, in the compound represented by formula (I) or the pharmaceutically acceptable salt thereof, two R³ together with the carbon atom to which they are attached form a 3- to 8-membered heterocyclyl ring.

In some embodiments, the compound represented by formula (I) or the pharmaceutically acceptable salt thereof has a structure represented by formula (III): 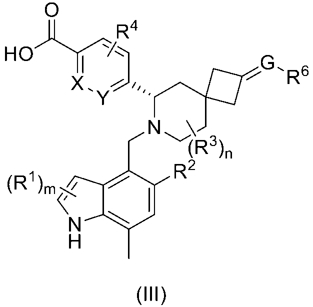 wherein X, Y, G, R¹, R², R³, R⁴, R⁶, m, and n are as defined in formula (I).

In some embodiments, the compound represented by formula (III) or the pharmaceutically acceptable salt thereof has a structure represented by formula (III-1) and formula (III-2): 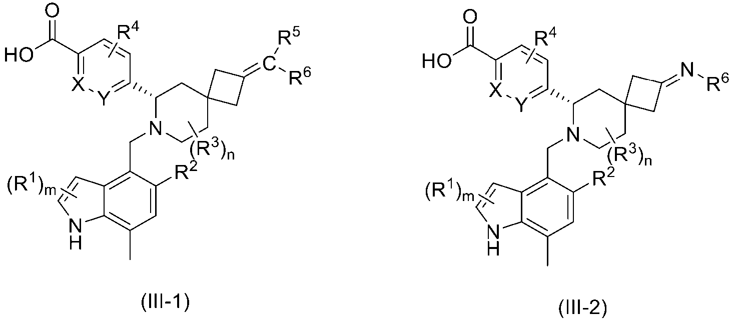 wherein X, Y, R¹, R², R³, R⁴, R⁵, R⁶, m, and n are as defined in formula (I).

In some embodiments, in the compound represented by formula (III-1) or the pharmaceutically acceptable salt thereof, R⁵ and R⁶ are the same or different, and each independently selected from the group consisting of H, halogen, CN, C₁₋₆ alkyl, and C₁₋₆ haloalkyl.

In some embodiments, in the compound represented by formula (III-1) or the pharmaceutically acceptable salt thereof, R⁵ and R⁶ are the same or different, and each independently selected from the group consisting of H, F, CN, and methyl.

In some embodiments, in the compound represented by formula (III-2) or the pharmaceutically acceptable salt thereof, R⁶ is C₁₋₆ alkoxy.

In some embodiments, in the compound represented by formula (III-2) or the pharmaceutically acceptable salt thereof, R⁶ is methoxy or ethoxy.

The present disclosure also provides a compound represented by formula (II) or a pharmaceutically acceptable salt thereof, 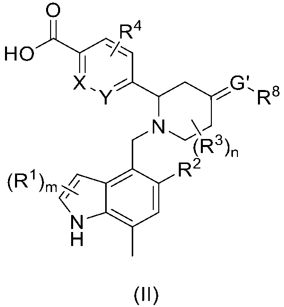 wherein
G' is CR⁷ or N;
R⁷ and R⁸ are the same or different, and each independently selected from the group consisting of H, halogen, CN, C₁₋₆ alkyl, C₁₋₆ haloalkyl, C₁₋₆ alkoxy, and C₁₋₆ haloalkoxy;
X, Y, R¹, R², R³, R⁴, m, and n are as defined in formula (I),
excluding

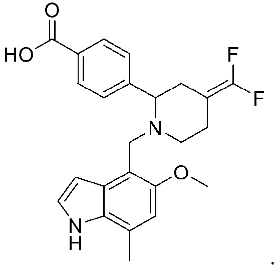

In some embodiments, the compound represented by formula (II) or the pharmaceutically acceptable salt thereof has a structure represented by formula (IV): 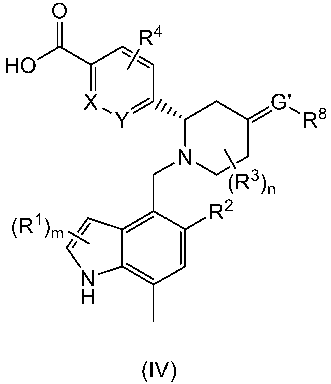 wherein X, Y, G', R¹, R², R³, R⁴, R⁸, m, and n are as defined in formula (II).

In some embodiments, the compound represented by formula (IV) or the pharmaceutically acceptable salt thereof has a structure represented by formula (IV-1) and formula (IV-2): 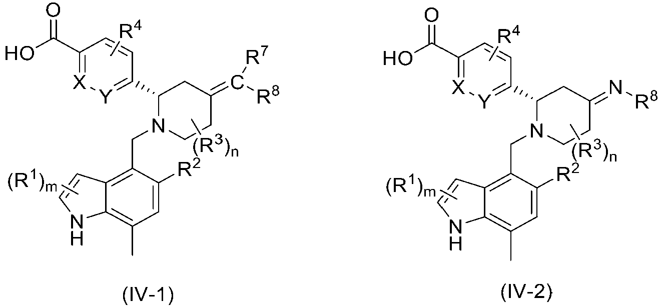 wherein X, Y, R¹, R², R³, R⁴, R⁷, R⁸, m, and n are as defined in formula (IV).

In some embodiments, in the compound represented by formula (IIG) or the pharmaceutically acceptable salt thereof, the compound represented by formula (IIG) has a structure represented by formula (IVG): 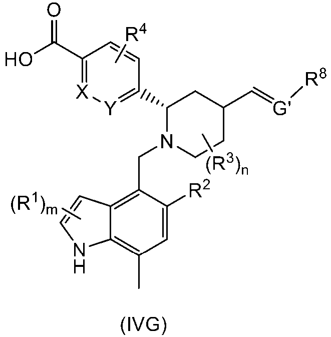 wherein X, Y, G', R¹, R², R³, R⁴, R⁸, m, and n are as defined in formula (IIG) described above.

In some embodiments, in the compound represented by formula (IVG) or the pharmaceutically acceptable salt thereof, the compound represented by formula (IVG) has a structure represented by formula (IVG-1) and formula (IVG-2): 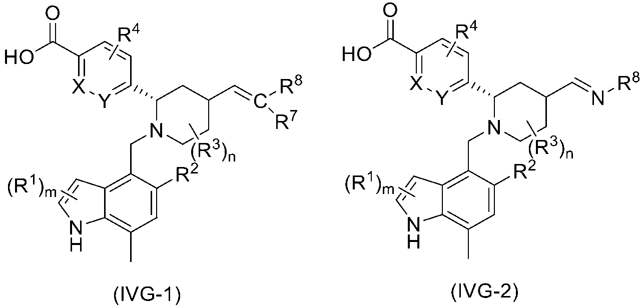 wherein X, Y, R¹, R², R³, R⁴, R⁷, R⁸, m, and n are as defined in formula (IVG).

In some embodiments, in the compound represented by formula (IIG) or the pharmaceutically acceptable salt thereof, the compound represented by formula (IIG) has a structure represented by formula (IVG-3): 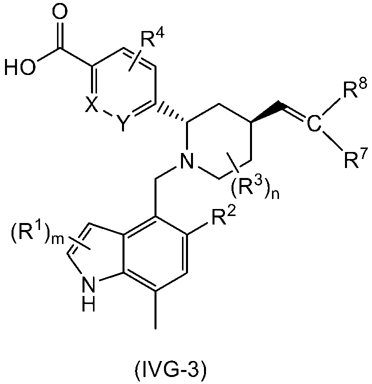 wherein X, Y, R¹, R², R³, R⁴, R⁷, R⁸, m, and n are as defined in formula (IIG) described above.

In some embodiments, in the compound represented by formula (IE), formula (IG), formula (IIG), formula (IVG), formula (II), formula (IV-1), formula (IVG-1), or formula (IVG-3) or the pharmaceutically acceptable salt thereof, R⁷ and R⁸ are the same or different, and each independently selected from the group consisting of H, halogen, CN, C₁₋₆ alkyl, and C₁₋₆ haloalkyl.

In some embodiments, in the compound represented by formula (IE), formula (IG), formula (IIG), formula (IVG), formula (II), formula (IV-1), formula (IVG-1), or formula (IVG-3) or the pharmaceutically acceptable salt thereof, R⁷ and R⁸ are the same or different, and each independently selected from the group consisting of H, F, CN, methyl, and -CHF₂.

In some embodiments, in the compound represented by formula (IE), formula (IG), formula (IIG), formula (IVG), formula (IVG-1), or formula (IVG-3) or the pharmaceutically acceptable salt thereof, R⁷ and R⁸ are H or F; alternatively, R⁷ and R⁸ together with the carbon atom to which they are attached form a cyclopropyl ring.

In some embodiments, in the compound represented by formula (IE), formula (IG), formula (IIG), formula (IVG), formula (IVG-1), or formula (IVG-3) or the pharmaceutically acceptable salt thereof, both R⁷ and R⁸ are F.

In some embodiments, in the compound represented by formula (IE), formula (IG), formula (IIG), formula (IVG), formula (II), formula (IVG-1), or formula (IVG-3) or the pharmaceutically acceptable salt thereof, R⁷ and R⁸ together with the carbon atom to which they are attached form a 3- to 6-membered cycloalkyl ring.

In some embodiments, in the compound represented by formula (IE), formula (IG), formula (IIG), formula (IVG), formula (II), formula (IVG-1), or formula (IVG-3) or the pharmaceutically acceptable salt thereof, R⁷ and R⁸ together with the carbon atom to which they are attached form a cyclopropyl ring.

In some embodiments, in the compound represented by formula (IV-2) and formula (IVG-2) or the pharmaceutically acceptable salt thereof, R⁸ is C₁₋₆ alkoxy.

In some embodiments, in the compound represented by formula (IV-2) and formula (IVG-2) or the pharmaceutically acceptable salt thereof, R⁸ is selected from the group consisting of methoxy and ethoxy.

In some embodiments, in the compound represented by formula (I), formula (II), formula (III), formula (III-1), or formula (III-2) or the pharmaceutically acceptable salt thereof, both X and Y are CH.

In some embodiments, in the compound represented by formula (I), formula (II), formula (III), formula (III-1), or formula (III-2) or the pharmaceutically acceptable salt thereof, X is N and Y is CH.

In some embodiments, in the compound represented by formula (I), formula (II), formula (III), formula (III-1), or formula (III-2) or the pharmaceutically acceptable salt thereof, X is CH and Y is N.

In some embodiments, in the compound represented by formula (I), formula (III), formula (III-1), or formula (III-2) or the pharmaceutically acceptable salt thereof, 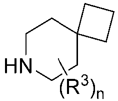 is

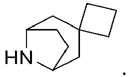

In some embodiments, in the compound represented by formula (IIG), formula (IVG), formula (IV), formula (IV-1), formula (IV-2), formula (IVG-1), formula (IVG-2), or formula (IVG-3) or the pharmaceutically acceptable salt thereof, both X and Y are CH.

In some embodiments, in the compound represented by formula (IIG), formula (IVG), formula (IV), formula (IV-1), formula (IV-2), formula (IVG-1), formula (IVG-2), or formula (IVG-3) or the pharmaceutically acceptable salt thereof, X is CH and Y is N.

In some embodiments, in the compound represented by formula (IIG), formula (IVG), formula (IV), formula (IV-1), formula (IV-2), formula (IVG-1), formula (IVG-2), or formula (IVG-3) or the pharmaceutically acceptable salt thereof, X is N and Y is CH.

In some embodiments, in the compound represented by formula (IE), formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, m is 1.

In some embodiments, in the compound represented by formula (IE), formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, n is 0.

In some embodiments, in the compound represented by formula (IE), formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, R¹ is H.

In some embodiments, in the compound represented by formula (IE), formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, R² is selected from the group consisting of Cl, methyl, methoxy, deuteromethoxy, ethynyl, and cyclopropyl.

In some embodiments, in the compound represented by formula (IE), formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, R² is selected from the group consisting of methyl, methoxy, deuteromethoxy, and cyclopropyl.

In some embodiments, in the compound represented by formula (IE), formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, R² is methyl.

In some embodiments, in the compound represented by formula (IE), formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, R² is methoxy or deuteromethoxy.

In some embodiments, in the compound represented by formula (IE), formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, R² is cyclopropyl.

In some embodiments, in the compound represented by formula (IE), formula (IG), formula (IIG), formula (IVG), formula (IVG-1), formula (IVG-2), or formula (IVG-3) or the pharmaceutically acceptable salt thereof, R³ is H, halogen, or C₁₋₆ alkyl.

In some embodiments, in the compound represented by formula (IE), formula (IG), formula (IIG), formula (IVG), formula (IVG-1), formula (IVG-2), or formula (IVG-3) or the pharmaceutically acceptable salt thereof, R³ is H or methyl.

In some embodiments, in the compound represented by formula (IE), formula (I), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, R⁴ is H, OH, halogen, C₁₋₆ alkyl, C₁₋₆ alkoxy, C₂₋₆ alkynyl, C₃₋₆ cycloalkyl, or C₁₋₆ haloalkyl.

In some embodiments, in the compound represented by formula (IE), formula (I), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, R⁴ is H, halogen, C₁₋₆ alkyl, or C₁₋₆ alkoxy.

In some embodiments, in the compound represented by formula (IE), formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, R⁴ is H, F, Cl, OH, methyl, methoxy, cyclopropyl, difluoromethyl, ethynyl, or propynyl.

In some embodiments, in the compound represented by formula (IE), formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3) or the pharmaceutically acceptable salt thereof, R⁴ is H, F, Cl, methyl, or methoxy.

In some embodiments, in the compound represented by formula (IE), formula (I), formula (IG), formula (II), formula (IIG), formula (III), formula (IV), formula (III-1), formula (III-2), formula (IV-1), formula (IV-2), formula (IVG), formula (IVG-1), formula (IVG-2), or formula (IVG-3) or the pharmaceutically acceptable salt thereof, n is 0 or 1.

In some embodiments, in the compound represented by formula (IE) or the pharmaceutically acceptable salt thereof, 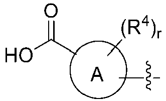 is 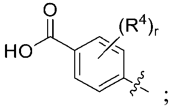
G' is CR⁷;
R⁷ and R⁸ are H or F;
alternatively, R⁷ and R⁸ together with the carbon atom to which they are attached form a cyclopropyl ring;
R⁰ is halogen or C₁₋₆ alkyl;
R¹ is H;
R² is selected from the group consisting of Cl, methyl, methoxy, deuteromethoxy, ethynyl, and cyclopropyl;
R³ is selected from the group consisting of H, halogen, and C₁₋₆ alkyl;
R⁴ is selected from the group consisting of H, OH, halogen, C₁₋₆ alkyl, C₁₋₆ alkoxy, C₂₋₆ alkynyl, C₃₋₆ cycloalkyl, and C₁₋₆ haloalkyl;
r is 0, 1, or 2;
m is 0 or 1;
n is 0, 1, or 2.

In some embodiments, in the compound represented by formula (IIG) or the pharmaceutically acceptable salt thereof,
both X and Y are CH;
G' is CR⁷;
both R⁷ and R⁸ are F;
R¹ is H;
R² is selected from the group consisting of methyl, methoxy, deuteromethoxy, and cyclopropyl;
R³ is H or methyl;
R⁴ is selected from the group consisting of H, halogen, C₁₋₆ alkyl, and C₁₋₆ alkoxy;
m is 0 or 1;
n is 0, 1, or 2.

Exemplary specific compounds for the compounds according to the present disclosure include, but are not limited to, the structures in Table A below:

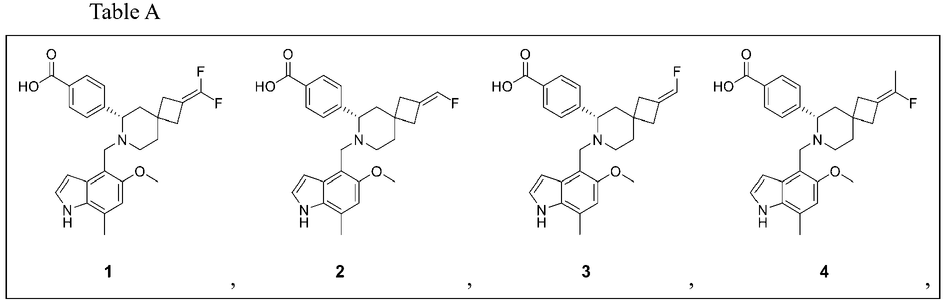

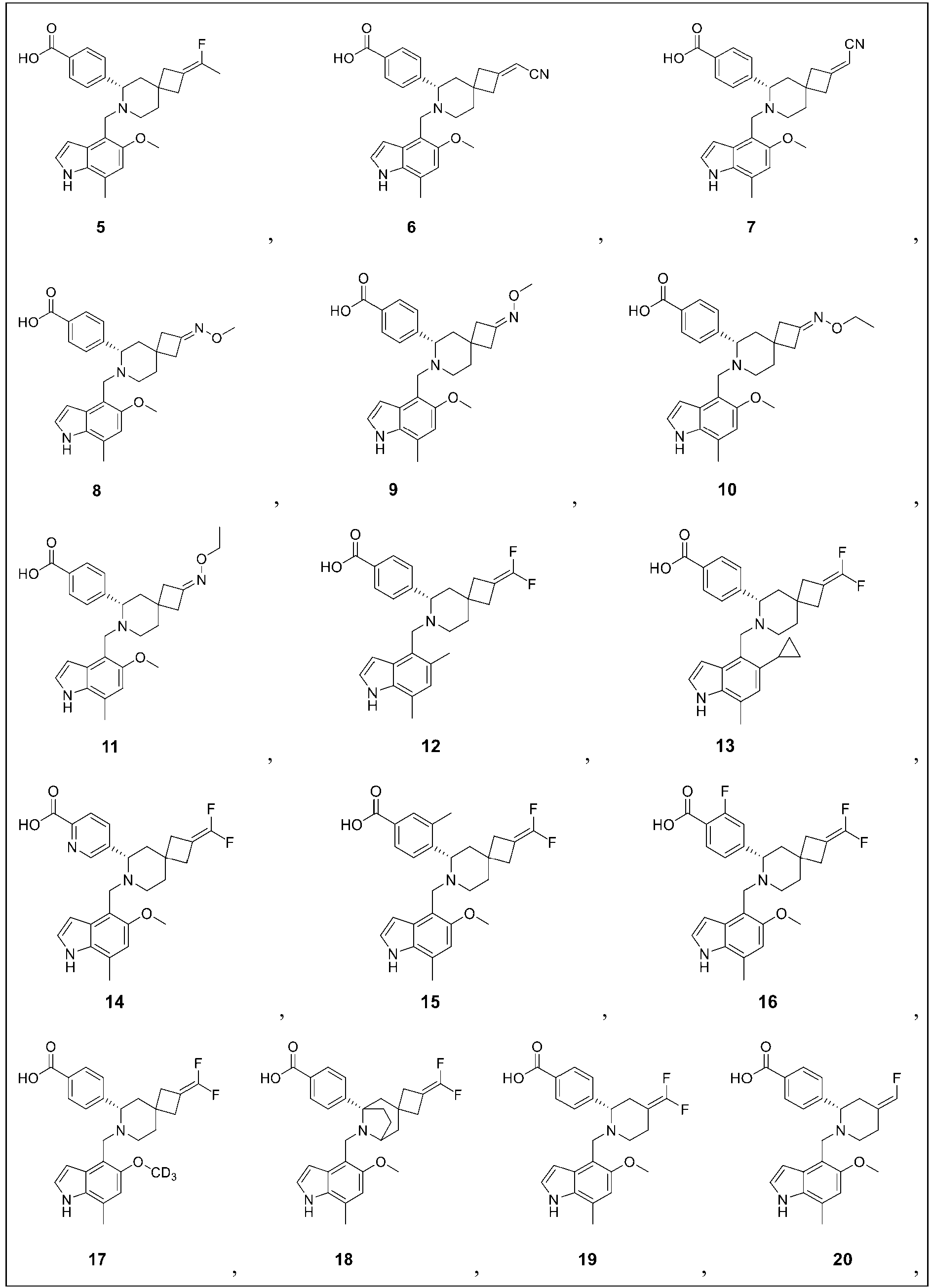

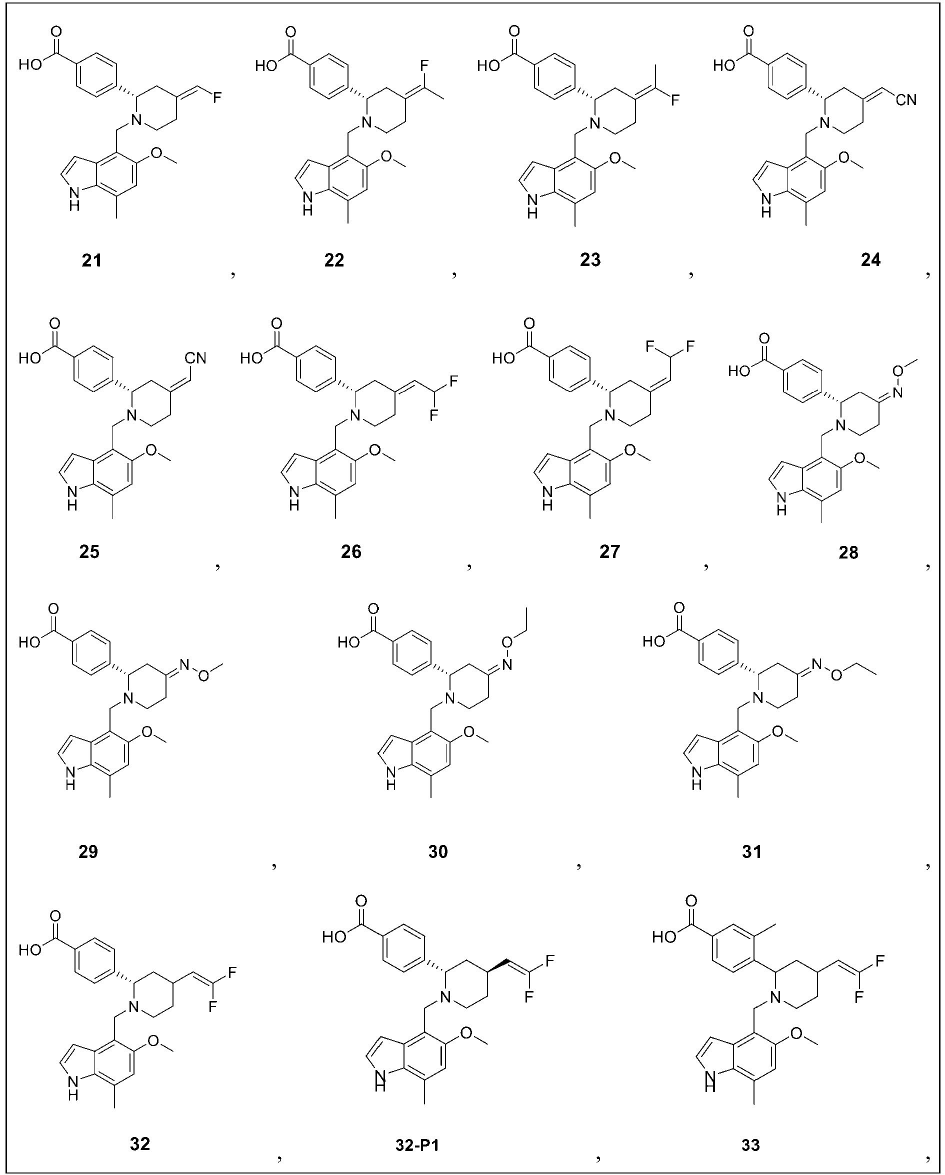

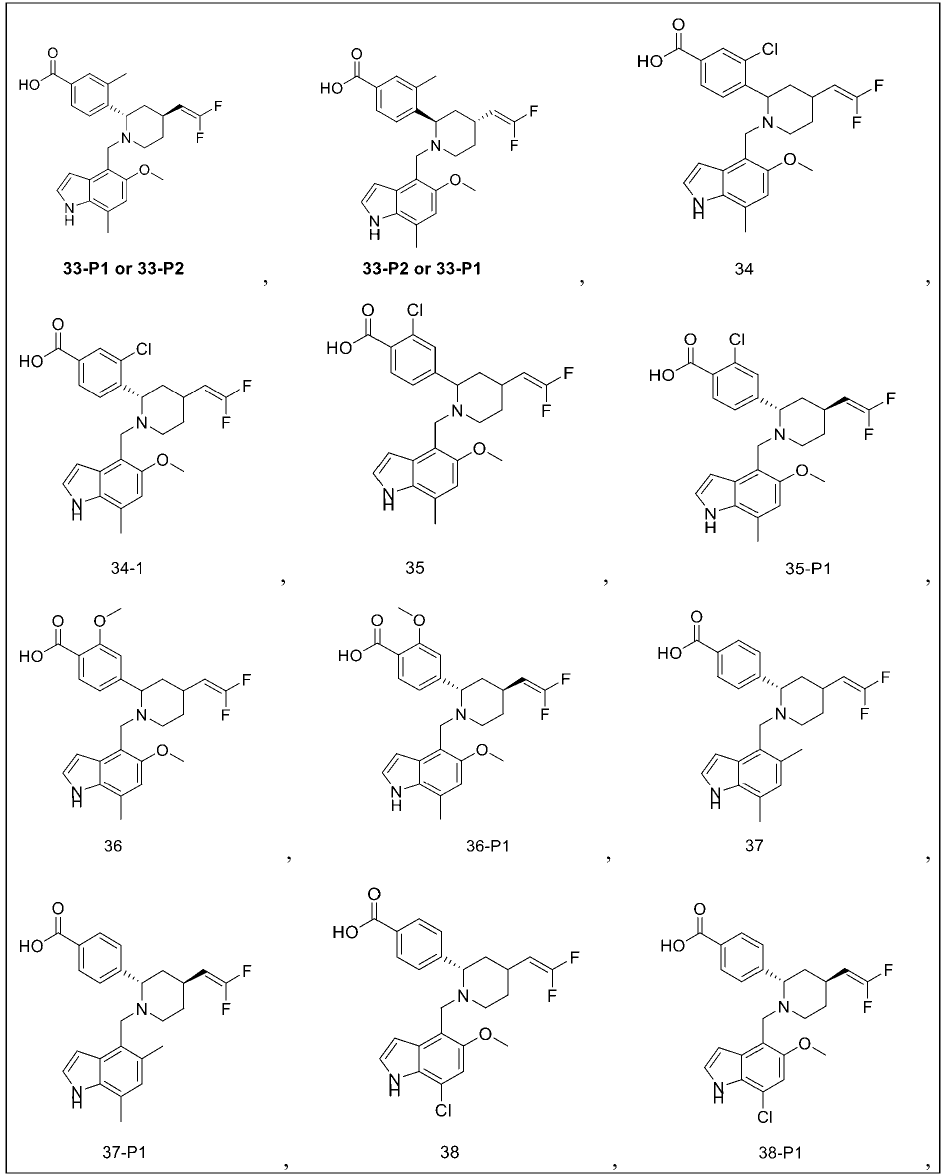

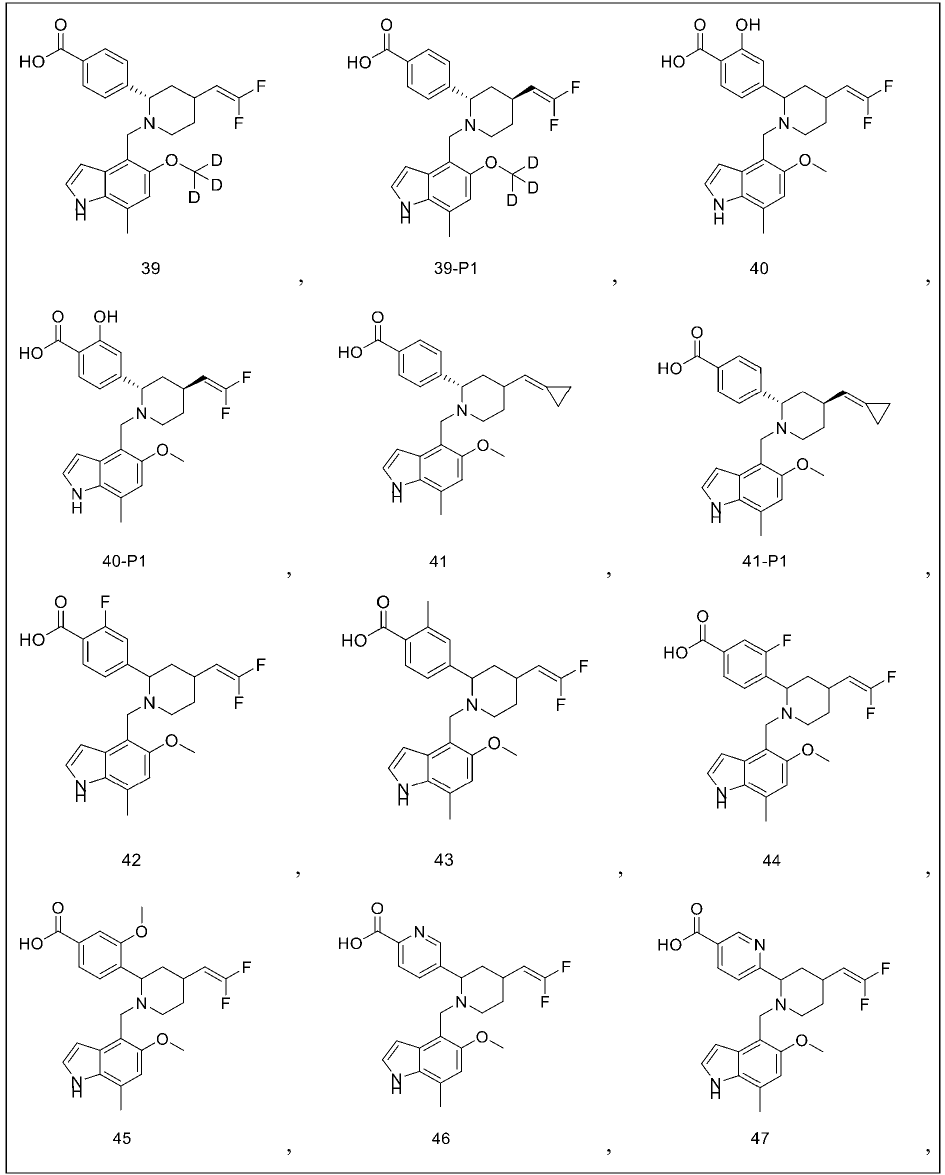

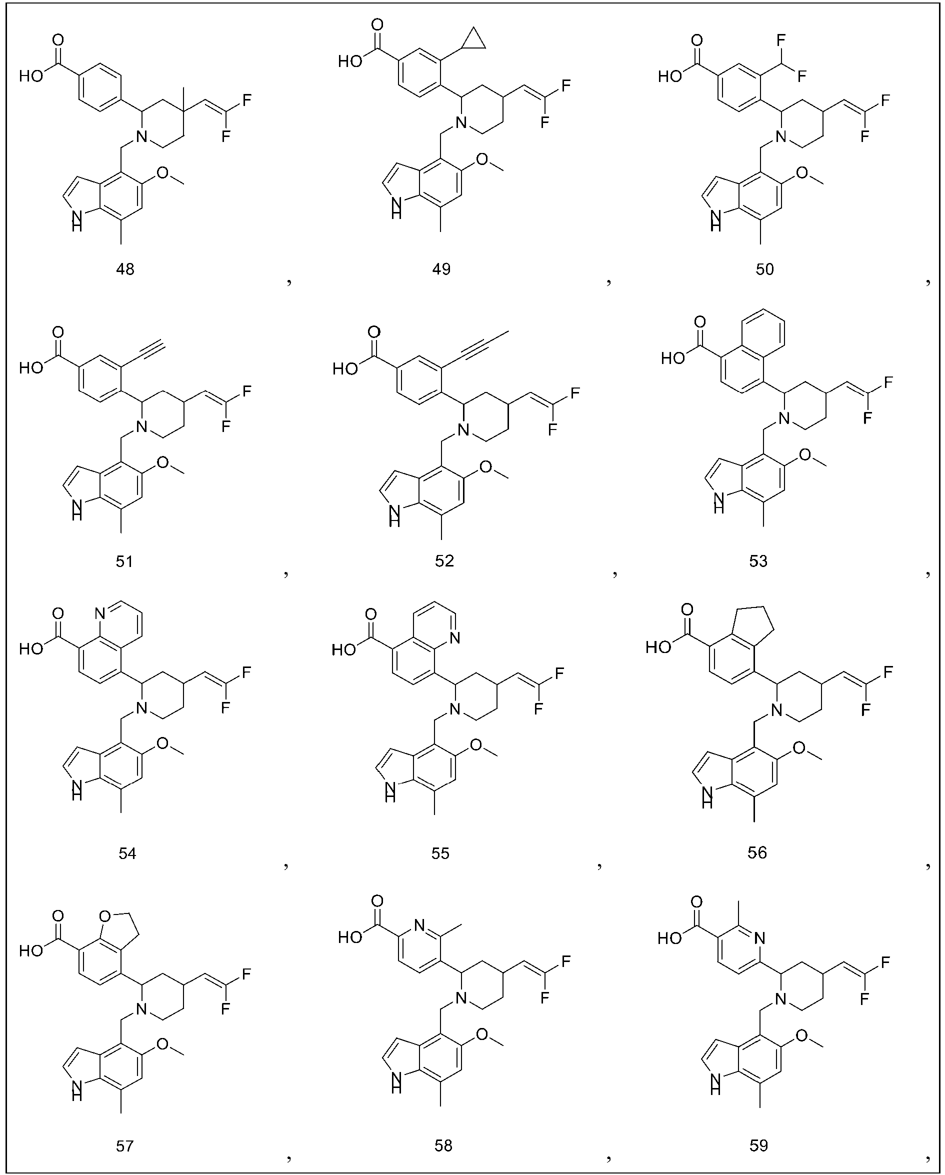

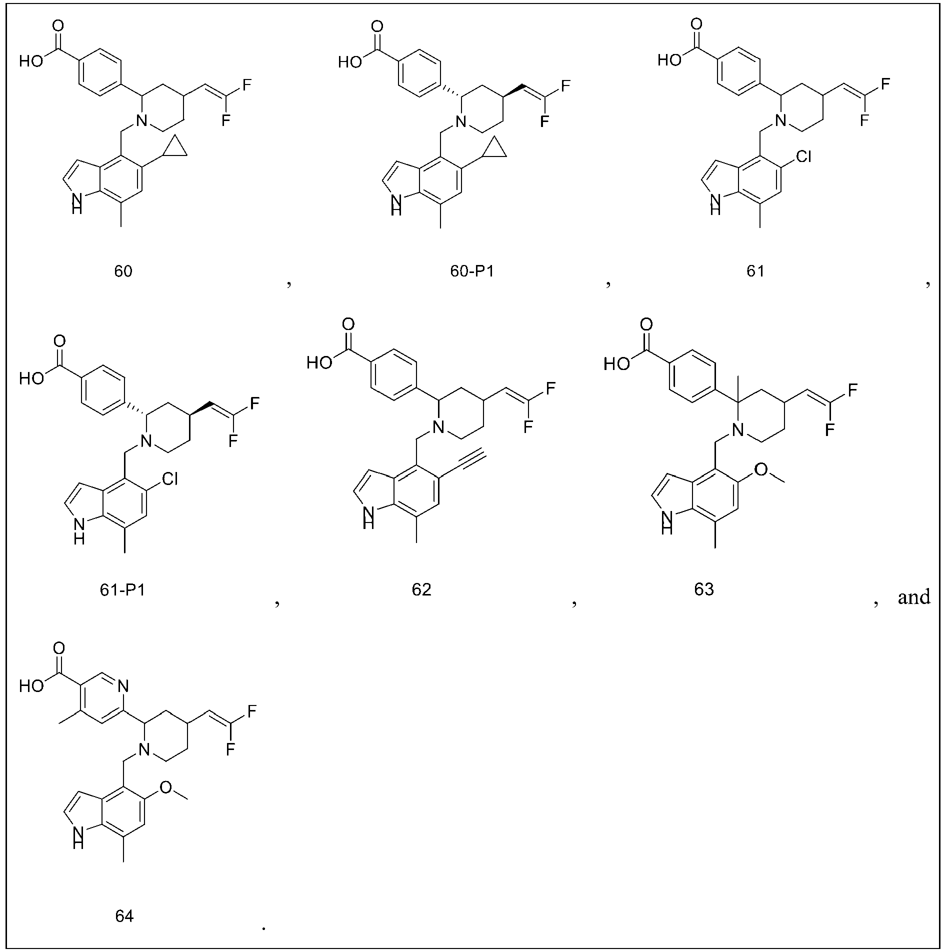

Exemplary specific compounds for the compounds according to the present disclosure further include, but are not limited to, the structures in Table B below:

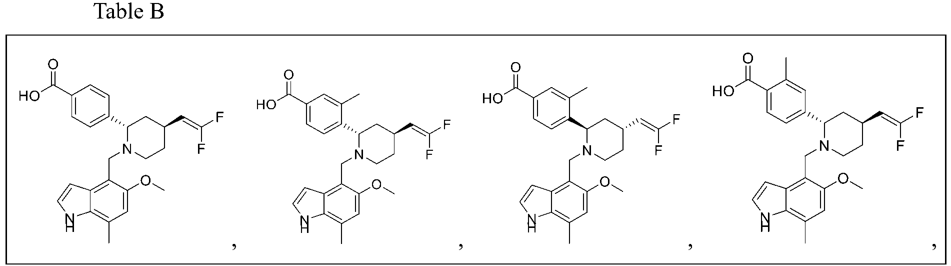

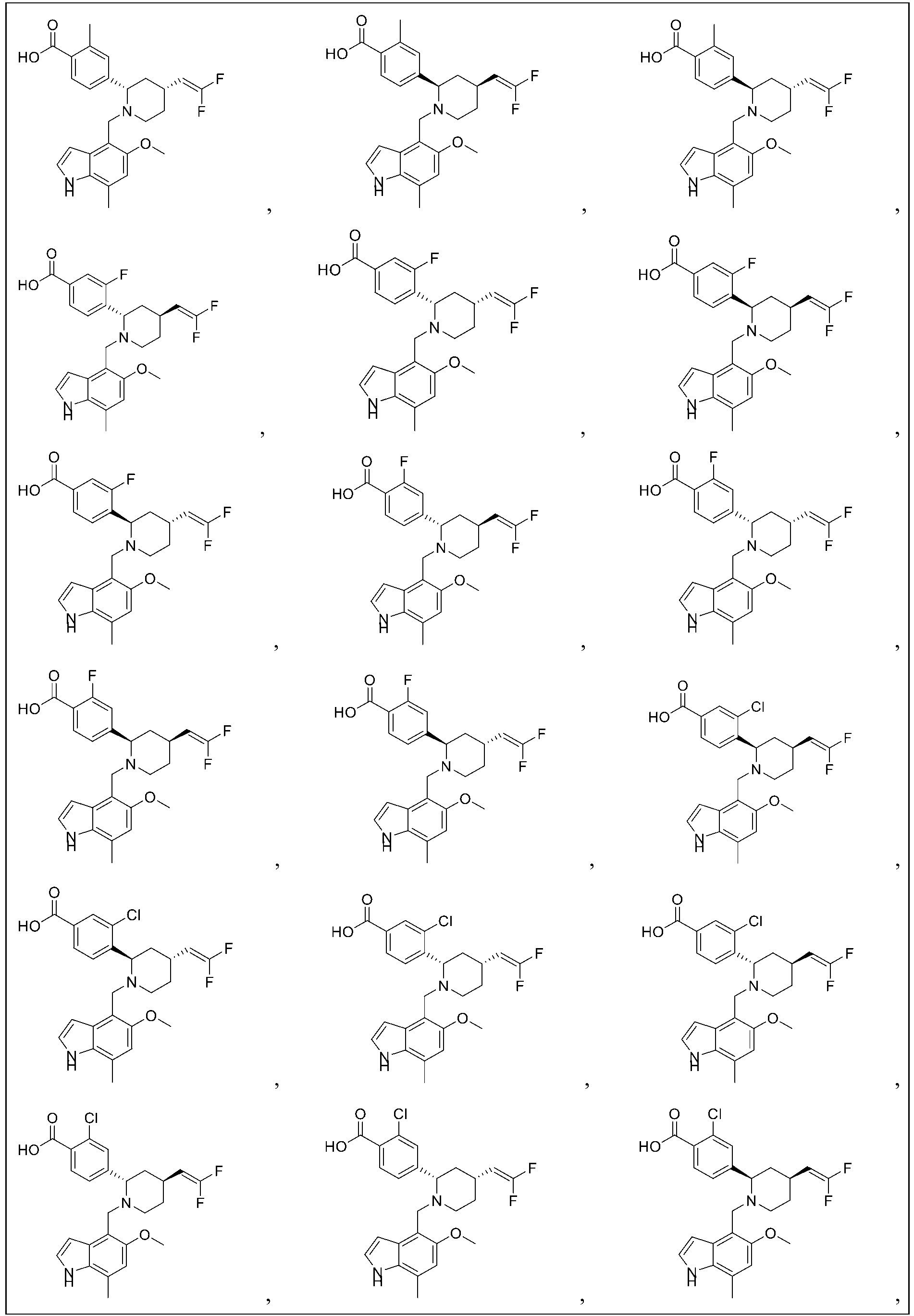

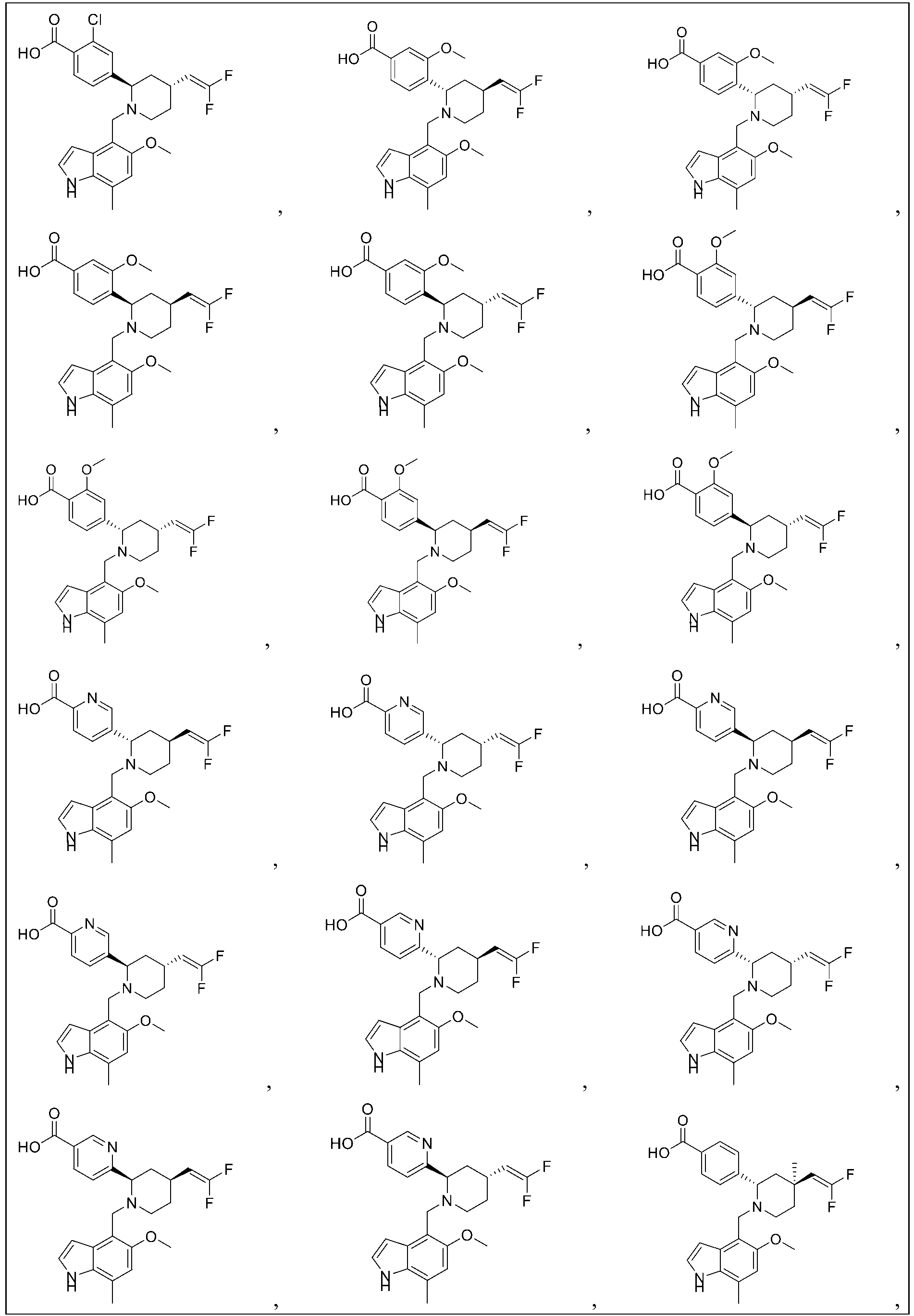

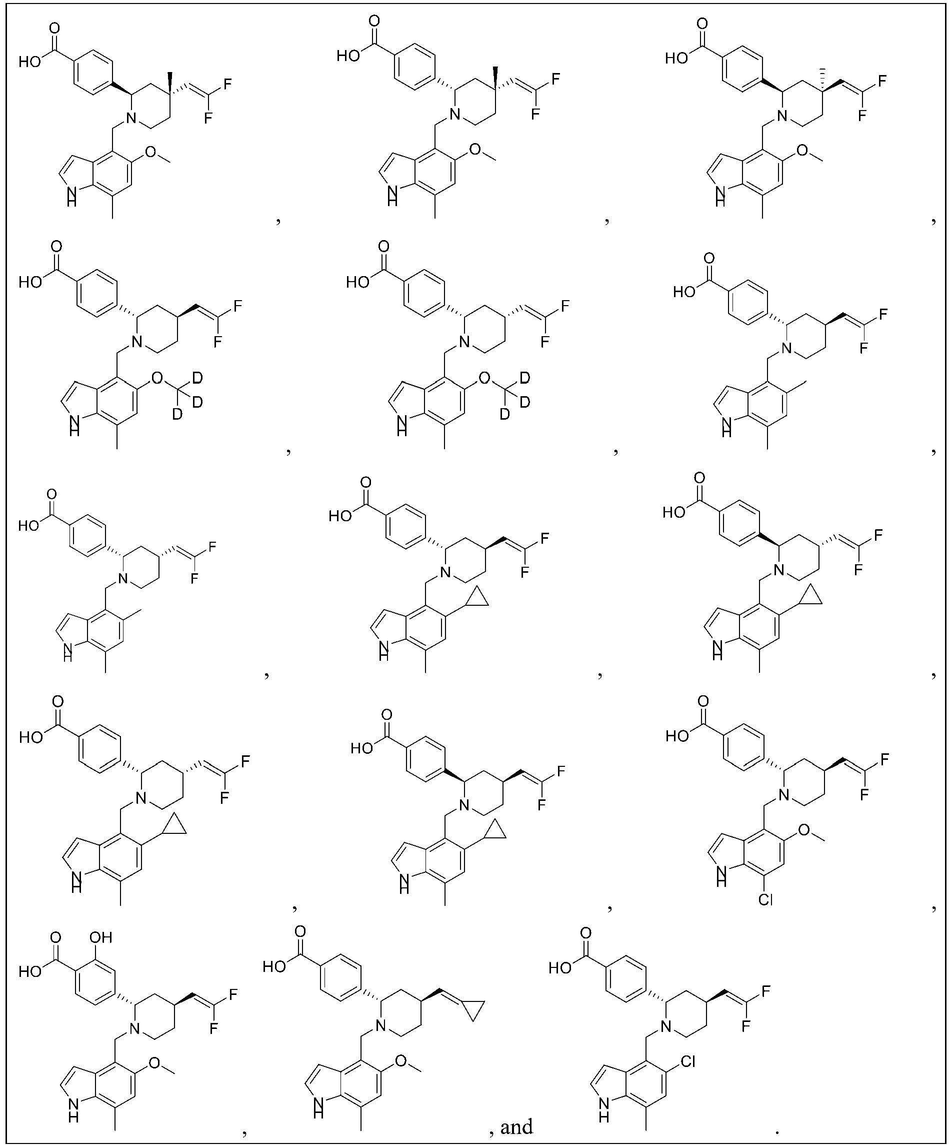

The present disclosure also provides a compound represented by formula (IA) or a salt thereof, 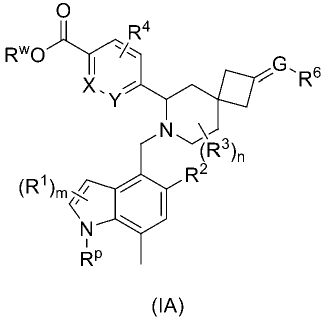 wherein
R^{w} is C₁₋₆ alkyl;
R^{p} is an amino protecting group, preferably benzyl, tert-butoxycarbonyl (Boc), 2-biphenyl-2-propoxycarbonyl, benzyloxycarbonyl, fluorenylmethoxycarbonyl (Fmoc), or trifluoroacetyl;
X, Y, G, R¹, R², R³, R⁴, R⁶, m, and n are as defined in formula (I).

The present disclosure also provides a compound represented by formula (IEA) or a salt thereof, 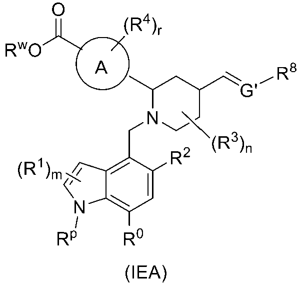 wherein
R^{w} is C₁₋₆ alkyl;
R^{p} is an amino protecting group, preferably benzyl, tert-butoxycarbonyl (Boc), 2-biphenyl-2-propoxycarbonyl, benzyloxycarbonyl, fluorenylmethoxycarbonyl (Fmoc), or trifluoroacetyl;
ring A, G', R⁰, R¹, R², R³, R⁴, R⁸, r, m, and n are as defined in formula (IE).

The present disclosure also provides a compound represented by formula (IGA) or a salt thereof, 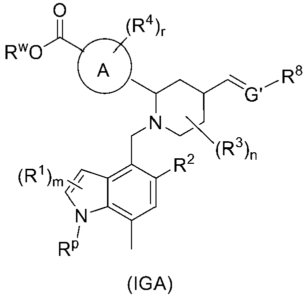 wherein
R^{w} is C₁₋₆ alkyl;
R^{p} is an amino protecting group, preferably benzyl, tert-butoxycarbonyl (Boc), 2-biphenyl-2-propoxycarbonyl, benzyloxycarbonyl, fluorenylmethoxycarbonyl (Fmoc), or trifluoroacetyl;
ring A, G', R¹, R², R³, R⁴, R⁸, r, m, and n are as defined in formula (IG).

The present disclosure also provides a compound represented by formula (IIA) or formula (IIGA) or a salt thereof, 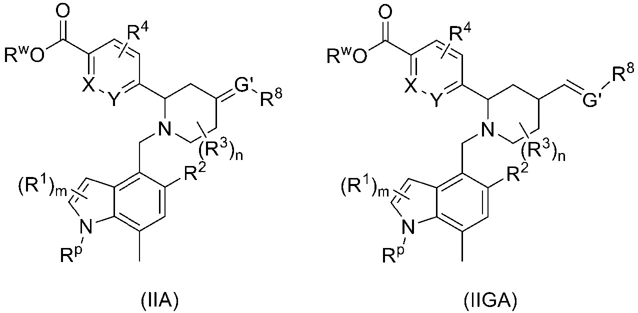 wherein
R^{w} is C₁₋₆ alkyl;
R^{p} is an amino protecting group, preferably benzyl, tert-butoxycarbonyl (Boc), 2-biphenyl-2-propoxycarbonyl, benzyloxycarbonyl, fluorenylmethoxycarbonyl (Fmoc), or trifluoroacetyl;
X, Y, G', R¹, R², R³, R⁴, R⁸, m, and n are as defined in formula (II).

The present disclosure also provides a compound represented by formula (IIIA), (IIIA-1), or (IIIA-2) or a salt thereof, 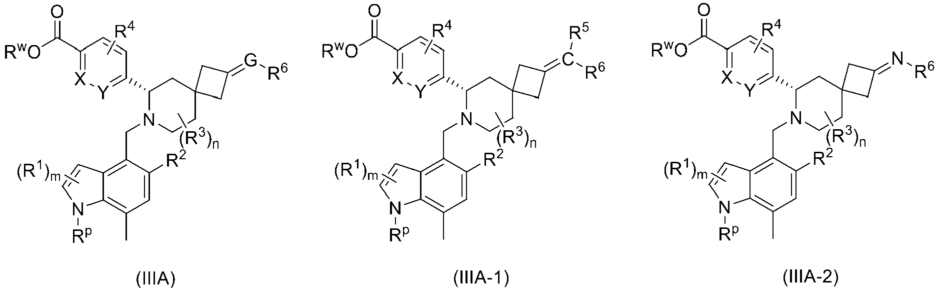 wherein R^{w} is C₁₋₆ alkyl;
R^{p} is an amino protecting group, preferably benzyl, tert-butoxycarbonyl (Boc), 2-biphenyl-2-propoxycarbonyl, benzyloxycarbonyl, fluorenylmethoxycarbonyl (Fmoc), or trifluoroacetyl;
X, Y, G, R¹, R², R³, R⁴, R⁵, R⁶, m, and n are as defined in formula (III).

The present disclosure also provides a compound represented by formula (IVA), (IVA-1), (IVA-2), (IVGA), (IVGA-1), or (IVGA-2) or a salt thereof, 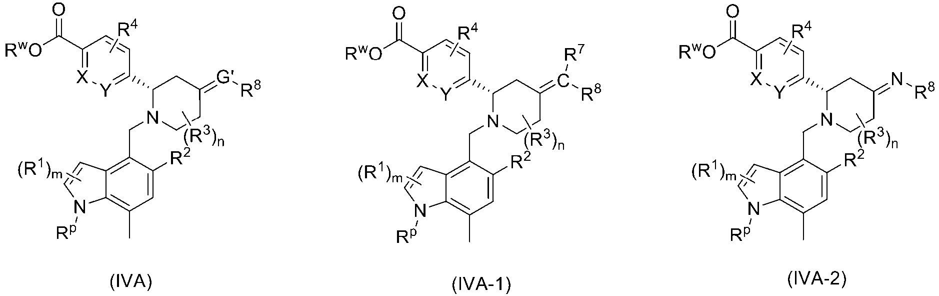 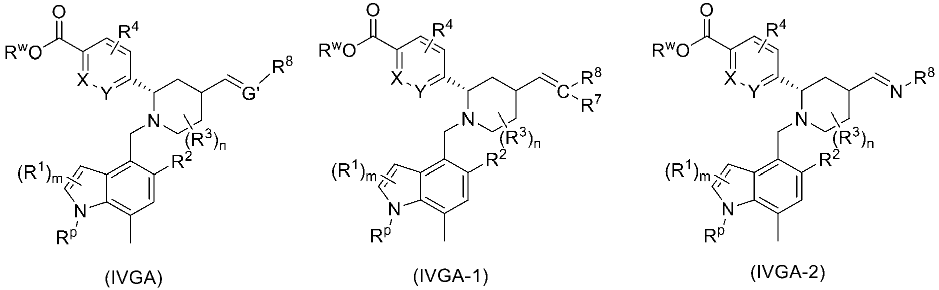 wherein R^{w} is C₁₋₆ alkyl;
R^{p} is an amino protecting group, preferably benzyl, tert-butoxycarbonyl (Boc), 2-biphenyl-2-propoxycarbonyl, benzyloxycarbonyl, fluorenylmethoxycarbonyl (Fmoc), or trifluoroacetyl;
X, Y, G', R¹, R², R³, R⁴, R⁷, R⁸, m, and n are as defined in formula (IV).

Exemplary specific compounds for the intermediate compounds according to the present disclosure include, but are not limited to, the structures in Table C below:

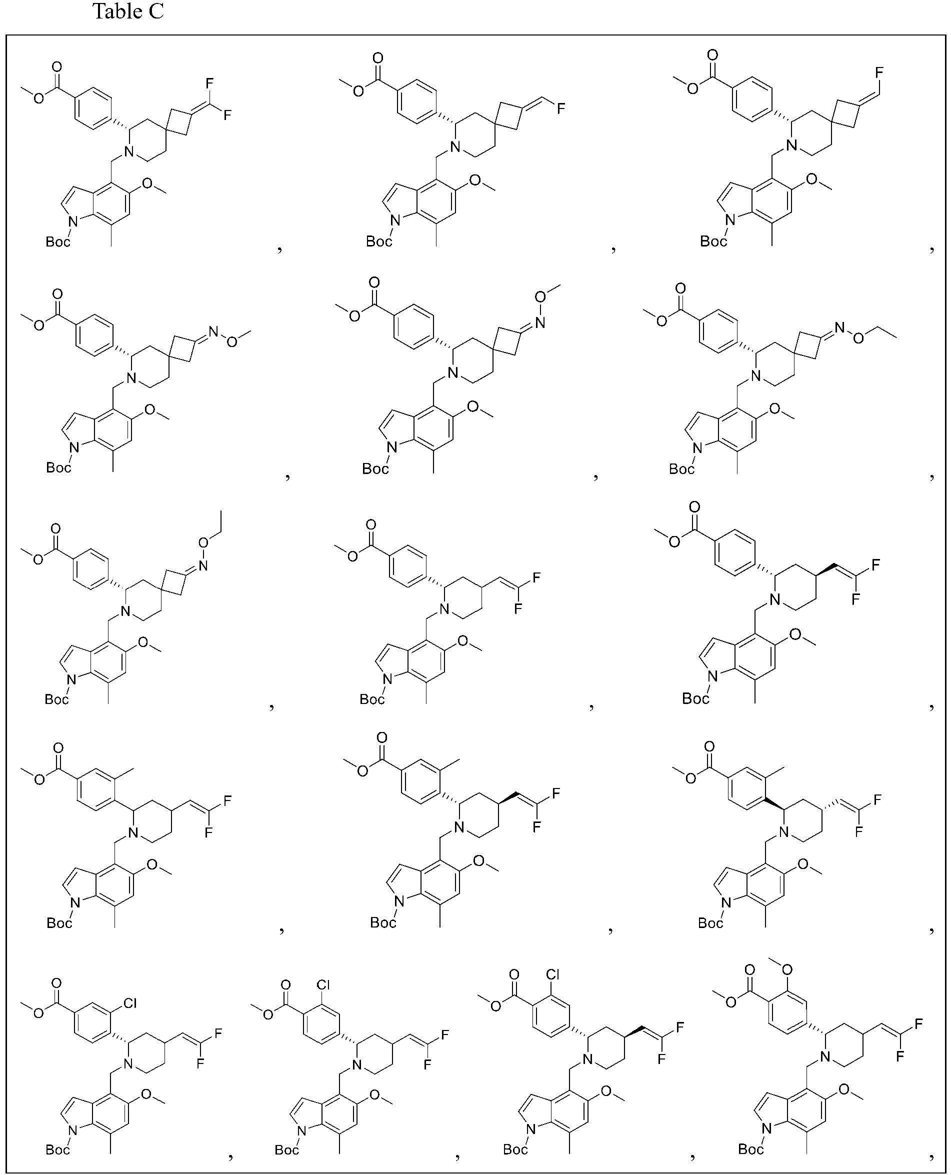

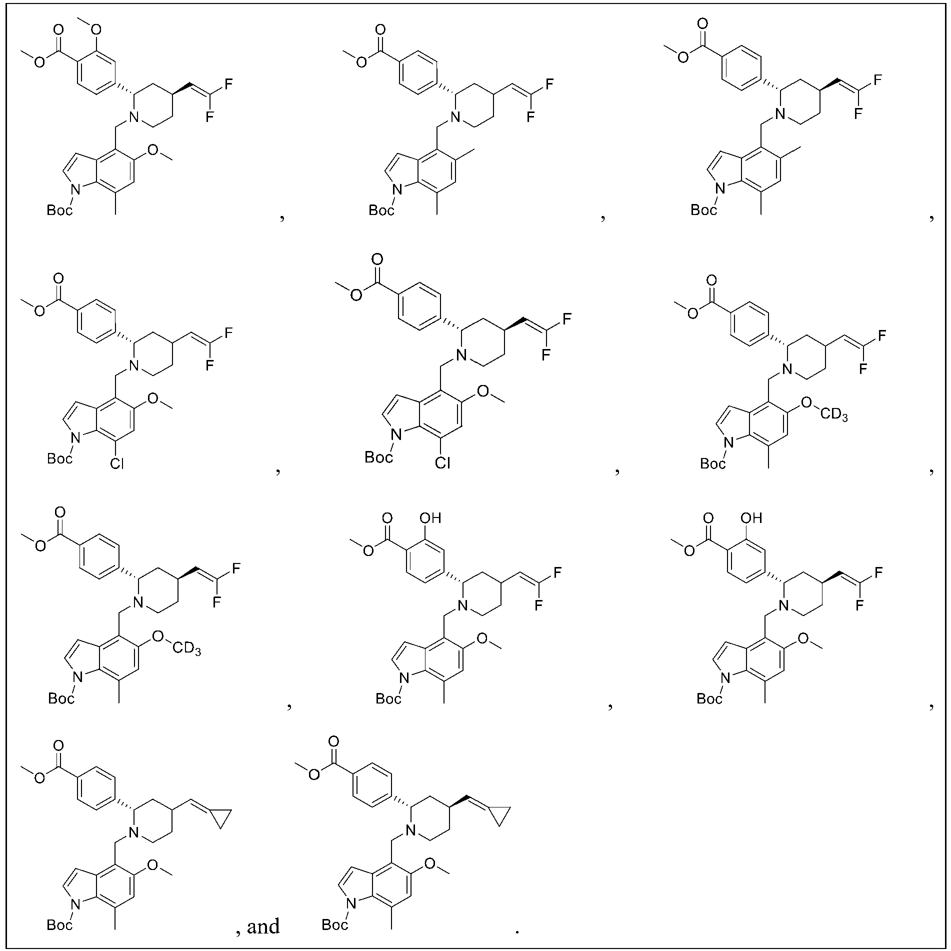

The present disclosure provides a method of preparing the compound represented by formula (I), comprising reacting the compound represented by formula (IA) to obtain the compound represented by formula (I), 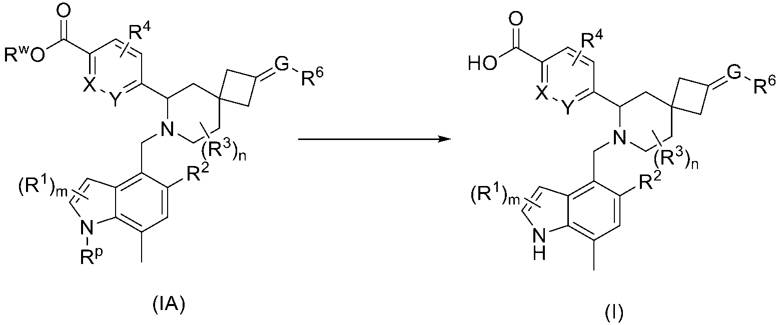 wherein R^{w} is C₁₋₆ alkyl;
R^{p} is an amino protecting group, preferably benzyl, *tert*-butoxycarbonyl (Boc), 2-biphenyl-2-propoxycarbonyl, benzyloxycarbonyl, fluorenylmethoxycarbonyl (Fmoc), or trifluoroacetyl;
X, Y, G, R¹, R², R³, R⁴, R⁶, m, and n are as defined in formula (I).

Referring to the method of preparing the compound represented by formula (I), the compound represented by formula (IIIA), (IIIA-1), or (IIIA-2) is reacted to obtain the corresponding compound represented by formula (III), (III-1), or (III-2), wherein R^{w} is C₁₋₆ alkyl; R^{p} is an amino protecting group, preferably benzyl, *tert*-butoxycarbonyl (Boc), 2-biphenyl-2-propoxycarbonyl, benzyloxycarbonyl, fluorenylmethoxycarbonyl (Fmoc), or trifluoroacetyl.

The present disclosure provides a method of preparing the compound represented by formula (II), comprising reacting the compound represented by formula (IIA) to obtain the compound represented by formula (II), 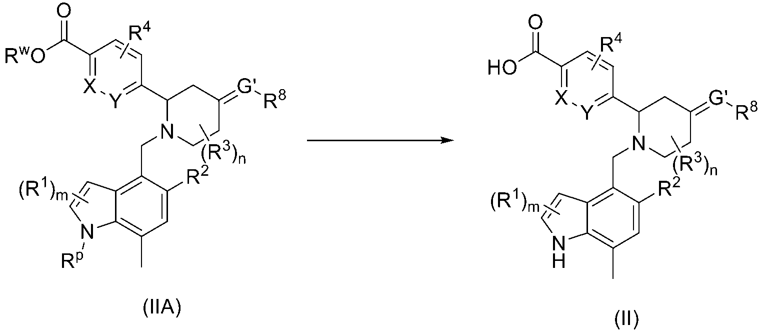 wherein R^{w} is C₁₋₆ alkyl;
R^{p} is an amino protecting group, preferably benzyl, *tert*-butoxycarbonyl (Boc), 2-biphenyl-2-propoxycarbonyl, benzyloxycarbonyl, fluorenylmethoxycarbonyl (Fmoc), or trifluoroacetyl;
X, Y, G', R¹, R², R³, R⁴, R⁸, m, and n are as defined in formula (II).

Referring to the method of preparing the compound represented by formula (II), the compound represented by formula (IVA), (IVA-1), or (IVA-2) is subjected to a hydrolysis reaction to obtain the corresponding compound represented by formula (IV), (IV-1), or (IV-2), wherein R^{w} is C₁₋₆ alkyl; R^{p} is an amino protecting group, preferably benzyl, *tert*-butoxycarbonyl (Boc), 2-biphenyl-2-propoxycarbonyl, benzyloxycarbonyl, fluorenylmethoxycarbonyl (Fmoc), or trifluoroacetyl.

The present disclosure provides a method of preparing the compound represented by formula (IE), comprising subjecting the compound represented by formula (IEA) to hydrolysis and deprotection reactions to obtain the compound represented by formula (IE), 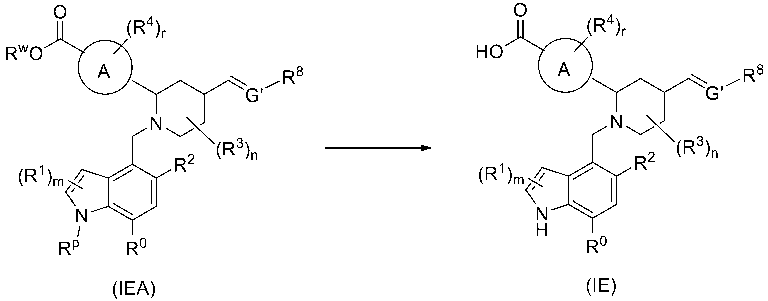 wherein R^{w} is C₁₋₆ alkyl;
R^{p} is an amino protecting group, preferably benzyl, *tert*-butoxycarbonyl (Boc), 2-biphenyl-2-propoxycarbonyl, benzyloxycarbonyl, fluorenylmethoxycarbonyl (Fmoc), or trifluoroacetyl;
ring A, G', R⁰, R¹, R², R³, R⁴, R⁸, r, m, and n are as defined in formula (IE).

The present disclosure provides a method of preparing the compound represented by formula (IG), comprising subjecting the compound represented by formula (IGA) to hydrolysis and deprotection reactions to obtain the compound represented by formula (IG), 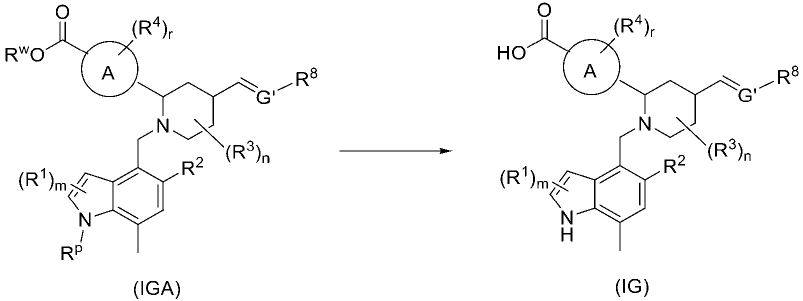 wherein R^{w} is C₁₋₆ alkyl;
R^{p} is an amino protecting group, preferably benzyl, *tert*-butoxycarbonyl (Boc), 2-biphenyl-2-propoxycarbonyl, benzyloxycarbonyl, fluorenylmethoxycarbonyl (Fmoc), or trifluoroacetyl;
ring A, G', R¹, R², R³, R⁴, R⁸, r, m, and n are as defined in formula (IG).

The present disclosure provides a method of preparing the compound represented by formula (IIG), comprising reacting the compound represented by formula (IIGA) to obtain the compound represented by formula (IIG), 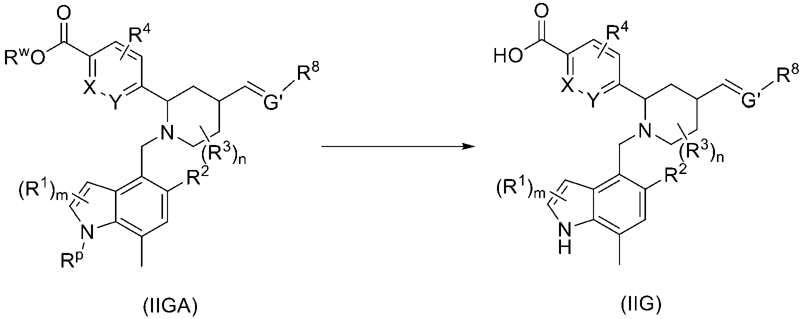 wherein R^{w} is C₁₋₆ alkyl;
R^{p} is an amino protecting group, preferably benzyl, *tert*-butoxycarbonyl (Boc), 2-biphenyl-2-propoxycarbonyl, benzyloxycarbonyl, fluorenylmethoxycarbonyl (Fmoc), or trifluoroacetyl;
X, Y, G', R¹, R², R³, R⁴, R⁸, m, and n are as defined in formula (II).

Referring to the method of preparing the compound represented by formula (II), the compound represented by formula (IVGA), (IVGA-1), or (IVGA-2) is subjected to a hydrolysis reaction to obtain the corresponding compound represented by formula (IVG), (IVG-1), or (IVG-2), wherein R^{w} is C₁₋₆ alkyl; R^{p} is an amino protecting group, preferably benzyl, *tert*-butoxycarbonyl (Boc), 2-biphenyl-2-propoxycarbonyl, benzyloxycarbonyl, fluorenylmethoxycarbonyl (Fmoc), or trifluoroacetyl.

The present disclosure also provides a compound represented by formula (IB) or a salt thereof, 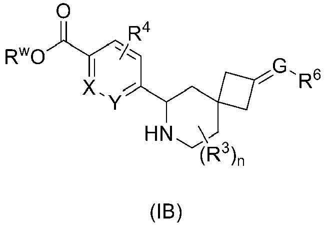 wherein R^{w} is C₁₋₆ alkyl; X, Y, G, R³, R⁴, R⁶, and n are as defined in formula (I).

The present disclosure also provides a compound represented by formula (IIIB) or a salt thereof, 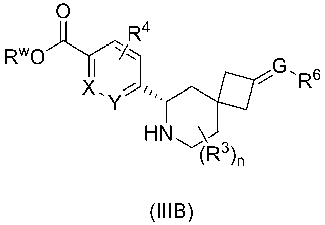 wherein R^{w} is C₁₋₆ alkyl; X, Y, G, R³, R⁴, R⁶, and n are as defined in formula (I).

The present disclosure also provides a compound represented by formula (IGB) or a salt thereof, 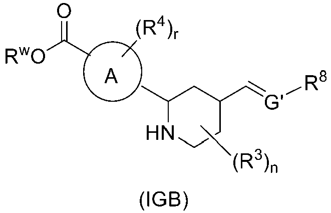 wherein R^{w} is C₁₋₆ alkyl; ring A, G', R³, R⁴, R⁸, r, and n are as defined in formula (IG).

The present disclosure also provides a compound represented by formula (IIB) or formula (IIGB) or a salt thereof, 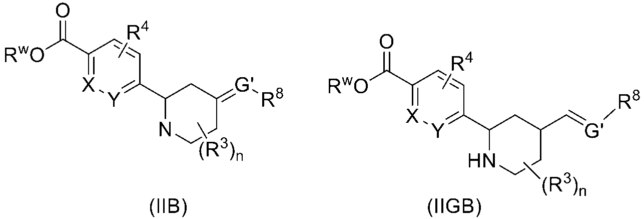 wherein R^{w} is C₁₋₆ alkyl; X, Y, G', R³, R⁴, R⁸, and n are as defined in formula (II).

The present disclosure also provides a compound represented by formula (IVB) or formula (IVGB) or a salt thereof, 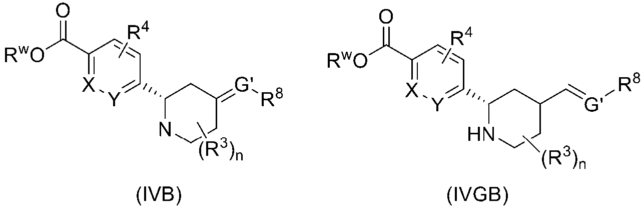 wherein R^{w} is C₁₋₆ alkyl; X, Y, G', R³, R⁴, R⁸, and n are as defined in formula (II).

The present disclosure also provides a method of preparing the compound represented by formula (IA), comprising reacting the compound represented by formula (IB) with a compound represented by formula (IC) to obtain the compound represented by formula (IA), 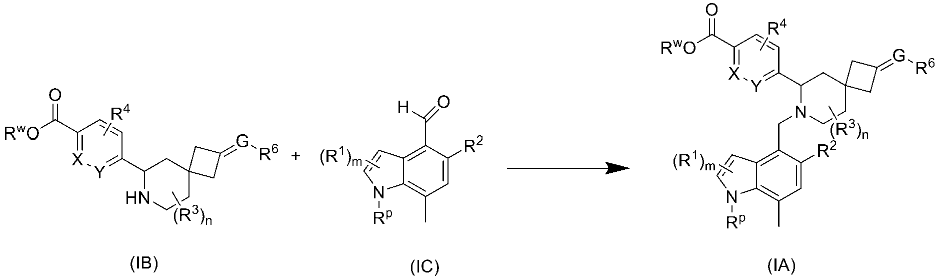 wherein R^{w}, R^{p}, X, Y, G, R¹, R², R³, R⁴, R⁶, m, and n are as defined in formula (IA).

Referring to the method of preparing the compound represented by formula (IA), the compound represented by formula (IIIB) is reacted with the compound represented by formula (IC) to obtain the compound represented by (IIIA).

The present disclosure also provides a method of preparing the compound represented by formula (IIA), comprising reacting the compound represented by formula (IIB) with the compound represented by formula (IC) to obtain the compound represented by formula (IIA), 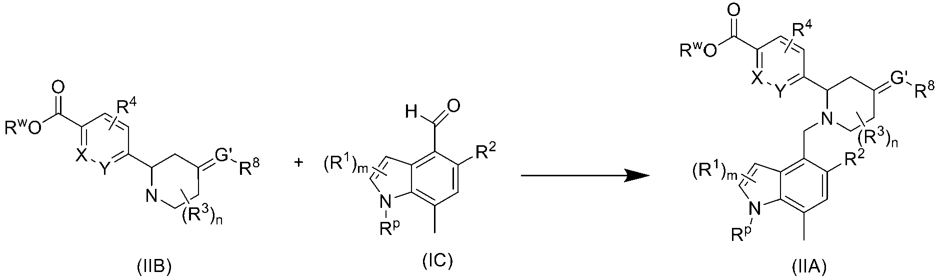 wherein R^{w}, R^{p}, X, Y, G', R¹, R², R³, R⁴, R⁸, m, and n are as defined in formula (IIA).

Referring to the method of preparing the compound represented by formula (IIA), the compound represented by formula (IVB) is reacted with the compound represented by formula (IC) to obtain the compound represented by (IVA).

The present disclosure also provides a method of preparing the compound represented by formula (IGA), comprising reacting the compound represented by formula (IGB) with the compound represented by formula (IC) to obtain the compound represented by formula (IGA), 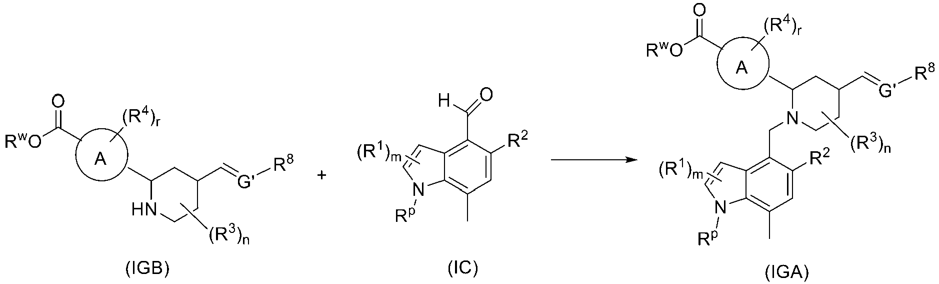 wherein R^{w}, R^{p}, ring A, G', R¹, R², R³, R⁴, R⁸, m, n, and r are as defined in formula (IG).

The present disclosure also provides a method of preparing the compound represented by formula (IIGA), comprising reacting the compound represented by formula (IIGB) with the compound represented by formula (IC) to obtain the compound represented by formula (IIGA), 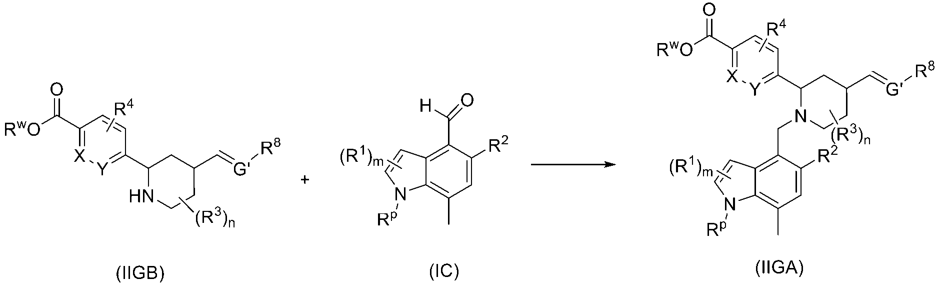 wherein R^{w}, R^{p}, X, Y, G', R¹, R², R³, R⁴, R⁸, m, and n are as defined in formula (IIA).

Referring to the method of preparing the compound represented by formula (IIGA), the compound represented by formula (IVGB) is reacted with the compound represented by formula (IC) to obtain the compound represented by (IVGA).

In another aspect, the present disclosure provides an isotopically labeled compound of the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, wherein the isotopic labeling is preferably deuterium (D or ²H) substitution for hydrogen (¹H).

In another aspect, the present disclosure provides a pharmaceutical composition comprising at least one therapeutically effective amount of the compound or a pharmaceutically acceptable salt thereof, and one or more pharmaceutically acceptable excipients.

In another aspect, the present disclosure also provides a use of the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition comprising the same, in the manufacture of a medicament for modulating the activity of an alternative complement pathway in a subject.

In another aspect, the present disclosure also provides a use of the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition comprising the same, in the manufacture of a medicament for preventing and/or treating a disease or condition affected by modulation of the alternative complement pathway.

The present disclosure also provides a use of the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition comprising the same, in the manufacture of a medicament for inhibiting complement factor B.

The present disclosure also provides a use of the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition comprising the same, in the manufacture of a medicament for preventing and/or treating a disease or condition mediated by a complement factor B inhibitor.

The present disclosure also provides a use of the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition comprising the same, in the manufacture of a medicament for preventing and/or treating an autoimmune disease, an inflammatory disease, a hematological disease, and a neurodegenerative disease; preferably, in the manufacture of a medicament for preventing and/or treating paroxysmal nocturnal hemoglobinuria (PNH), primary glomerulonephritis (IgAN), membranous nephropathy (MN), diabetic kidney disease (DKD), C3 glomerulonephritis (C3G), lupus nephritis (LN), proliferative nephritis, systemic lupus erythematosus (SLE), age-related macular degeneration (AMD), geographic atrophy (GA), atypical hemolytic uremic syndrome (aHUS), hemolytic uremic syndrome (HUS), complications of hemodialysis, hemolytic anemia or hemodialysis, neuromyelitis optica (NMO), arthritis, rheumatoid arthritis, psoriatic arthritis, liver inflammation, dermatomyositis and amyotrophic lateral sclerosis, myasthenia gravis (MG), Guillain-Barré syndrome (GBS), neuromyelitis optica spectrum disorder (NMOSD), uveitis, retinitis pigmentosa, macular edema, Behçet's uveitis, multifocal choroiditis, Vogt-Koyanagi-Harada syndrome, intermediate uveitis, birdshot retinochoroiditis, sympathetic ophthalmia, ocular pemphigoid, ocular pemphigus, non-arteritic ischemic optic neuropathy, postoperative inflammation, retinal vein occlusion, glaucoma, Doyne honeycomb retinal dystrophy/Malattia Leventinese, Sorsby's fundus dystrophy, late-onset retinal macular dystrophy, North Carolina macular dystrophy, Stargardt disease, keratitis, anti-neutrophil cytoplasmic antibody-associated vasculitis (ANCA-AAV), Crohn's disease, adult respiratory distress syndrome, myocarditis, post-ischemia-reperfusion injury, myocardial infarction, balloon angioplasty, post-pump syndrome following cardiopulmonary bypass or renal bypass, atherosclerosis, hemodialysis, renal ischemia, acute kidney injury, mesenteric artery reperfusion after aortic reconstruction, *etc.*

The present disclosure also provides a method of inhibiting complement factor B, comprising administering to a patient in need thereof a therapeutically effective amount of the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the isotopically labeled compound, or the pharmaceutical composition comprising the same.

The present disclosure also provides a method of preventing and/or treating a disease or condition mediated by complement factor B, comprising administering to a patient in need thereof a therapeutically effective amount of the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the isotopically labeled compound, or the pharmaceutical composition comprising the same.

The present disclosure also provides a method of preventing and/or treating an autoimmune disease, an inflammatory disease, a hematological disease, and a neurodegenerative disease, comprising administering to a patient in need thereof a therapeutically effective amount of the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition comprising the same.

The present disclosure also provides a method of preventing and/or treating paroxysmal nocturnal hemoglobinuria (PNH), primary glomerulonephritis (IgAN), membranous nephropathy (MN), diabetic kidney disease (DKD), C3 glomerulonephritis (C3G), lupus nephritis (LN), proliferative nephritis, systemic lupus erythematosus (SLE), age-related macular degeneration (AMD), geographic atrophy (GA), atypical hemolytic uremic syndrome (aHUS), hemolytic uremic syndrome (HUS), complications of hemodialysis, hemolytic anemia or hemodialysis, neuromyelitis optica (NMO), arthritis, rheumatoid arthritis, psoriatic arthritis, liver inflammation, dermatomyositis and amyotrophic lateral sclerosis, myasthenia gravis (MG), Guillain-Barré syndrome (GBS), neuromyelitis optica spectrum disorder (NMOSD), uveitis, retinitis pigmentosa, macular edema, Behçet's uveitis, multifocal choroiditis, Vogt-Koyanagi-Harada syndrome, intermediate uveitis, birdshot retinochoroiditis, sympathetic ophthalmia, ocular pemphigoid, ocular pemphigus, non-arteritic ischemic optic neuropathy, postoperative inflammation, retinal vein occlusion, glaucoma, Doyne honeycomb retinal dystrophy/Malattia Leventinese, Sorsby's fundus dystrophy, late-onset retinal macular dystrophy, North Carolina macular dystrophy, Stargardt disease, keratitis, anti-neutrophil cytoplasmic antibody-associated vasculitis (ANCA-AAV), Crohn's disease, adult respiratory distress syndrome, myocarditis, post-ischemia-reperfusion injury, myocardial infarction, balloon angioplasty, post-pump syndrome following cardiopulmonary bypass or renal bypass, atherosclerosis, hemodialysis, renal ischemia, acute kidney injury, mesenteric artery reperfusion after aortic reconstruction, *etc.,* comprising administering to a patient in need thereof a therapeutically effective amount of the compound represented by formula (I), formula (IG), formula (II), formula (IIG), formula (III), formula (IV), formula (IVG), formula (III-1), formula (III-2), formula (IV- 1), formula (IV-2), formula (IVG-1), formula (IVG-2), or formula (IVG-3), or the compound shown in Table A or Table B, or a pharmaceutically acceptable salt thereof, or the pharmaceutical composition comprising the same.

The present disclosure also provides the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition, for use as a medicament.

The present disclosure also provides the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition, for use as a complement factor B inhibitor.

The present disclosure also provides the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition comprising the same, for use as a medicament for preventing and/or treating a disease or condition mediated by complement factor B.

The present disclosure also provides the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition comprising the same, for use as a medicament for preventing and/or treating an autoimmune disease, an inflammatory disease, a hematological disease, and a neurodegenerative disease.

The present disclosure also provides the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or the pharmaceutical composition comprising the same, for use as a medicament for preventing and/or treating paroxysmal nocturnal hemoglobinuria (PNH), primary glomerulonephritis (IgAN), membranous nephropathy (MN), diabetic kidney disease (DKD), C3 glomerulonephritis (C3G), lupus nephritis (LN), proliferative nephritis, systemic lupus erythematosus (SLE), age-related macular degeneration (AMD), geographic atrophy (GA), atypical hemolytic uremic syndrome (aHUS), hemolytic uremic syndrome (HUS), complications of hemodialysis, hemolytic anemia or hemodialysis, neuromyelitis optica (NMO), arthritis, rheumatoid arthritis, psoriatic arthritis, liver inflammation, dermatomyositis and amyotrophic lateral sclerosis, myasthenia gravis (MG), Guillain-Barré syndrome (GBS), neuromyelitis optica spectrum disorder (NMOSD), uveitis, retinitis pigmentosa, macular edema, Behçet's uveitis, multifocal choroiditis, Vogt-Koyanagi-Harada syndrome, intermediate uveitis, birdshot retinochoroiditis, sympathetic ophthalmia, ocular pemphigoid, ocular pemphigus, non-arteritic ischemic optic neuropathy, postoperative inflammation, retinal vein occlusion, glaucoma, Doyne honeycomb retinal dystrophy/Malattia Leventinese, Sorsby's fundus dystrophy, late-onset retinal macular dystrophy, North Carolina macular dystrophy, Stargardt disease, keratitis, anti-neutrophil cytoplasmic antibody-associated vasculitis (ANCA-AAV), Crohn's disease, adult respiratory distress syndrome, myocarditis, post-ischemia-reperfusion injury, myocardial infarction, balloon angioplasty, post-pump syndrome following cardiopulmonary bypass or renal bypass, atherosclerosis, hemodialysis, renal ischemia, acute kidney injury, mesenteric artery reperfusion after aortic reconstruction, *etc.*

The disease mediated by complement factor B according to the present disclosure is selected from the group consisting of an autoimmune disease, an inflammatory disease, a hematological disease, and a neurodegenerative disease.

The disease mediated by complement factor B according to the present disclosure is selected from the group consisting of paroxysmal nocturnal hemoglobinuria (PNH), diabetic kidney disease (DKD), primary glomerulonephritis (IgAN), membranous nephropathy (MN), C3 glomerulonephritis (C3G), lupus nephritis (LN), proliferative nephritis, systemic lupus erythematosus (SLE), age-related macular degeneration (AMD), geographic atrophy (GA), atypical hemolytic uremic syndrome (aHUS), hemolytic uremic syndrome (HUS), complications of hemodialysis, hemolytic anemia or hemodialysis, neuromyelitis optica (NMO), arthritis, rheumatoid arthritis, psoriatic arthritis, liver inflammation, dermatomyositis and amyotrophic lateral sclerosis, myasthenia gravis (MG), Guillain-Barré syndrome (GBS), neuromyelitis optica spectrum disorder (NMOSD), uveitis, retinitis pigmentosa, macular edema, Behçet's uveitis, multifocal choroiditis, Vogt-Koyanagi-Harada syndrome, intermediate uveitis, birdshot retinochoroiditis, sympathetic ophthalmia, ocular pemphigoid, ocular pemphigus, non-arteritic ischemic optic neuropathy, postoperative inflammation, retinal vein occlusion, glaucoma, Doyne honeycomb retinal dystrophy/Malattia Leventinese, Sorsby's fundus dystrophy, late-onset retinal macular dystrophy, North Carolina macular dystrophy, Stargardt disease, keratitis, anti-neutrophil cytoplasmic antibody-associated vasculitis (ANCA-AAV), Crohn's disease, adult respiratory distress syndrome, myocarditis, post-ischemia-reperfusion injury, myocardial infarction, balloon angioplasty, post-pump syndrome following cardiopulmonary bypass or renal bypass, atherosclerosis, hemodialysis, renal ischemia, acute kidney injury, and mesenteric artery reperfusion after aortic reconstruction.

In some embodiments, the unit dose of the pharmaceutical composition is 0.001 mg to 1000 mg.

In some embodiments, based on the total weight of the composition, the pharmaceutical composition comprises 0.01% to 99.99% of the compound or a pharmaceutically acceptable salt thereof or an isotopically labeled compound thereof. In some embodiments, the pharmaceutical composition comprises 0.1% to 99.9% of the compound or a pharmaceutically acceptable salt thereof or an isotopically labeled compound thereof. In some embodiments, the pharmaceutical composition comprises 0.5% to 99.5% of the compound or a pharmaceutically acceptable salt thereof or an isotopically labeled compound thereof.

In some embodiments, the pharmaceutical composition comprises 1% to 99% of the compound represented by formula (I), formula (IG), formula (II), formula (IIG), formula (III), formula (IV), formula (IVG), formula (III-1), formula (III-2), formula (IV-1), formula (IV-2), formula (IVG-1), formula (IVG-2), or formula (IVG-3), or the compound shown in Table A or Table B, or a pharmaceutically acceptable salt thereof, or an isotopically labeled compound thereof.

In some embodiments, based on the total weight of the composition, the pharmaceutical composition comprises 0.01% to 99.99% of one or more pharmaceutically acceptable excipients. In some embodiments, the pharmaceutical composition comprises 0.1% to 99.9% of one or more pharmaceutically acceptable excipients. In some embodiments, the pharmaceutical composition comprises 1% to 99% of one or more pharmaceutically acceptable excipients.

When used as a medicament, the compound of the present disclosure may be administered in the form of a pharmaceutical composition. These compositions can be prepared in a manner well-known in the pharmaceutical field and may be administered by various routes, depending on whether local or systemic treatment is desired and the area to be treated. Administration may be topical (*e.g.,* transdermal, dermal, ocular, and mucosal routes including intranasal, vaginal, and rectal delivery), pulmonary (*e.g*., by inhalation or insufflation of powders or aerosols, including via nebulizers; intratracheal, intranasal), oral, or parenteral. Parenteral administration includes intravenous, intraarterial, subcutaneous, intraperitoneal, or intramuscular injection or infusion; or intracranial administration such as intrathecal or intraventricular administration. These compositions may be administered parenterally in a single bolus dose or via, for example, a continuous infusion pump.

In the preparation of the compositions of the present disclosure, the active ingredient is typically mixed with excipients, and the compositions may be in the following forms: tablets, pills, powders, lozenges, sachets, cachets, elixirs, suspensions, emulsions, solutions, syrups, aerosols (solid or dissolved in a liquid vehicle), ointments containing, for example, up to 10% by weight of the active compound, soft and hard gelatin capsules, suppositories, sterile injectable solutions, and sterile packaged powders.

The "excipient" of the present disclosure refers to components other than the active ingredient, including, for example, diluents, fillers, absorbents, wetting agents, binders, disintegrants, and lubricants.

In another aspect, the pharmaceutically acceptable salts of the compounds of the present disclosure may be inorganic salts or organic salts. If these compounds have a basic center, they may form acid addition salts. If these compounds have an acidic center, they may form base addition salts. If these compounds include both an acidic center *(e.g.,* carboxyl) and a basic center *(e.g.,* amino), they may also form inner salts.

In another aspect, the compounds of the present disclosure may exist in specific geometric or stereoisomeric forms. For example, *cis* and *trans* isomers, (-)- and (+)- enantiomers, (R)- and (S)-enantiomers, diastereomers, (D)-isomers, (L)-isomers, racemic mixtures and other mixtures, as well as enantiomerically or diastereomerically enriched mixtures, all of which fall within the scope of the present disclosure. Additional asymmetric carbon atoms may be present in substituents such as alkyl. All these isomers and their mixtures are included within the scope of the present disclosure.

In the chemical structures of the compounds of the present disclosure, the bond "

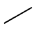 " indicates an unspecified configuration, " 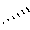 " or " 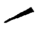 " indicates an absolute configuration, *i.e.,* if chiral isomers exist in the chemical structure, the bond " 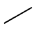 " may be " 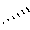 " or " 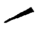 ", or simultaneously include both configurations " 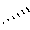 " and " 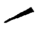 ", and " 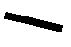 " indicates the presence of axial chirality.

The bond " 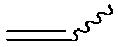 " indicates an unspecified configuration, including the *cis* (*E*) or *trans* (*Z*) configuration.

Additionally, the compounds and intermediates of the present disclosure may also exist in various tautomeric forms, all of which are included within the scope of the present disclosure. "Tautomers" refer to structural isomers with different energies that can interconvert via a low-energy barrier. For example, proton tautomers (also known as proton-transfer tautomers) include interconversion via proton migration, such as keto-enol isomerization, imine-enamine isomerization, and lactam-lactim isomerization. All tautomeric forms of all compounds of the present disclosure are within the scope of the present disclosure. The name of a compound designated by a single method does not exclude any tautomers.

The present disclosure also includes isotopically labeled compounds of the present disclosure, which are identical in structure to those described herein, except that one or more atoms are replaced by atoms having an atomic weight or mass number different from the atomic weight or mass number typically found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl, respectively. All isotopic variations of the compounds of the present disclosure, whether radioactive or not, are included within the scope of the present disclosure.

Unless otherwise specified, when a position is specifically designated as deuterium (D), it shall be understood to have a deuterium abundance at least 1000 times greater than the natural abundance of deuterium (which is 0.015%) *(i.e.,* at least 10% deuterium incorporation). The abundance of deuterium in the compound in the example may be at least 1000 times greater than the natural abundance of deuterium, at least 2000 times greater than the natural abundance of deuterium, at least 3000 times greater than the natural abundance of deuterium, at least 4000 times greater than the natural abundance of deuterium, at least 5000 times greater than the natural abundance of deuterium, at least 6000 times greater than the natural abundance of deuterium, or even higher. Each available hydrogen atom attached to a carbon atom can be independently replaced by a deuterium atom. Those skilled in the art can synthesize deuterated forms of the compounds by referring to relevant literature. In the preparation of deuterated forms of compounds, commercially available deuterated starting materials can be used, or they can be synthesized using conventional techniques with deuterated reagents, including, but not limited to, deuterated borane, trideuterated borane tetrahydrofuran solution, deuterated lithium aluminum hydride, deuterated iodoethane, and deuterated iodomethane.

The term "therapeutically effective amount" of the present disclosure refers to the amount of an active compound or medicament sought by researchers, veterinarians, physicians, or other clinicians to elicit a biological or medical response in tissues, systems, animals, individuals, or humans, including one or more of the following: (1) Disease prevention: for example, preventing a disease, disorder, or condition in individuals susceptible to the disease, disorder, or condition but who have not yet experienced or exhibited pathology or symptoms of the disease. (2) Disease suppression: for example, suppressing a disease, disorder, or condition in individuals currently experiencing or exhibiting pathology or symptoms of the disease, disorder, or condition *(i.e.,* halting further progression of the pathology and/or symptoms). (3) Disease alleviation: for example, alleviating a disease, disorder, or condition in individuals currently experiencing or exhibiting pathology or symptoms of the disease, disorder, or condition *(i.e.,* reversing the pathology and/or symptoms). For a medicament or pharmacologically active agent, a "therapeutically effective amount" refers to a sufficient amount of the medicament or agent that is non-toxic but can achieve the desired effect. The determination of an effective amount varies from person to person, depending on the age and general condition of a subject, as well as the specific active substance. The appropriate effective amount in individual cases can be determined by those skilled in the art through routine testing.

The term "pharmaceutically acceptable" of the present disclosure refers to compounds, materials, compositions, and/or dosage forms that are, within the scope of sound medical judgment, suitable for contact with patient tissues without excess toxicity, irritation, allergic reactions, or other problems or complications, and possess a reasonable benefit/risk ratio while being effective for their intended use.

The term "patient" of the present disclosure refers to any animal, including mammals, preferably mice, rats, other rodents, rabbits, dogs, cats, pigs, cattle, sheep, horses, or primates, and most preferably humans.

### Beneficial Effects

The present disclosure provides a small molecule compound that can serve as a complement factor B inhibitor. Such compounds or pharmaceutical compositions can be used for effectively treating or preventing a disease mediated by complement factor B. Compounds of such structure exhibit good biological activity.

### Term Definitions and Descriptions

Unless stated to the contrary, the terms used in the specification and claims have the following meanings.

The term "alkyl" refers to a saturated aliphatic hydrocarbon group, which is a linear or branched group containing 1 to 20 carbon atoms, preferably an alkyl group containing 1 to 12 *(e.g., 1,* 2, 3, 4, 5, 6, 7, 8, 9, 10, 11, and 12) carbon atoms, and more preferably an alkyl group containing 1 to 6 carbon atoms (C₁₋₆ alkyl). Non-limiting examples of alkyl groups include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, various branched isomers thereof, and the like. The alkyl group may be substituted or unsubstituted.

The term "alkylene" refers to a divalent alkyl group (wherein the alkyl is as defined above) containing 1 to 20 (*e.g.,* 1, 2, 3, 4, 5, 6, 7, 8, 9, 10, 11, 12, 13, 14, 15, 16, 17, 18, 19, and 20) carbon atoms (*i.e.,* C₁₋₂₀ alkylene). The alkylene group is preferably an alkylene group containing 1 to 12 carbon atoms (*i.e.,* C₁₋₁₂ alkylene), and more preferably an alkylene group containing 1 to 6 carbon atoms (*i.e.,* C₁₋₆ alkylene). Non-limiting examples of alkylene groups include, but are not limited to, methylene (-CH₂-), 1,1-ethylene (-CH(CH₃)-), 1,2-ethylene (-CH₂CH₂)-, 1,1-propylene (-CH(CH₂CH₃)-), 1,2-propylene (-CH₂CH(CH₃)-), 1,3-propylene (-CH₂CH₂CH₂-), and 1,4-butylene (-CH₂CH₂CH₂CH₂-). The alkylene group may be substituted or unsubstituted. When substituted, it may be substituted at any available connecting point, and the substituent is preferably selected from the group consisting of one or more of alkyl, alkenyl, alkynyl, alkoxy, haloalkoxy, cycloalkyloxy, heterocyclyloxy, alkylthio, alkylamino, halogen, mercapto, hydroxyl, nitro, cyano, cycloalkyl, heterocyclyl, aryl, heteroaryl, cycloalkoxy, heterocycloalkoxy, cycloalkylthio, heterocycloalkylthio, and oxo.

The term "alkoxy" refers to -O-(alkyl), wherein the alkyl is as defined herein. The alkoxy group is preferably an alkoxy group containing 1 to 12 (*e.g.,* 1, 2, 3, 4, 5, 6, 7, 8, 9, 10, 11, and 12) carbon atoms (C₁₋₁₂ alkoxy), and more preferably an alkoxy group containing 1 to 6 carbon atoms (C₁₋₆ alkoxy). Non-limiting examples of alkoxy groups include methoxy, ethoxy, propoxy, and butoxy. The alkoxy group may be substituted or unsubstituted.

The term "cycloalkyl" refers to a saturated or partially unsaturated monocyclic or polycyclic hydrocarbon substituent, wherein the cycloalkyl ring may contain 3 to 20 carbon atoms (*i.e.,* 3- to 20-membered cycloalkyl or C₃₋₂₀ cycloalkyl), preferably 3 to 12 carbon atoms (*i.e.,* 3- to 12-membered cycloalkyl or C₃₋₁₂ cycloalkyl) or 3 to 8 (*e.g.,* 3, 4, 5, 6, 7, and 8) carbon atoms (*i.e.,* 3- to 8-membered cycloalkyl or C₃₋₈ cycloalkyl), more preferably 3 to 6 carbon atoms (*i.e.,* 3- to 6-membered cycloalkyl or C₃₋₆ cycloalkyl). Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, *etc.* Polycyclic cycloalkyl groups include spiro, fused, and bridged cycloalkyl groups.

The term "spirocycloalkyl" refers to a 5- to 20-membered polycyclic group in which each monocycle in the system shares one carbon atom (referred to as the spiro atom), and may contain one or more double bonds. The spirocycloalkyl group is preferably a 6- to 14-membered spirocycloalkyl group, and more preferably a 7- to 10-membered (*e.g*., 7-, 8-, 9- or 10-membered) spirocycloalkyl group. Based on the number of shared spiro atoms between rings, the spirocycloalkyl group is divided into monocyclic, bicyclic, or polycyclic spirocycloalkyl groups, preferably monocyclic and bicyclic spirocycloalkyl groups, and more preferably 3-membered/5-membered, 3-membered/6-membered, 4-membered/4-membered, 4-membered/5-membered, 4-membered/6-membered, 5-membered/5-membered, or 5-membered/6-membered monocyclic spirocycloalkyl groups. Non-limiting examples of spirocycloalkyl groups include:

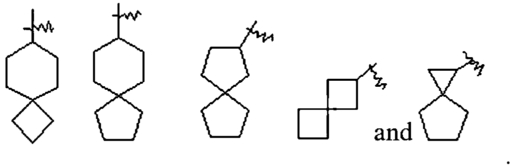

The term "fused cycloalkyl" refers to an all-carbon 5- to 20-membered polycyclic group in which each ring in the system shares an adjacent pair of carbon atoms with other rings in the system, and one or more rings may contain one or more double bonds. The fused cycloalkyl group is preferably a 6- to 14-membered fused cycloalkyl group, and more preferably a 7- to 10-membered (*e.g.*, 7-, 8-, 9- or 10-membered) fused cycloalkyl group. Based on the number of constituent rings, the fused cycloalkyl group can be divided into bicyclic, tricyclic, tetracyclic, or polycyclic fused cycloalkyl groups, preferably bicyclic or tricyclic fused cycloalkyl groups, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused cycloalkyl groups. Non-limiting examples of fused cycloalkyl groups include:

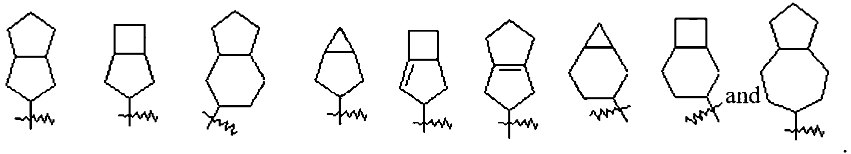

The term "bridged cycloalkyl" refers to an all-carbon 5- to 20-membered polycyclic group in which any two rings share two non-directly connected carbon atoms, and may contain one or more double bonds. The bridged cycloalkyl group is preferably a 6- to 14-membered bridged cycloalkyl group, and more preferably a 7- to 10-membered (*e.g*., 7-, 8-, 9- or 10-membered) bridged cycloalkyl group. Based on the number of constituent rings, the bridged cycloalkyl group can be divided into bicyclic, tricyclic, tetracyclic, or polycyclic bridged cycloalkyl groups, preferably bicyclic, tricyclic, or tetracyclic bridged cycloalkyl groups, and more preferably bicyclic or tricyclic bridged cycloalkyl groups. Non-limiting examples of bridged cycloalkyl groups include:

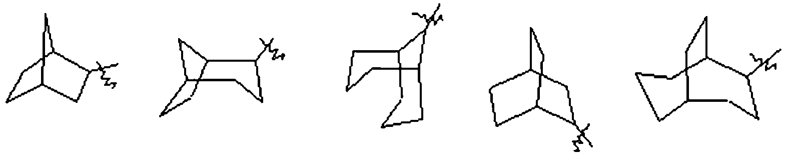

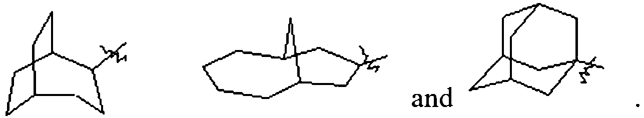

The cycloalkyl ring includes the cycloalkyl ring as described herein (including monocyclic, spiro, fused, and bridged rings) fused to an aryl, heteroaryl, or heterocycloalkyl ring, wherein the ring attached to the parent structure is the cycloalkyl ring. Non-limiting examples include 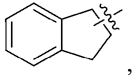 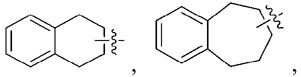 *etc*.; preferably 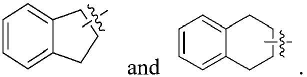 The cycloalkyl group may be substituted or unsubstituted.

The term "heterocyclyl" or "heterocycloalkyl" refers to a saturated or partially unsaturated monocyclic or polycyclic substituent containing 3 to 20 ring atoms, wherein one or more ring atoms are heteroatoms selected from the group consisting of nitrogen, oxygen, and sulfur (wherein the sulfur may optionally be oxidized, *i.e.,* to form sulfoxide or sulfone), excluding ring portions containing -O-O-, - O-S-, or -S-S-, with the remaining ring atoms being carbon. Preferably, the heterocyclyl group contains 3 to 12 (*e.g.,* 3, 4, 5, 6, 7, 8, 9, 10, 11, and 12) ring atoms, of which 1 to 4 (*e.g.,* 1, 2, 3, and 4) are heteroatoms; more preferably, the heterocyclyl group contains 3 to 8 (*e.g.,* 3, 4, 5, 6, 7, and 8) ring atoms, of which 1 to 3 (*e.g.,* 1, 2, and 3) are heteroatoms; even more preferably, the heterocyclyl group contains 3 to 6 ring atoms, of which 1 to 3 are heteroatoms; and most preferably, the heterocyclyl group contains 5 or 6 ring atoms, of which 1 to 3 are heteroatoms. Non-limiting examples of monocyclic heterocyclyl groups include pyrrolidinyl, tetrahydropyranyl, 1,2,3,6-tetrahydropyridinyl, piperidinyl, piperazinyl, morpholinyl, thiomorpholinyl, homopiperazinyl, *etc.* Polycyclic heterocyclyl groups include spiro, fused, and bridged heterocyclyl groups.

The term "spiroheterocyclyl" refers to a 5- to 20-membered polycyclic heterocyclyl group in which each monocycle in the system shares one atom (referred to as the spiro atom), wherein one or more ring atoms are heteroatoms selected from the group consisting of nitrogen, oxygen, and sulfur (wherein the sulfur may optionally be oxidized, *i.e.,* to form sulfoxide or sulfone), with the remaining ring atoms being carbon. The spiroheterocyclyl group may contain one or more double bonds. The spiroheterocyclyl group is preferably a 6- to 14-membered spiroheterocyclyl group, and more preferably a 7- to 10-membered (*e.g*., 7-, 8-, 9- or 10-membered) spiroheterocyclyl group. Based on the number of shared spiro atoms between rings, the spiroheterocyclyl group is divided into monocyclic, bicyclic, or polycyclic spiroheterocyclyl groups, preferably monocyclic and bicyclic spiroheterocyclyl groups, and more preferably 3-membered/5-membered, 3-membered/6-membered, 4-membered/4-membered, 4-membered/5-membered, 4-membered/6-membered, 5-membered/5-membered, or 5-membered/6-membered monocyclic spiroheterocyclyl groups. Non-limiting examples of spiroheterocyclyl groups include:

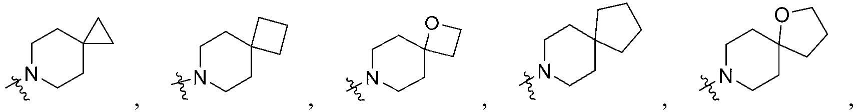

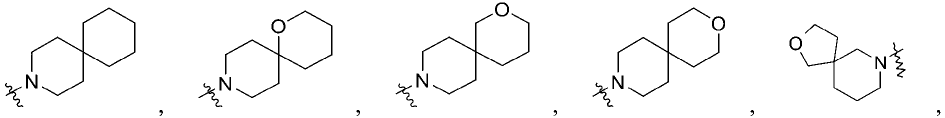

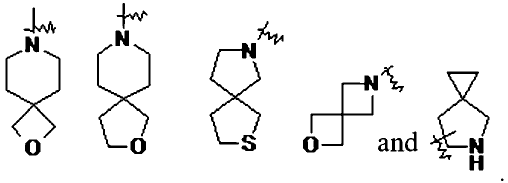

The term "fused heterocyclyl" refers to a 5- to 20-membered polycyclic heterocyclyl group in which each ring in the system shares an adjacent pair of atoms with other rings in the system, and one or more rings may contain one or more double bonds, wherein one or more ring atoms are heteroatoms selected from the group consisting of nitrogen, oxygen, and sulfur (wherein the sulfur may optionally be oxidized, *i.e.,* to form sulfoxide or sulfone), with the remaining ring atoms being carbon. The fused heterocyclyl group is preferably a 6- to 14-membered fused heterocyclyl group, and more preferably a 7- to 10-membered (*e.g.*, 7-, 8-, 9- or 10-membered) fused heterocyclyl group. Based on the number of constituent rings, the fused heterocyclyl group can be divided into bicyclic, tricyclic, tetracyclic, or polycyclic fused heterocyclyl groups, preferably bicyclic or tricyclic fused heterocyclyl groups, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused heterocyclyl groups. Non-limiting examples of fused heterocyclyl groups include:

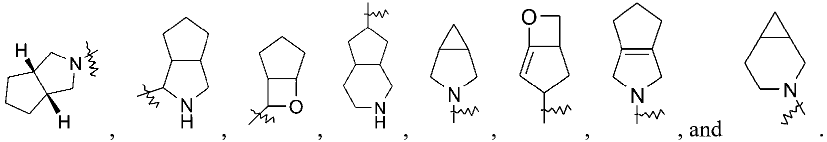

The term "bridged heterocyclyl" refers to a 5- to 14-membered polycyclic heterocyclyl group in which any two rings share two non-directly connected atoms, and may contain one or more double bonds, wherein one or more ring atoms are heteroatoms selected from the group consisting of nitrogen, oxygen, and sulfur (wherein the sulfur may optionally be oxidized, *i.e.,* to form sulfoxide or sulfone), with the remaining ring atoms being carbon. The bridged heterocyclyl group is preferably a 6- to 14-membered bridged heterocyclyl group, and more preferably a 7- to 10-membered (*e.g.*, 7-, 8-, 9- or 10-membered) bridged heterocyclyl group. Based on the number of constituent rings, the bridged heterocyclyl group can be divided into bicyclic, tricyclic, tetracyclic, or polycyclic bridged heterocyclyl groups, preferably bicyclic, tricyclic, or tetracyclic bridged heterocyclyl groups, and more preferably bicyclic or tricyclic bridged heterocyclyl groups. Non-limiting examples of bridged heterocyclyl groups include:

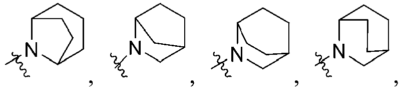

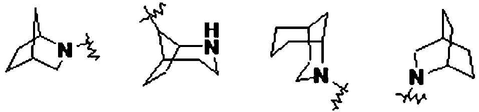

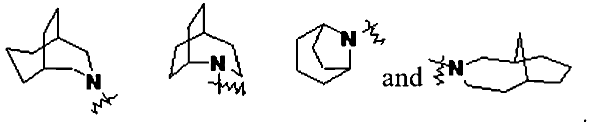

The heterocyclyl ring includes the heterocyclyl ring as described herein (including monocyclic, spiro, fused, and bridged heterocycles) fused to an aryl, heteroaryl, or cycloalkyl ring, wherein the ring attached to the parent structure is the heterocyclyl ring. Non-limiting examples include: 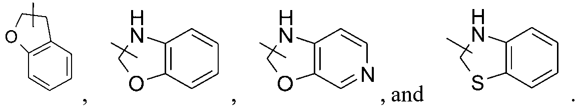 The heterocyclyl group may be substituted or unsubstituted.

The term "aryl" refers to an all-carbon 6- to 14-membered monocyclic or fused polycyclic group (fused polycyclic group refers to rings sharing adjacent pairs of carbon atoms) with a conjugated π-electron system, preferably 6- to 10-membered, such as phenyl and naphthyl. The aryl ring includes the aryl ring as described herein fused to a heteroaryl, heterocyclyl, or cycloalkyl ring, wherein the ring attached to the parent structure is the aryl ring. Non-limiting examples include: 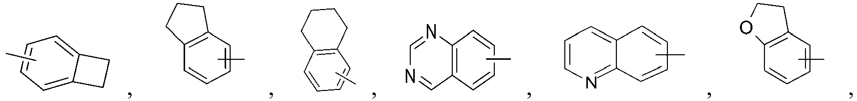 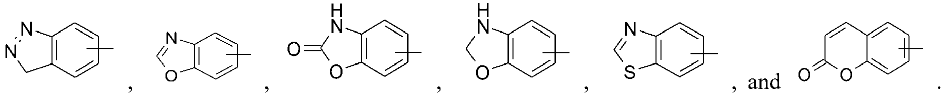 The aryl group may be substituted or unsubstituted.

The term "heteroaryl" refers to a heteroaromatic system containing 1 to 4 *(e.g.,* 1, 2, 3, and 4) heteroatoms and 5 to 14 ring atoms, wherein the heteroatoms are selected from the group consisting of oxygen, sulfur, and nitrogen. The heteroaryl group is preferably a 5- to 10-membered *(e.g.,* 5-, 6-, 7-, 8-, 9-, or 10-membered) heteroaryl group, more preferably a 5-membered or 6-membered heteroaryl group, such as furanyl, thienyl, pyridinyl, pyrrolyl, *N*-alkylpyrrolyl, pyrimidinyl, pyrazinyl, pyridazinyl, imidazolyl, pyrazolyl, triazolyl, tetrazolyl, *etc.* The heteroaryl ring includes the heteroaryl ring as described herein fused to an aryl, heterocyclyl, or cycloalkyl ring, wherein the ring attached to the parent structure is the heteroaryl ring. Non-limiting examples include: 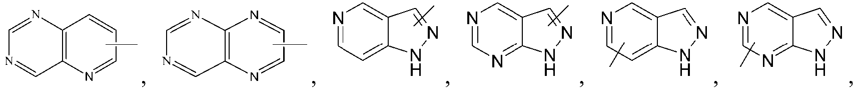 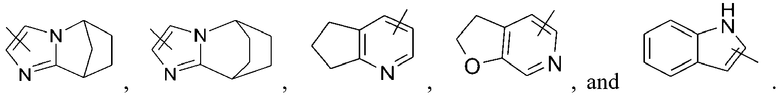 The heteroaryl group may be substituted or unsubstituted.

The terms "alkyl", "alkoxy", "cycloalkyl", "heterocyclyl", "aryl", "heteroaryl", and the like herein may be substituted or unsubstituted. When substituted, they may be substituted at any available connecting point, and the substituents are preferably independently and optionally selected from the group consisting of one or more identical or different substituents, including, but not limited to, halogen, alkyl, alkoxy, haloalkyl, haloalkoxy, hydroxyl, hydroxyalkyl, cyano, amino, nitro, cycloalkyl, heterocyclyl, aryl, and heteroaryl.

The cycloalkyl, heterocyclyl, aryl, and heteroaryl groups include residues derived by removing one hydrogen atom from a parent ring atom, or residues derived by removing two hydrogen atoms from the same or two different ring atoms of the parent ring atom, namely "divalent cycloalkyl", "divalent heterocyclyl", "arylene", and "heteroarylene".

The term "amino protecting group" refers to a group that is easily removable and introduced onto an amino group, such that the amino group remains unchanged while other parts of the compound undergo reactions. Non-limiting examples include: (trimethylsilyl)ethoxymethyl, tetrahydropyranyl, *tert*-butoxycarbonyl (Boc), acetyl, *p*-toluenesulfonyl (Ts), benzyl, allyl, *p*-methoxybenzyl, *etc.* These groups may be optionally substituted by 1 to 3 substituents selected from the group consisting of halogen, alkoxy, and nitro.

The term "cycloalkyloxy" refers to cycloalkyl-O-, wherein the cycloalkyl is as defined herein.

The term "heterocyclyloxy" refers to heterocyclyl-O-, wherein the heterocyclyl is as defined herein.

The term "haloalkyl" refers to an alkyl group substituted by one or more halogens, wherein the alkyl is as defined herein.

The term "haloalkoxy" refers to an alkoxy group substituted by one or more halogens, wherein the alkoxy is as defined herein.

The term "hydroxyalkyl" refers to an alkyl group substituted by one or more hydroxyl groups, wherein the alkyl is as defined herein.

The term "halogen" refers to F, Cl, Br, or I.

The term "hydroxyl" refers to -OH.

The term "amino" refers to -NH₂.

The term "cyano" refers to -CN.

The term "nitro" refers to -NO₂.

The term "oxo group" or "oxo" refers to "=O".

The term "carbonyl" refers to C=O.

The term "carboxyl" refers to -C(O)OH.

The term "carboxylate ester group" refers to -C(O)O(alkyl) or -C(O)O(cycloalkyl), wherein the alkyl and cycloalkyl are as defined herein.

"Optional" or "optionally" means that the subsequently described event or circumstance may but does not have to occur, and that the description includes instances where the event or circumstance occurs and instances where it does not. For example, "a heterocycloalkyl group optionally substituted by an alkyl group" means that the alkyl group may but does not have to be present, and the description includes both cases where the heterocycloalkyl group is substituted by an alkyl group and where the heterocycloalkyl group is not substituted by an alkyl group.

"Substituted" refers to one or more hydrogen atoms in a group, preferably up to 5, and more preferably 1 to 3 hydrogen atoms, being independently replaced by a corresponding number of substituents. When there is a variable group on a ring, and when the variable group is optionally CH, it should be understood that the carbon atom at this position may also be substituted. For example, in formula (IIG), when X or Y is CH, R⁴ may be substituted on the corresponding carbon atom. It goes without saying that substituents are only present at their possible chemical positions, and those skilled in the art can determine (through experimentation or theory) possible or impossible substitutions without undue effort. For example, amino or hydroxyl groups with free hydrogen may be unstable when combined with carbon atoms containing unsaturated bonds.

### DETAILED DESCRIPTION OF THE PREFERRED EMBODIMENT

The technical solutions of the present disclosure will be further illustrated in detail with reference to the following specific examples. It should be understood that the following examples are merely exemplary illustrations and explanations of the present disclosure, and should not be construed as limiting the scope of the present disclosure. All techniques implemented based on the contents of the present disclosure are encompassed within the intended scope of the present disclosure.

Unless otherwise specified, the raw materials and reagents used in the following examples are commercially available or can be prepared by known methods.

The structure of compounds is determined by nuclear magnetic resonance (NMR) or/and mass spectrometry (MS). NMR chemical shifts (*δ*) are given in units of 10⁻⁶ (ppm). The NMR measurements are performed using a Bruker ASCEND^{™}-400 nuclear magnetic resonance spectrometer, using deuterated dimethyl sulfoxide (DMSO-*d₆*), deuterated chloroform (CDCl₃), and deuterated methanol (CD₃OD) as solvents, with tetramethylsilane (TMS) as the internal standard. The MS measurements are performed using Agilent 6110, Agilent 1100, Agilent 6120, and Agilent G6125B liquid chromatography-mass spectrometers.

The HPLC measurements are performed using a Shimadzu HPLC-2010C high-performance liquid chromatograph (XBRIDGE 2.1 × 50 mm, 3.5 µm chromatographic column).

Chiral HPLC analysis is performed using THARSFC X5.

The silica gel plates for thin-layer chromatography (TLC) are Yantai Qingdao GF254 silica gel plates. The specification for TLC is 0.15 mm to 0.2 mm, while the specification for thin-layer chromatographic purification of products is 0.4 mm to 0.5 mm.

Column chromatography is generally performed using Qingdao Haiyang Silica Gel 200 to 300 mesh silica gel as the carrier.

The preparative high-performance liquid chromatography is performed using Waters 2767, Waters 2545, and Chuangxin Tongheng LC3000 preparative chromatograph.

Chiral preparative column chromatography is performed using Shimadzu LC-20AP and THARSFC PREP 80.

The CombiFlash rapid preparative instrument is Combiflash Rf200 (TELEDYNE ISCO).

The pressurized hydrogenation reaction is performed using a Beijing Jiawei Kechuang Technology GCD-500G hydrogen generator.

The microwave reaction is performed using a Biotage initiator+ microwave reactor.

Unless otherwise specified in the examples, all reactions are conducted under an argon or nitrogen atmosphere.

An argon or nitrogen atmosphere refers to connecting the reaction flask to an argon or nitrogen balloon with a volume of approximately 1 liter.

A hydrogen atmosphere refers to connecting the reaction flask to a hydrogen balloon with a volume of approximately 1 liter.

Unless otherwise specified in the examples, the reaction temperature is room temperature, ranging from 20°C to 30°C.

Those skilled in the art should understand that the resolved chiral compounds can be distinguished by their retention times on a chiral chromatographic column. Therefore, the chiral compounds resolved based on the sequence of retention times are correspondingly distinguished by the numbered suffixes P1 and P2. That is, for example, suffix P1 corresponds to a chiral compound with a certain chiral structure that is eluted earlier from the chiral chromatographic column, while suffix P2 corresponds to a chiral compound with a certain chiral structure that is eluted later from the chiral chromatographic column. If the absolute configuration of a compound is listed in the structural formula, it does not imply a direct correspondence with the compounds with the numbered suffixes P1 and P2, but merely illustrates the two possible forms of the absolute configuration. The absolute configuration of the compounds with the numbered suffixes P1 and P2 is determined based on the absolute configuration objectively corresponding to the specific retention time.

Reagent names corresponding to English abbreviations:

| Reagent abbreviation | Reagent name |
|---|---|
| Boc anhydride | Di-*tert*-butyl dicarbonate |
| THF | Tetrahydrofuran |
| DMF | *N,N-Dimethylformamide* |
| H₂O | Water |
| AcOH | Acetic acid |
| Zn | Zinc powder |
| CDCl₃ | Deuterated chloroform |
| DMSO-*d₆* | Deuterated dimethyl sulfoxide |
| CD₃OD | Deuterated methanol |
| PE | Petroleum ether |
| EA | Ethyl acetate |
| DCM | Dichloromethane |
| MeOH | Methanol |
| Pd₂(dba)₃ | Tris(dibenzylideneacetone)dipalladium |
| NaOH | Sodium hydroxide |
| TMSI | Iodotrimethylsilane |
| DME | 1,2-Dimethoxyethane |
| Cbz | Benzyloxycarbonyl |
| DMA | *N,N-Dimethylacetamide* |
| i-PrMgCl·LiCl | Isopropylmagnesium chloride lithium chloride complex |
| NMP | *N*-Methylpyrrolidone |

### Examples

### Synthesis of Intermediate Compound A1

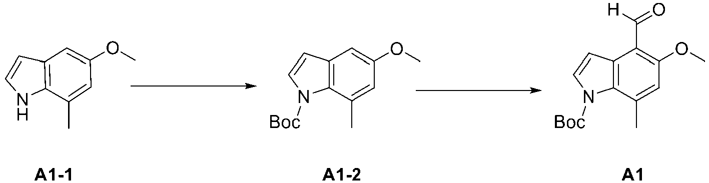

### Step 1: Synthesis of Compound A1-2

Dichloromethane (50 mL), compound A1-1 (3 g), Boc anhydride (5.68 g), 4-dimethylaminopyridine (227 mg), and triethylamine (2.26 g) were sequentially added to a 250 mL single-necked flask. The reaction mixture was stirred at room temperature for 16 hours. After the reaction was completed, the reaction mixture was quenched with saturated ammonium chloride solution (5 mL) and extracted with dichloromethane (20 mL × 3). The combined organic phases were washed with water (5 mL), dried over anhydrous sodium sulfate, filtered, and the filtrate was concentrated. The residue was purified by column chromatography (petroleum ether/ethyl acetate = 10/1) to obtain compound A1-2 (4.6 g, yield: 94%). MS m/z (ESI): 262.0 [M+1]⁺.

### Step 2: Synthesis of Compound A1

Dichloromethane (80 mL), N-methylformanilide (3.8 g), and oxalyl chloride (3.6 g) were sequentially added to a 250 mL single-necked flask. The reaction mixture was stirred at room temperature for 3 hours. The reaction temperature was then lowered to -14°C, followed by the addition of compound A1-2 (2.5 g). The reaction system was naturally warmed to room temperature and stirred at room temperature for 1 hour. After the reaction was completed, the reaction mixture was poured into ice water (100 mL) and extracted with dichloromethane (100 mL × 3). The combined extracts were washed with water (10 mL × 2), dried over anhydrous sodium sulfate, filtered, and the filtrate was concentrated. The residue was purified by column chromatography (petroleum ether/ethyl acetate = 20/1) to obtain compound A1 (1.3 g, yield: 47%). MS m/z (ESI): 290.0 [M+1]⁺. ¹H NMR (400 MHz, CDCl₃) *δ* 10.65 (s, 1H), 7.65 (d, *J* = 3.4 Hz, 1H), 7.49 (d, *J =* 3.4 Hz, 1H), 6.76 (s, 1H), 3.98 (s, 3H), 2.70 (s, 3H), 1.65 (s, 9H).

### Synthesis of Intermediate Compound A2

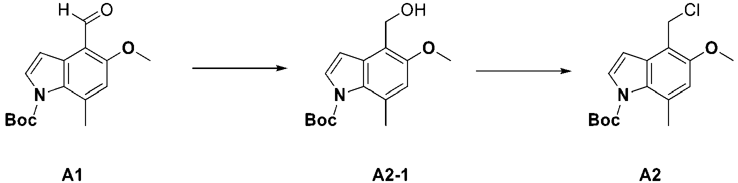

### Step 1: Synthesis of Compound A2-1

Sodium borohydride (392 mg, 10.4 mmol) was added to a solution of compound A1 (1.5 g, 5.18 mmol) in methanol (15 mL) at 0°C. The reaction mixture was stirred at room temperature for 1 hour. The reaction mixture was quenched with water (10 mL) and extracted with dichloromethane (10 mL × 3). The combined organic phases were washed with saturated sodium chloride aqueous solution (10 mL × 2), dried over anhydrous sodium sulfate, filtered, and the filtrate was concentrated under reduced pressure. The residue was purified by silica gel column chromatography (petroleum ether/ethyl acetate = 1/10 to 1/2) to obtain compound A2-1 (1.1 g, yield: 72.8%). MS m/z (ESI): 274.05 [M-17]⁺.

### Step 2: Synthesis of Compound A2

(Chloromethylene)dimethylammonium chloride (879 mg, 6.87 mmol) was added to a solution of compound A2-1 (1.1 g, 3.78 mmol) in dichloromethane (25 mL). The reaction mixture was stirred at room temperature for 1 hour under a nitrogen atmosphere. After the reaction was completed, the reaction mixture was added with saturated sodium bicarbonate solution (10 mL) and extracted with dichloromethane (10 mL × 3). The combined organic phases were washed with saturated sodium chloride aqueous solution (10 mL × 2), dried over anhydrous sodium sulfate, filtered, and the filtrate was concentrated under reduced pressure to obtain compound A2 (1.23 g, crude product). The crude product was directly used in the next step.

### Example 1 (Compound 1)

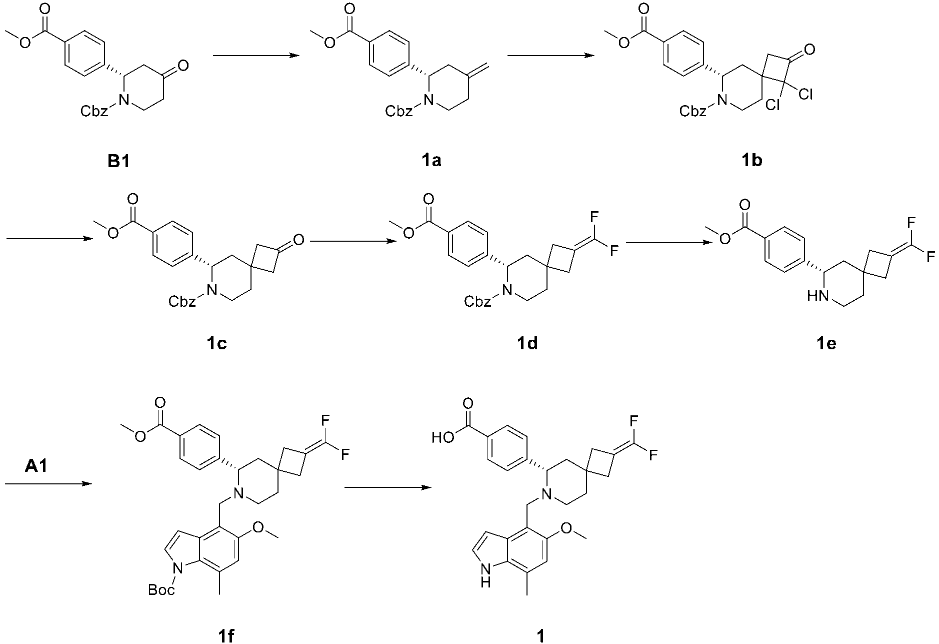

### Step 1: Synthesis of Compound 1a

Potassium tert-butoxide (16.3 mL, 16.3 mmol) was added to a solution of methyltriphenylphosphonium bromide (5.84 g, 16.3 mmol) in THF (100 mL) at 0°C. The mixture was stirred at 30°C for 30 minutes, then cooled to 0°C, followed by the addition of a solution of compound B1 (2 g, 5.4 mmol, CAS: 2408761-20-4) in THF (20 mL). The reaction mixture was stirred at 0°C for 1 hour. After the reaction was completed, the reaction mixture was concentrated under reduced pressure. The residue was diluted with water (100 mL) and extracted with ethyl acetate (100 mL × 3). The combined organic phases were concentrated under reduced pressure, and the residue was purified by silica gel column chromatography (PE/EA = 10/1 to 5/1) to obtain compound 1a (1.17 g, yield: 58.8%). MS m/z (ESI): 366.3 [M+1]⁺.

### Step 2: Synthesis of Compound 1b

A solution of trichloroacetyl chloride (24.5 g, 0.13 mmol) in DME (30 mL) was slowly added dropwise to a solution of compound 1a (3.26 g, 8.93 mmol) and zinc-copper reagent (17.3 g, 0.13 mmol) in DME (90 mL) at room temperature. The reaction mixture was stirred at 30°C for 3 hours. After the reaction was completed, the reaction mixture was quenched with saturated sodium bicarbonate solution, filtered, and the filtrate was extracted with ethyl acetate (100 mL × 3). The combined organic phases were concentrated under reduced pressure to obtain compound 1b (4.3 g, crude product). MS m/z (ESI): 475.9 [M+1]⁺.

### Step 3: Synthesis of Compound 1c

Zinc powder (8.70 g, 0.13 mol) was added to a solution of compound 1b (4.3 g, crude product) in acetic acid (100 mL) at room temperature. The reaction mixture was stirred at 80°C for 16 hours. After the reaction was completed, the reaction mixture was filtered, and the filtrate was concentrated under reduced pressure. The residue was diluted with water (50 mL), then added with saturated sodium bicarbonate solution to adjust the pH to 8, and extracted with ethyl acetate (100 mL × 3). The combined organic phases were concentrated under reduced pressure, and the residue was purified by silica gel column chromatography (PE/EA = 5/1 to 3/1) to obtain compound 1c (3.61 g, two-step yield: 99.2%).

### Step 4: Synthesis of Compound 1d

Dibromodifluoromethane (4.44 g, 21.13 mmol) was added to THF (60 mL) at 0°C. The mixture was stirred at 0°C for 10 minutes, followed by the addition of tris(dimethylamino)phosphine (3.40 g, 20.64 mmol). The mixture was stirred at 0°C for another 1 hour, followed by the addition of compound 1c (2.00 g, 4.91 mmol). The reaction mixture was slowly warmed to room temperature, stirred at room temperature for 40 minutes, followed by the addition of zinc powder (1.30 g, 19.66 mmol) and tris(dimethylamino)phosphine (280 mg, 2.06 mmol). The reaction mixture was stirred at 85°C for 7 hours. After the reaction was completed, the reaction mixture was diluted with water (60 mL) and extracted with ethyl acetate (100 mL × 3). The combined organic phases were concentrated under reduced pressure, and the residue was purified by silica gel column chromatography (PE/EA = 20/1 to 5/1) to obtain compound 1d (1.26 g, yield: 58.1%). MS m/z (ESI): 442.3 [M+1]⁺.

### Step 5: Synthesis of Compound 1e

TMSI (1.36 g, 6.80 mmol) was added to a solution of compound 1d (600 mg, 1.36 mmol) in DCM (12 mL) at 0°C. The reaction mixture was stirred at room temperature for 4 hours. After the reaction was completed, the reaction mixture was added with saturated sodium bicarbonate solution to adjust the pH to more than 7, and extracted with ethyl acetate (50 mL × 3). The combined organic phases were concentrated under reduced pressure, and the residue was purified by silica gel column chromatography (DCM/MeOH = 50/1 to 20/1) to obtain compound 1e (220 mg, yield: 52.6%). MS m/z (ESI): 308.2 [M+1]⁺.

### Step 6: Synthesis of Compound 1f

Sodium triacetoxyborohydride (580 mg, 2.74 mmol) was added to a solution of compound 1e (210 mg, 0.68 mmol) and compound A1 (297 mg, 1.03 mmol) in THF (3 mL) at room temperature. The reaction mixture was stirred at 40°C for 16 hours. After the reaction was completed, the reaction mixture was quenched with saturated sodium bicarbonate solution (30 mL) and extracted with ethyl acetate (30 mL × 3). The combined organic phases were concentrated under reduced pressure, and the residue was purified by silica gel column chromatography (PE/EA = 50/1 to 10/1) to obtain compound 1f (150 mg, yield: 37.8%). MS m/z (ESI): 581.5 [M+1]⁺.

### Step 7: Synthesis of Compound 1

NaOH (48 mg, 1.20 mmol) was added to a mixed solution of compound 1f (70 mg, 0.12 mmol) in methanol, tetrahydrofuran, and water (1 mL/1 mL/1 mL). The reaction mixture was stirred at 40°C for 16 hours. After the reaction was completed, the reaction mixture was added with glacial acetic acid to adjust the pH to 5, and concentrated under reduced pressure to remove methanol and tetrahydrofuran. The resulting mixture was filtered, and the filter cake was purified by preparative high-performance liquid chromatography (chromatographic column: Xbridge-C18; 19 × 150 mm, 5 µm; mobile phase: acetonitrile-water (0.1% ammonium bicarbonate); gradient: 20 to 70%; column temperature: 25°C; flow rate: 15 mL/min; wavelength: 214 nm; column pressure: 80 bar) to obtain compound 1 (19.6 mg, yield: 35.1%). MS m/z (ESI): 467.1 [M+1]⁺. ¹H NMR (400 MHz, DMSO-*d₆*): δ 10.82 (s, 1H), 7.96 (d, *J =* 8.0 Hz, 2H), 7.67 (d, *J =* 7.6 Hz, 2H), 7.25 (s, 1H), 6.65 (s, 1H), 6.46 (s, 1H), 3.70 (s, 3H), 3.52 (d, *J =* 11.6 Hz, 1H), 3.23-3.12 (m, 2H), 2.66-2.60 (m, 2H), 2.43-2.31 (m, 6H), 2.01-1.93 (m, 1H), 1.78-1.41 (m, 4H).

### Example 2 (Compound 19)

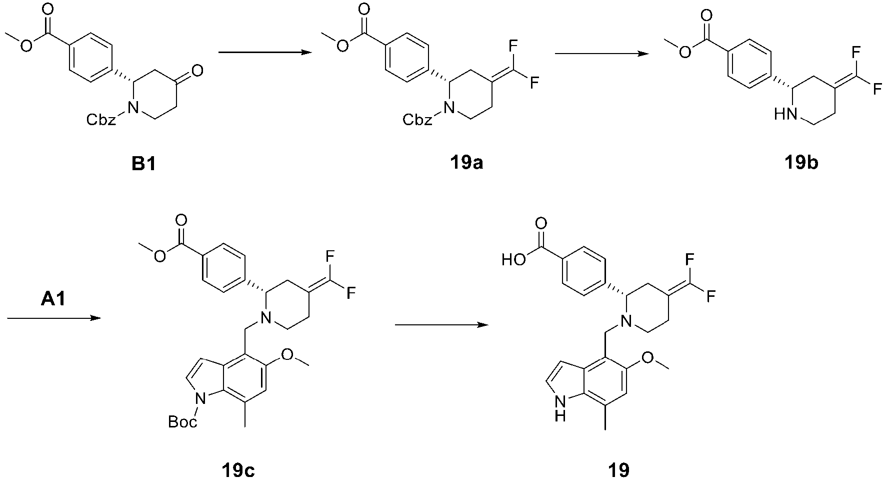

### Step 1: Synthesis of Compound 19a

Dibromodifluoromethane (3.7 g, 17.57 mmol) was added to THF (50 mL) at 0°C. The mixture was stirred at 0°C for 10 minutes, followed by the addition of tris(dimethylamino)phosphine (2.8 g, 17.57 mmol). The mixture was stirred at 0°C for another 1 hour, followed by the addition of compound B1 (1.50 g, 4.09 mmol, CAS: 2408761-20-4). The reaction mixture was slowly warmed to room temperature, stirred at room temperature for 40 minutes, followed by the addition of zinc powder (1.06 g, 16.35 mmol) and tris(dimethylamino)phosphine (168 mg, 1.03 mmol). The reaction mixture was stirred at 85°C for 3 hours. After the reaction was completed, the reaction mixture was diluted with water (50 mL) and extracted with ethyl acetate (100 mL × 3). The combined organic phases were concentrated under reduced pressure, and the residue was purified by silica gel column chromatography (PE/EA = 20/1 to 5/1) to obtain compound 19a (1.1 g, yield: 68.8%). MS m/z (ESI): 402.0 [M+1]⁺.

### Step 2: Synthesis of Compound 19b

TMSI (2.60 g, 13.06 mmol) was added to a solution of compound 19a (1.05 g, 2.61 mmol) in DCM (30 mL) at 0°C. The reaction mixture was stirred at room temperature for 16 hours. After the reaction was completed, the reaction mixture was added with saturated sodium bicarbonate solution to adjust the pH to more than 7, and extracted with ethyl acetate (100 mL × 3). The combined organic phases were purified by silica gel column chromatography (DCM/MeOH = 50/1 to 20/1) to obtain compound 19b (500 mg, yield: 71.2%). MS m/z (ESI): 268.1 [M+1]⁺.

### Step 3: Synthesis of Compound 19c

Sodium triacetoxyborohydride (635 mg, 3.00 mmol) was added to a solution of compound 19b (200 mg, 0.75 mmol) and compound A1 (325 mg, 1.12 mmol) in THF (3 mL) at room temperature. The reaction mixture was stirred at 40°C for 16 hours. After the reaction was completed, the reaction mixture was quenched with saturated sodium bicarbonate solution and extracted with ethyl acetate (30 mL × 3). The combined organic phases were concentrated under reduced pressure, and the residue was purified by silica gel column chromatography (PE/EA = 50/1 to 10/1) to obtain compound 19c (110 mg, yield: 27.2%). MS m/z (ESI): 541.3 [M+1]⁺.

### Step 4: Synthesis of Compound 19

NaOH (74 mg, 1.85 mmol) was added to a mixed solution of compound 19c (100 mg, 0.19 mmol) in methanol, tetrahydrofuran, and water (1 mL/1 mL/1 mL). The reaction mixture was stirred at 40°C for 16 hours. After the reaction was completed, the reaction mixture was added with glacial acetic acid to adjust the pH to approximately 5, and concentrated under reduced pressure. The residue was purified by preparative high-performance liquid chromatography (chromatographic column: Xbridge-C18; 19 × 150 mm, 5 µm; mobile phase: acetonitrile-water (0.1% ammonium bicarbonate); gradient: 20 to 50%; column temperature: 25°C; flow rate: 15 mL/min; wavelength: 214 nm; column pressure: 80 bar) to obtain compound 19 (54.8 mg, yield: 69.4%). MS m/z (ESI): 427.1 [M+1]⁺. ¹H NMR (400 MHz, DMSO-*d₆*): δ 10.84 (s, 1H), 7.98 (d, *J =* 8.0 Hz, 2H), 7.69 (d, *J =* 8.4 Hz, 2H), 7.26 (t, *J =* 3.0 Hz, 1H), 6.66 (s, 1H), 6.45 (t, *J =* 2.2 Hz, 1H), 3.71 (s, 3H), 3.57-3.31 (m, 1H), 3.30-3.17 (m, 2H), 2.87-2.82 (m, 1H), 2.43-2.35 (m, 4H), 2.27-2.14 (m, 2H), 2.01-1.96 (m, 2H).

### Example 3 (Compounds 22 and 23)

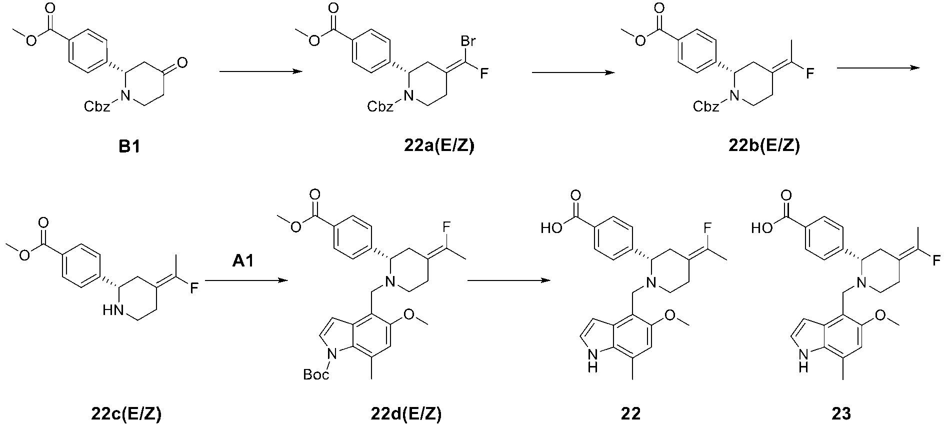

### Step 1: Synthesis of Compound 22a

Diethylzinc (4.1 mL, 8.17 mmol) was added to a solution of compound B1 (1 g, 2.72 mmol, CAS: 2408761-20-4), tribromofluoromethane (2.20 g, 8.17 mmol), and triphenylphosphine (2.10 g, 8.17 mmol) in THF (60 mL) at 0°C. The reaction mixture was stirred at room temperature for 2 hours. After the reaction was completed, the reaction mixture was added with MeOH (20 mL) and stirred for 30 minutes, then diluted with water (60 mL), and extracted with ethyl acetate (100 mL × 3). The combined organic phases were concentrated under reduced pressure, and the residue was purified by silica gel column chromatography (PE/EA= 40/1 to 10/1) to obtain compound 22a (1.25 g, yield: 96.2%, mixture of (E) and (Z) isomers). MS m/z (ESI): 462.1 [M+1]⁺.

### Step 2: Synthesis of Compound 22b

XantPhos-Pd-G2 (213 mg, 0.24 mmol, CAS: 1375325-77-1) and Pd₂(dba)₃ (220 mg, 0.24 mmol) were added to a mixed solution of compound 22a (1.10 g, 2.38 mmol, mixture of (E) and (Z) isomers), methylboronic acid (429 mg, 7.14 mmol), and sodium carbonate (505 mg, 4.76 mmol) in DMF and water (40 mL/10 mL) at room temperature. The reaction mixture was stirred at 80°C for 6 hours. After the reaction was completed, the reaction mixture was diluted with water (200 mL) and extracted with ethyl acetate (200 mL × 3). The combined organic phases were concentrated under reduced pressure, and the residue was purified by silica gel column chromatography (PE/EA = 40/1 to 10) to obtain compound 22b (200 mg, yield: 21.1%, mixture of (E) and (Z) isomers). MS m/z (ESI): 398.3 [M+1]⁺.

### Step 3: Synthesis of Compound 22c

TMSI (272 mg, 1.86 mmol) was added to a solution of compound 22b (470 mg, 1.18 mmol, mixture of (E) and (Z) isomers) in DCM (10 mL) at 0°C. The reaction mixture was stirred at 0°C for 2 hours. After the reaction was completed, the reaction mixture was added with saturated sodium bicarbonate solution to adjust the pH to more than 7, and extracted with ethyl acetate (50 mL × 3). The combined organic phases were purified by silica gel column chromatography (DCM/MeOH = 50/1 to 20/1) to obtain compound 22c (130 mg, yield: 41.8%, mixture of (E) and (Z) isomers). MS m/z (ESI): 264.2 [M+1]⁺.

### Step 4: Synthesis of Compound 22d

Sodium triacetoxyborohydride (388 mg, 1.83 mmol) was added to a solution of compound 22c (120 mg, 0.46 mmol, mixture of (E) and (Z) isomers) and compound A1 (199 mg, 0.69 mmol) in THF (2 mL) at room temperature. The reaction mixture was stirred at 40°C for 16 hours. After the reaction was completed, the reaction mixture was quenched with saturated sodium bicarbonate solution and extracted with ethyl acetate (30 mL × 3). The combined organic phases were concentrated under reduced pressure, and the residue was purified by silica gel column chromatography (PE/EA = 20/1 to 5/1) to obtain compound 22d (110 mg, yield: 44.9%, mixture of (E) and (Z) isomers) as a white solid product. MS m/z (ESI): 537.4 [M+1]⁺. Chiral HPLC: retention time = 4.814 min, 5.510 min.

### Step 5: Synthesis of Compounds 22 and 23

NaOH (74 mg, 1.86 mmol) was added to a mixed solution of compound 22d (100 mg, 0.19 mmol, mixture of (E) and (Z) isomers) in methanol, tetrahydrofuran, and water (1 mL/1 mL/1 mL). The reaction mixture was stirred at 40°C for 16 hours. After the reaction was completed, the reaction mixture was added with glacial acetic acid to adjust the pH to 5, and concentrated under reduced pressure. The residue was purified by preparative high-performance liquid chromatography (chromatographic column: Xbridge-C18; 19 × 150 mm, 5 µm; mobile phase: acetonitrile-water (0.1% ammonium bicarbonate); gradient: 20 to 70%; column temperature: 25°C; flow rate: 15 mL/min; wavelength: 214 nm; column pressure: 80 bar) to obtain compounds 22 and 23 (36.5 mg, yield: 46.2%, mixture of (E) and (Z) isomers). MS m/z (ESI): 423.1 [M+1]⁺. ¹H NMR (400 MHz, DMSO-*d₆*): δ 10.83 (s, 1H), 7.98 (d, *J=* 8.0 Hz, 2H), 7.72-7.66 (m, 2H), 7.26 (s, 1H), 6.65 (s, 1H), 6.44 (s, 1H), 3.70 (s, 3H), 3.58-3.52 (m, 1H), 3.20-3.15 (m, 2H), 2.87-2.65 (m, 2H), 2.43-2.30 (m, 4H), 2.21-2.15 (m, 1H), 2.01-1.72 (m, 5H).

### Example 4 (Compounds 26 and 27)

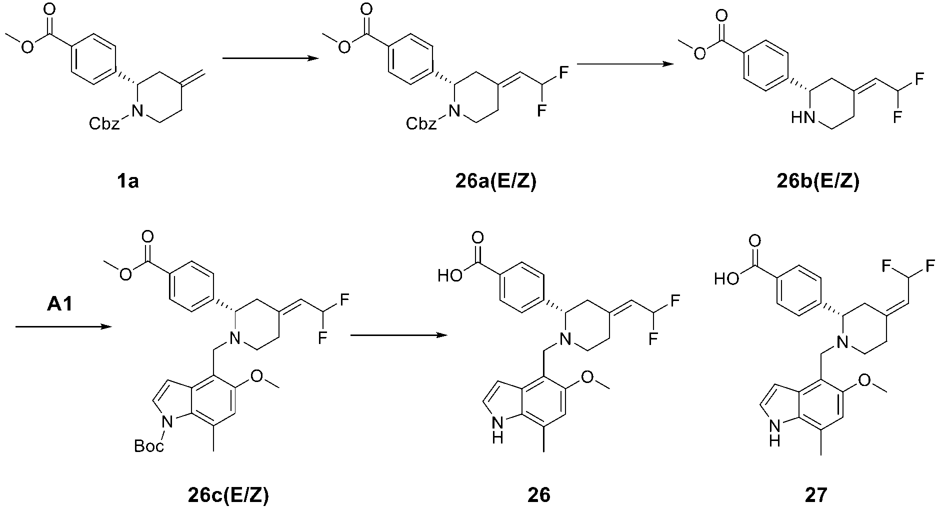

### Step 1: Synthesis of Compound 26a

Triphenylphosphine (372 mg, 1.42 mmol) was added to a solution of compound 1a (2.59 g, 7.09 mmol), (difluoromethyl)triphenylphosphonium bromide (5.0 g, 12.76 mmol), and tris(2-phenylpyridine)iridium (232 mg, 0.35 mmol) in DMA (70 mL) at room temperature. The reaction mixture was stirred under irradiation with a blue LED (AC220V, 450 to 460 nm, Xuzhou Aijia Electronic Technology Co., Ltd.) for 24 hours. After the reaction was completed, the reaction mixture was diluted with water (400 mL) and extracted with ethyl acetate (400 mL × 3). The combined organic phases were concentrated under reduced pressure, and the residue was purified by silica gel column chromatography (PE/EA = 40/1 to 10/1) to obtain compound 26a (2.50 g, yield: 84.5%, mixture of (E) and (Z) isomers). MS m/z (ESI): 416.2 [M+1]⁺.

### Step 2: Synthesis of Compound 26b

TMSI (6 g, 30.05 mmol) was added to a solution of compound 26a (2.50 g, 6.01 mmol, mixture of (E) and (Z) isomers) in DCM (50 mL) at 0°C. The reaction mixture was stirred at room temperature for 3 hours. After the reaction was completed, the reaction mixture was added with saturated sodium bicarbonate solution to adjust the pH to more than 7, and extracted with ethyl acetate (200 mL × 3). The combined organic phases were concentrated under reduced pressure, and the residue was purified by silica gel column chromatography (DCM/MeOH = 50/1 to 20/1) to obtain compound 26b (1.10 g, yield: 65.1%, mixture of (E) and (Z) isomers). MS m/z (ESI): 282.1 [M+1]⁺.

### Step 3: Synthesis of Compound 26c

Sodium triacetoxyborohydride (3.30 g, 15.66 mmol) was added to a solution of compound 26b (1.10 g, 3.91 mmol, mixture of (E) and (Z) isomers) and compound A1 (1.70 g, 5.87 mmol) in THF (20 mL) at room temperature. The reaction mixture was stirred at 40°C for 16 hours. After the reaction was completed, the reaction mixture was quenched with saturated sodium bicarbonate solution and extracted with ethyl acetate (40 mL × 3). The combined organic phases were concentrated under reduced pressure, and the residue was purified by silica gel column chromatography (PE/EA = 50/1 to 10/1) to obtain compound 26c (75 mg, yield: 3.5%, mixture of (E) and (Z) isomers). MS m/z (ESI): 555.4 [M+1]⁺. Chiral HPLC: retention time = 4.822 min, 4.963 min.

### Step 4: Synthesis of Compounds 26 and 27

NaOH (54 mg, 1.35 mmol) was added to a mixed solution of compound 26c (75 mg, 0.14 mmol, mixture of (E) and (Z) isomers) in methanol, tetrahydrofuran, and water (1 mL/1 mL/1 mL). The reaction mixture was stirred at 40°C for 16 hours. After the reaction was completed, the reaction mixture was added with glacial acetic acid to adjust the pH to approximately 5, and concentrated under reduced pressure. The residue was purified by preparative high-performance liquid chromatography (chromatographic column: Xbridge-C18; 19 × 150 mm, 5 µm; mobile phase: acetonitrile-water (0.1% formic acid); gradient: 25 to 70%; column temperature: 25°C; flow rate: 15 mL/min; wavelength: 214 nm; column pressure: 80 bar) to obtain compounds 26 and 27 (24.2 mg, yield: 40.3%, mixture of (E) and (Z) isomers). MS m/z (ESI): 441.1 [M+1]⁺. ¹H NMR (400 MHz, DMSO-*d₆*): δ 10.84 (s, 1H), 8.01-7.97 (m, 2H), 7.73-7.68 (m, 2H), 7.26 (s, 1H), 6.92-6.53 (m, 2H), 6.42 (s, 1H), 5.52-5.40 (m, 1H), 3.71 (s, 3H), 3.58-3.51 (m, 1H), 3.40-3.17 (m, 2H), 2.92-2.57 (m, 2H), 2.43-2.30 (m, 4H), 2.29-1.93 (m, 3H).

### Example 5 (Compounds 28 and 29)

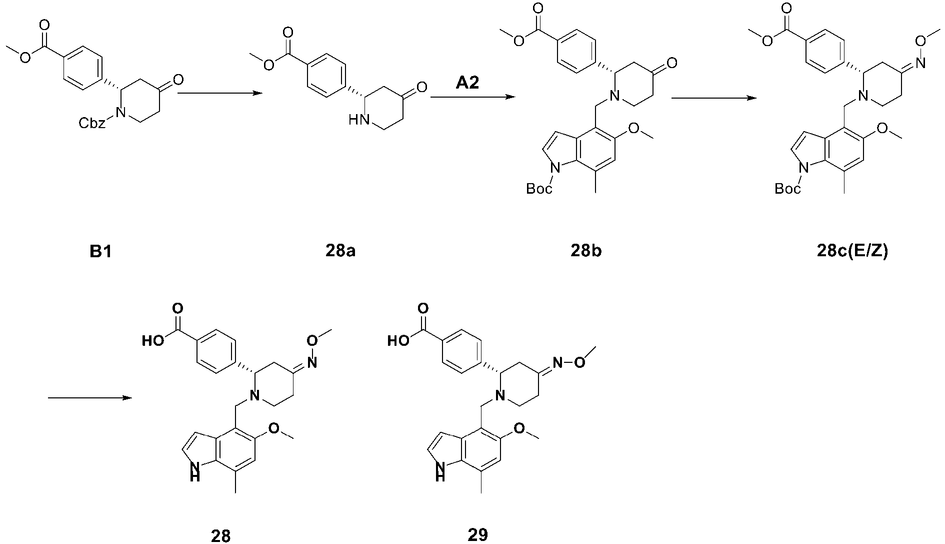

### Step 1: Synthesis of Compound 28a

Palladium on carbon (150 mg, 10%) was added to a mixed solution of compound B1 (1.5 g, 4.08 mmol) in tetrahydrofuran and ethyl acetate (8.0 mL/8.0 mL). The reaction mixture was stirred at room temperature for 16 hours under a hydrogen atmosphere. After the reaction was completed, the reaction mixture was filtered, and the filter cake was washed with chilled ethyl acetate (20 mL). The filtrate was concentrated under reduced pressure to obtain compound 28a (0.9 g, yield: 94.5%). MS m/z (ESI): 252.10 [M+19]⁺.

### Step 2: Synthesis of Compound 28b

Compound A2 (1.08 g, 3.49 mmol), potassium iodide (1.16 g, 6.97 mmol), and cesium carbonate (4.54 g, 13.9 mmol) were sequentially added to a solution of compound 28a (900 mg, 3.83 mmol) in *N,N*-dimethylformamide (35.0 mL). The reaction mixture was stirred at room temperature for 2 hours. After the reaction was completed, the reaction mixture was cooled to room temperature, diluted with water (40 mL), and extracted with ethyl acetate (30 mL × 3). The combined organic phases were washed with saturated sodium chloride aqueous solution (30 mL × 2), dried over anhydrous sodium sulfate, filtered, and the filtrate was concentrated under reduced pressure. The residue was purified by silica gel column chromatography (petroleum ether/ethyl acetate = 1/10 to 1/2) to obtain compound 28b (1.38 g, yield: 78.2%). MS m/z (ESI): 525.10 [M+19]⁺.

### Step 3: Synthesis of Compound 28c

Methylhydroxylamine hydrochloride (217 mg, 2.61 mmol) was added to a solution of compound 28b (660 mg, 1.3 mmol) and triethylamine (264 mg, 2.61 mmol) in methanol (8.0 mL). The reaction mixture was stirred at room temperature for 2 hours. After the reaction was completed, the reaction mixture was diluted with water (5 mL) and extracted with dichloromethane (10 mL × 3). The combined organic phases were washed with saturated sodium chloride aqueous solution (10 mL × 2), dried over anhydrous sodium sulfate, filtered, and the filtrate was concentrated under reduced pressure to obtain compound 28c (660 mg, crude product, mixture of (E) and (Z) isomers). MS m/z (ESI): 536.10 [M+1]⁺.

### Step 4: Synthesis of Compounds 28 and 29

Lithium hydroxide monohydrate (94.0 mg, 2.24 mmol) was added to a mixed solution of compound 28c (400 mg, 0.747 mmol, mixture of (E) and (Z) isomers) in methanol and water (6.0 mL/1.5 mL). The reaction mixture was stirred at 60°C for 16 hours. After the reaction was completed, the reaction mixture was concentrated under reduced pressure. The residue was purified by preparative high-performance liquid chromatography (preparative column: YMC Triart C18, 30 × 250 mm; flow rate: 40 mL/min; column temperature: room temperature; mobile phase: A: water (0.1% ammonia water), B: preparative grade acetonitrile) to obtain compound 28 (or compound 29) (37.5 mg, yield: 11.9%) and compound 29 (or compound 28) (50.1 mg, yield: 15.9%).

### Compound 28 (or Compound 29):

MS m/z (ESI): 422.10 [M+1]⁺. HPLC: retention time = 14.857 min, UV = 223 nm. ¹H NMR (400 MHz, DMSO-*d₆*) δ 10.84 (s, 1H), 7.97 (*d, J =* 8.0 Hz, 2H), 7.67 *(d, J=* 8.0 Hz, 2H), 7.26 (s, 1H), 6.66 (s, 1H), 6.46 (s, 1H), 3.71 (s, 6H), 3.58 (d, *J =* 12 Hz, 1H), 3.39-3.35 (m, 1H), 3.28-3.24 (m, 1H), 3.09-3.05 (m, 1H), 2.90-2.86 (m, 1H), 2.42 (s, 3H), 2.22-2.10 (m, 4H).

### Compound 29 (or Compound 28):

MS m/z (ESI): 422.10 [M+1]⁺. HPLC: retention time = 14.953 min, UV = 223 nm. ¹H NMR (400 MHz, DMSO-*d₆*) 10.86 (s, 1H), 8.00 (d, *J* = 8.0 Hz, 2H), 7.70 (*d, J =* 8.0 Hz, 2H), 7.32-7.26 (m, 1H), 6.68 (s, 1H), 6.48-6.44 (m, 1H), 3.73 (d, *J* = 1.2 Hz, 6H), 3.59 (d, *J =* 12 Hz, 1H), 3.47-3.44 (m, 1H), 3.30-3.25 (m, 1H), 2.92-2.85 (m, 2H), 2.44 (s, 3H), 2.39-2.29 (m, 2H), 2.12-2.07 (m, 1H), 1.96-1.88 (m, 1H).

### Example 6 (Compounds 30 and 31)

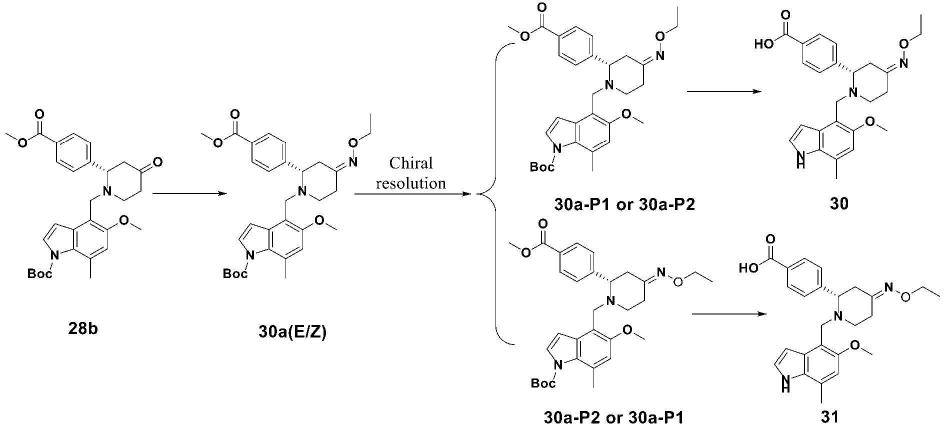

### Step 1: Synthesis of Compound 30a

Ethylhydroxylamine hydrochloride (223 mg, 2.29 mmol) was added to a solution of compound 28b (580 mg, 1.14 mmol) and triethylamine (232 mg, 2.29 mmol) in methanol (6.0 mL). The reaction mixture was stirred at room temperature for 1 hour. After the reaction was completed, the reaction mixture was diluted with water (5 mL) and extracted with dichloromethane (10 mL × 3). The combined organic phases were washed with saturated sodium chloride solution (10 mL × 2), dried over anhydrous sodium sulfate, filtered, and the filtrate was concentrated under reduced pressure. The residue was purified by silica gel column chromatography (petroleum ether/ethyl acetate = 1/10 to 1/2) to obtain compound 30a (568 mg, yield: 90.26%, mixture of (E) and (Z) isomers). Compound 30a (568 mg, mixture of (E) and (Z) isomers) was resolved by preparative chiral chromatography (preparative column: CHIRAL ART Amylose-C NEO, 30 × 250 mm; flow rate: 30 mL/min; column temperature: room temperature; mobile phase: A: n-hexane, B: ethanol 17%) to obtain compound 30a-P1 (230 mg, yield: 34.3%), MS m/z (ESI): 550.15 [M+1]⁺, chiral HPLC: retention time = 7.362 min, UV = 220 nm, and compound 30a-P2 (240 mg, yield: 35.8%), MS m/z (ESI): 550.15 [M+1]⁺, chiral HPLC: retention time = 10.590 min, UV = 220 nm.

### Step 2: Synthesis of Compounds 30 and 31

Lithium hydroxide monohydrate (52.6 mg, 1.26 mmol) was added to a mixed solution of compound 30a-P1 (230 mg, 0.418 mmol) in methanol and water (3.0 mL/1.0 mL). The reaction mixture was stirred at 60°C for 16 hours. After the reaction was completed, the reaction mixture was concentrated under reduced pressure. The residue was purified by preparative high-performance liquid chromatography (preparative column: YMC Triart C18, 30 × 250 mm; flow rate: 40 mL/min; column temperature: room temperature; mobile phase: A: water (0.1% ammonia water), B: preparative grade acetonitrile) to obtain compound 30 (or compound 31) (128.7 mg, yield: 70.6%). MS m/z (ESI): 436.10 [M+1]⁺. ¹H NMR (400 MHz, DMSO-*d₆*) δ 10.84 (s, 1H), 7.99-7.94 (m, 2H), 7.65 (d, *J* = 8.0 Hz, 2H), 7.26 (t, *J =* 2.8 Hz, 1H), 6.66 (s, 1H), 6.48-6.42 (m, 1H), 4.00-3.93 (m, 2H), 3.71 (s, 3H), 3.59 (d, *J =* 12.0 Hz, 1H), 3.39-3.34 (m, 2H), 3.10-3.04 (m, 1H), 2.90-2.85 (m, 1H), 2.42 (s, 3H), 2.23-2.12 (m, 4H), 1.15 (t, *J =* 7.2 Hz, 3H).

Lithium hydroxide monohydrate (55.0 mg, 1.31 mmol) was added to a mixed solution of compound 30a-P2 (240 mg, 0.436 mmol) in methanol and water (3.0 mL/1.0 mL). The reaction mixture was stirred at 60°C for 16 hours. After the reaction was completed, the reaction mixture was concentrated under reduced pressure. The residue was purified by preparative high-performance liquid chromatography (preparative column: YMC Triart C18, 30 × 250 mm; flow rate: 40 mL/min; column temperature: room temperature; mobile phase: A: water (0.1% ammonia water), B: preparative grade acetonitrile) to obtain compound 31 (or compound 30) (129.1 mg, yield: 67.9%), MS m/z (ESI): 436.10 [M+1]⁺. ¹H NMR (400 MHz, DMSO-*d₆*) δ 10.86 (d, *J =* 8.8 Hz, 1H), 7.94 (t, *J* = 7.6 Hz, 2H), 7.58-7.55 (m, 2H), 7.25 (s, 1H), 6.65 (s, 1H), 6.43 (s, 1H), 3.97 (q, *J =* 7.2 Hz, 2H), 3.71 (s, 3H), 3.58 (d, *J =* 11.2 Hz, 1H), 3.22 (d, *J =* 12.0 Hz, 2H), 2.91-2.84 (m, 2H), 2.42 (s, 3H), 2.40-2.29 (m, 2H), 2.10-2.02 (m, 1H), 1.93-1.85 (m, 1H), 1.15 (t, *J=* 7.2 Hz, 3H).

### Example 7 (Compounds 2 and 3)

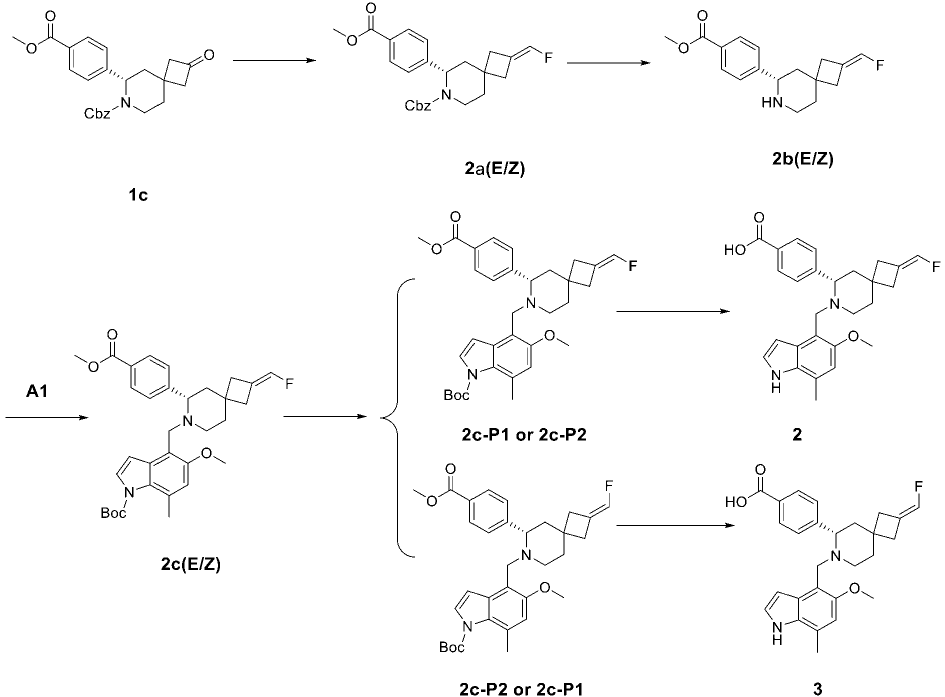

### Step 1: Synthesis of Compound 2a

Potassium *tert*-butoxide (4.5 mL, 4.5 mmol) was added to a solution of (fluoromethyl)triphenylphosphonium tetrafluoroborate (1.70 g, 4.42 mmol) in THF (15 mL) at 0°C. The reaction mixture was stirred at 30°C for 0.5 hours, then cooled to 0°C, followed by the addition of a solution of compound 1c (600 mg, 1.47 mmol) in THF (3 mL). The reaction mixture was stirred at 0°C for 40 minutes. After the reaction was completed, the reaction mixture was diluted with water (30 mL) and extracted with ethyl acetate (30 mL × 3). The combined organic phases were concentrated under reduced pressure, and the residue was purified by silica gel column chromatography (PE/EA = 10/1 to 5/1) to obtain compound 2a (369 mg, yield: 59.1%, mixture of (E) and (Z) isomers). MS m/z (ESI): 424.4 [M+1]⁺.

### Step 2: Synthesis of Compound 2b

TMSI (946 mg, 4.73 mmol) was added to a solution of compound 2a (400 mg, 0.95 mmol) in DCM (10 mL) at room temperature. The reaction mixture was stirred at room temperature for 4 hours. After the reaction was completed, the reaction mixture was added with saturated sodium bicarbonate solution to adjust the pH to more than 7, and extracted with DCM (30 mL × 3). The combined organic phases were concentrated under reduced pressure, and the residue was purified by silica gel column chromatography (DCM/MeOH = 50/1 to 10/1) to obtain compound 2b (260 mg, yield: 95.2%, mixture of (E) and (Z) isomers). MS m/z (ESI): 290.2 [M+1]⁺.

### Step 3: Synthesis of Compounds 2c-P1 and 2c-P2

Sodium triacetoxyborohydride (646 mg, 3.04 mmol) was added to a solution of compound 2b (220 mg, 0.76 mmol) and compound A1 (330 mg, 1.14 mmol) in THF (4 mL) at room temperature. The reaction mixture was stirred at 40°C for 16 hours. After the reaction was completed, the reaction mixture was quenched with saturated sodium bicarbonate solution and extracted with ethyl acetate (50 mL × 3). The combined organic phases were concentrated under reduced pressure, and the residue was purified by silica gel column chromatography (PE/EA = 20/1 to 5/1) to obtain compound 2c (195 mg, yield: 45.5%, mixture of (E) and (Z) isomers). Compound 2c (230 mg, combined from small-scale batches) was resolved by preparative chiral chromatography [equipment: Gilson GX-281; column: Chiralpark IE 250 mm × 30 mm, 10 µm; mobile phase: Hex/EtOH = 95-5; flow rate: 25 mL/min] to obtain compound 2c-P1 (90 mg, yield: 39.1%) and compound 2c-P2 (80 mg, yield: 34.8%).
Compound 2c-P1:
MS m/z (ESI): 563.6 [M+1]⁺. Chiral HPLC: retention time = 9.95 min, UV = 254 nm.
Compound 2c-P2:
MS m/z (ESI): 563.6 [M+1]⁺. Chiral HPLC: retention time = 11.36 min, UV = 254 nm.

### Step 4: Synthesis of Compounds 2 and 3

NaOH (50 mg, 1.25 mmol) was added to a mixed solution of compound 2c-P1 (70 mg, 0.12 mmol) in methanol, tetrahydrofuran, and water (1 mL/1 mL/1 mL). The reaction mixture was stirred at 40°C for 16 hours. After the reaction was completed, the reaction mixture was added with glacial acetic acid to adjust the pH to 5, and concentrated under reduced pressure. The residue was purified by preparative high-performance liquid chromatography (chromatographic column: Xbridge-C18; 19 × 150 mm, 5 µm; mobile phase: acetonitrile-water (0.1% ammonium bicarbonate); gradient: 20 to 70%; column temperature: 25°C; flow rate: 15 mL/min; wavelength: 214 nm; column pressure: 80 bar) to obtain compound 2 (or compound 3) (23.1 mg, yield: 41.8%). MS m/z (ESI): 449.2 [M+1]⁺. ¹H NMR (400 MHz, DMSO-*d₆*): δ 10.81 (s, 1H), 7.96 (d, *J =* 8.0 Hz, 2H), 7.67 (d, *J =* 7.2 Hz, 2H), 7.26-7.23 (m, 1H), 6.75-6.51 (m, 2H), 6.47 (s, 1H), 3.70 (s, 3H), 3.56-3.50 (m, 1H), 3.24-3.12 (m, 2H), 2.67-2.56 (m, 2H), 2.43-2.27 (m, 6H), 2.02-1.93 (m, 1H), 1.75-1.41 (m, 4H).

NaOH (57 mg, 1.42 mmol) was added to a mixed solution of compound 2c-P2 (80 mg, 0.14 mmol) in methanol, tetrahydrofuran, and water (1 mL/1 mL/1 mL). The reaction mixture was stirred at 40°C for 16 hours. After the reaction was completed, the reaction mixture was added with glacial acetic acid to adjust the pH to 5, and concentrated under reduced pressure. The residue was purified by preparative high-performance liquid chromatography (chromatographic column: Xbridge-C18; 19 × 150 mm, 5 µm; mobile phase: acetonitrile-water (0.1% ammonium bicarbonate); gradient: 20 to 70%; column temperature: 25°C; flow rate: 15 mL/min; wavelength: 214 nm; column pressure: 80 bar) to obtain compound 3 (or compound 2) (40.8 mg, yield: 64.7%). MS m/z (ESI): 449.2 [M+1]⁺. ¹H NMR (400 MHz, DMSO-*d₆*): δ 10.81 (s, 1H), 7.96 (d, *J =* 8.4 Hz, 2H), 7.67 (d, *J =* 7.2 Hz, 2H), 7.26-7.23 (m, 1H), 6.79-6.53 (m, 2H), 6.48-6.44 (m, 1H), 3.70 (s, 3H), 3.56-3.50 (m, 1H), 3.24-3.12 (m, 2H), 2.68-2.51 (m, 2H), 2.49-2.32 (m, 6H), 2.02-1.93 (m, 1H), 1.75-1.39 (m, 4H).

### Example 8 (Compounds 8 and 9)

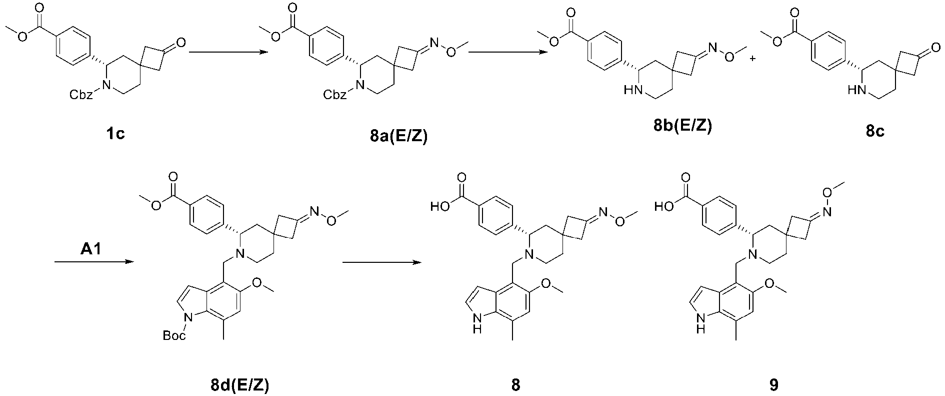

### Step 1: Synthesis of Compound 8a

Sodium acetate (406 mg, 4.95 mmol) was added to a solution of compound 1c (650 mg, 1.65 mmol) and methylhydroxylamine hydrochloride (276 mg, 3.30 mmol) in MeOH (15 mL) at room temperature. The reaction mixture was stirred at 60°C for 16 hours. After the reaction was completed, the reaction mixture was cooled to room temperature, diluted with water (20 mL), and extracted with ethyl acetate (20 mL × 3). The combined organic phases were concentrated under reduced pressure, and the residue was purified by silica gel column chromatography (PE/EA = 10/1 to 3/1) to obtain compound 8a (470 mg, yield: 65.3%, mixture of (E) and (Z) isomers). MS m/z (ESI): 437.4 [M+1]⁺.

### Step 2: Synthesis of Compound 8b

TMSI (986 mg, 4.93 mmol) was slowly added dropwise to a solution of compound 8a (430 mg, 0.98 mmol, mixture of (E) and (Z) isomers) in DCM (10 mL) under an ice bath. The reaction mixture was stirred at 30°C for 3 hours. After the reaction was completed, the reaction mixture was added with sodium bicarbonate solution to adjust the pH to more than 7, and extracted with ethyl acetate (20 mL × 3). The combined organic phases were concentrated under reduced pressure, and the residue was purified by preparative high-performance liquid chromatography (chromatographic column: Xbridge-C18; 19 × 150 mm, 5 µm; mobile phase: acetonitrile-water (0.1% ammonium bicarbonate); gradient: 25 to 75%; column temperature: 25°C; flow rate: 15 mL/min; wavelength: 214 nm; column pressure: 80 bar) to obtain compound 8b (70 mg, yield: 23.7%, mixture of (E) and (Z) isomers), MS m/z (ESI): 274.2 [M+1]⁺, and compound 8c (140 mg, yield: 52.4%), MS m/z (ESI): 303.3 [M+1]⁺.

### Step 3: Synthesis of Compound 8d

Sodium triacetoxyborohydride (168 mg, 0.79 mmol) was added to a solution of compound 8b (60 mg, 0.20 mmol, mixture of (E) and (Z) isomers) and compound A1 (80 mg, 0.27 mmol) in THF (2 mL) at room temperature. The reaction mixture was stirred at 40°C for 16 hours. After the reaction was completed, the reaction mixture was added with ethyl acetate (30 mL) and washed with saturated sodium bicarbonate solution (30 mL × 3). The organic phase was concentrated under reduced pressure, and the residue was purified by preparative high-performance liquid chromatography (chromatographic column: Xbridge-C18; 19 × 150 mm, 5 µm; mobile phase: acetonitrile-water (0.1% ammonium bicarbonate); gradient: 25 to 60%; column temperature: 25°C; flow rate: 15 mL/min; wavelength: 214 nm; column pressure: 80 bar) to obtain compound 8d (47 mg, yield: 41.2%, mixture of (E) and (Z) isomers). MS m/z (ESI): 576.6 [M+1]⁺.

### Step 4: Synthesis of Compounds 8 and 9

NaOH (17.4 mg, 0.43 mmol) was added to a mixed solution of compound 8d (25 mg, 0.043 mmol, mixture of (E) and (Z) isomers) in methanol, tetrahydrofuran, and water (0.3 mL/0.3 mL/0.3 mL). The reaction mixture was stirred at 40°C for 24 hours. After the reaction was completed, the reaction mixture was added with acetic acid to adjust the pH to 6, and concentrated under reduced pressure. The residue was purified by preparative high-performance liquid chromatography (chromatographic column: Xbridge-C18; 19 × 150 mm, 5 µm; mobile phase: acetonitrile-water (0.1% ammonium bicarbonate); gradient: 5 to 60%; column temperature: 25°C; flow rate: 15 mL/min; wavelength: 214 nm; column pressure: 80 bar) to obtain a mixture of compounds 8 and 9 (16.1 mg, yield: 80.0%). MS m/z (ESI): 462.2 [M+1]⁺. ¹H NMR (400 MHz, DMSO-*d₆*): δ 10.81 (s, 1H), 7.96 (d, *J* = 8.0 Hz, 2H), 7.68 (d, *J =* 7.2 Hz, 2H), 7.24 (t, *J =* 2.8 Hz, 1H), 6.65 (s, 1H), 6.47 (s, 1H), 3.70-3.64 (m, 6H), 3.53 (d, *J =* 12.0 Hz, 1H), 3.31-3.14 (m, 3H), 2.82-2.75 (m, 1H), 2.68-2.57 (m, 3H), 2.44 (s, 3H), 1.97-1.95 (m, 1H), 1.68-1.67 (m, 2H), 1.51-1.49 (m, 2H).

### Example 9 (Compounds 10 and 11)

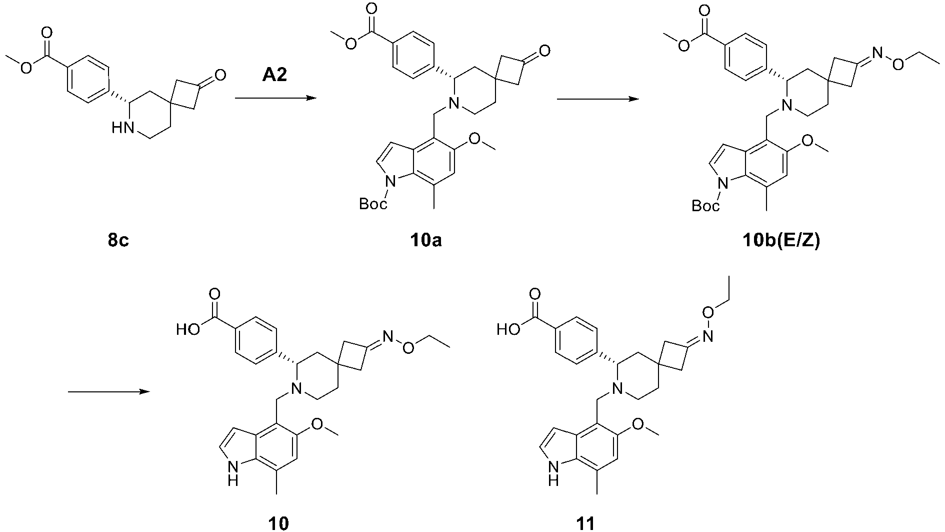

### Step 1: Synthesis of Compound 10a

Cesium carbonate (572 mg, 1.76 mmol) and potassium iodide (146 mg, 0.88 mmol) were sequentially added to a solution of compound 8c (120 mg, 0.44 mmol) and compound A2 (176 mg, 0.57 mmol) in DMF (5 mL) at room temperature. The reaction mixture was stirred at 30°C for 3 hours. After the reaction was completed, the reaction mixture was diluted with water (10 mL) and extracted with ethyl acetate (10 mL × 3). The combined organic phases were concentrated under reduced pressure, and the residue was purified by preparative high-performance liquid chromatography (chromatographic column: Xbridge-C18; 19 × 150 mm, 5 µm; mobile phase: acetonitrile-water (0.1% ammonium bicarbonate); gradient: 25 to 70%; column temperature: 25°C; flow rate: 15 mL/min; wavelength: 214 nm; column pressure: 80 bar) to obtain compound 10a (80 mg, yield: 33.3%). MS m/z (ESI): 547.5 [M+1]⁺.

### Step 2: Synthesis of Compound 10b

Sodium acetate (26.8 mg, 0.33 mmol) was added to a solution of compound 10a (60 mg, 0.11 mmol) and ethylhydroxylamine hydrochloride (21.4 mg, 0.22 mmol) in MeOH (8 mL) at room temperature. The reaction mixture was stirred at 60°C for 16 hours. After the reaction was completed, the reaction mixture was cooled to room temperature, diluted with water (10 mL), and extracted with ethyl acetate (10 mL × 3). The combined organic phases were concentrated under reduced pressure, and the residue was purified by preparative high-performance liquid chromatography (chromatographic column: Xbridge-C18; 19 × 150 mm, 5 µm; mobile phase: acetonitrile-water (0.1% ammonium bicarbonate); gradient: 20 to 70%; column temperature: 25°C; flow rate: 15 mL/min; wavelength: 214 nm; column pressure: 80 bar) to obtain compound 10b (40.0 mg, yield: 62.5%, mixture of (E) and (Z) isomers). MS m/z (ESI): 590.6 [M+1]⁺.

### Step 3: Synthesis of Compounds 10 and 11

NaOH (17 mg, 0.42 mmol) was added to a mixed solution of compound 10b (25 mg, 0.042 mmol) in methanol, tetrahydrofuran, and water (0.3 mL/0.3 mL/0.3 mL). The reaction mixture was stirred at 40°C for 24 hours. After the reaction was completed, the reaction mixture was added with acetic acid to adjust the pH to 6, and concentrated under reduced pressure. The residue was purified by preparative high-performance liquid chromatography (chromatographic column: Xbridge-C18; 19 × 150 mm, 5 µm; mobile phase: acetonitrile-water (0.1% ammonium bicarbonate); gradient: 5 to 60%; column temperature: 25°C; flow rate: 15 mL/min; wavelength: 214 nm; column pressure: 80 bar) to obtain a mixture of compounds 10 and 11 (13.0 mg, yield: 68.4%). MS m/z (ESI): 476.2 [M+1]⁺. ¹H NMR (400 MHz, DMSO-*d₆*): δ 10.81 (s, 1H), 7.96 (d, *J =* 8.0 Hz, 2H), 7.68 (d, *J =* 7.2 Hz, 2H), 7.25 (t, *J =* 2.8 Hz, 1H), 6.65 (s, 1H), 6.47 (*t, J =* 2.4 Hz, 1H), 3.98-3.91 (m, 2H), 3.73 (s, 3H), 3.53 (d, *J =* 12.0 Hz, 1H), 3.22-3.12 (m, 4H), 2.81-2.60 (m, 3H), 2.38 (s, 3H), 2.00-1.98 (m, 1H), 1.69-1.67 (m, 2H), 1.51-1.49 (m, 2H), 1.18-1.11 (m, 3H).

### Example 10 (Compound 32-P1)

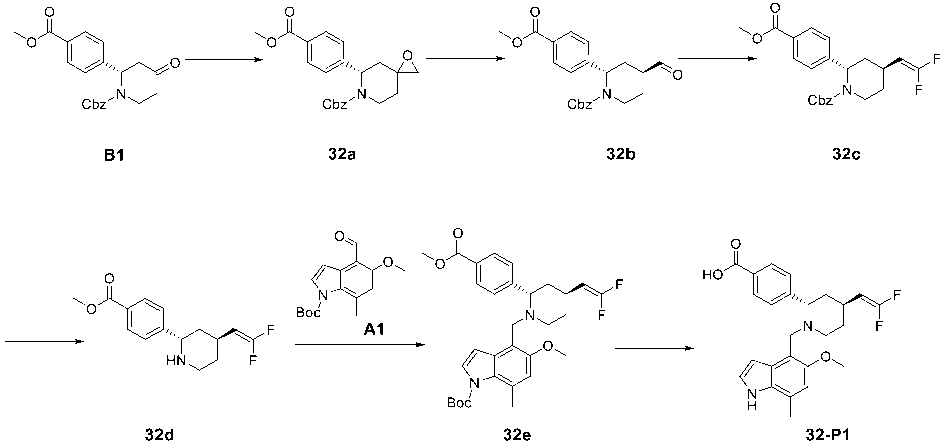

### Step 1: Synthesis of Compound 32a

Potassium tert-butoxide solution (6.54 mL, 6.54 mmol) was slowly added to a mixed solution of trimethylsulfoxonium iodide (1.44 g, 6.54 mmol) in THF and DMSO (10 mL/10 mL) under a nitrogen atmosphere. The mixture was stirred at room temperature for 0.5 hours, followed by the addition of a solution of compound B1 (2 g, 5.45 mmol) in DMSO (10 mL). The reaction mixture was stirred at 30°C for 16 hours. After the reaction was completed, the reaction mixture was poured into ice water and extracted with ethyl acetate (50 mL × 3). The combined organic phases were concentrated under reduced pressure, and the residue was purified by silica gel column chromatography (PE/EA= 5/1 to 2/1) to obtain compound 32a (1.81 g, yield: 87.0%). MS m/z (ESI): 382.4 [M+1]⁺.

### Step 2: Synthesis of Compound 32b

Tris(pentafluorophenyl)borane (1.34 g, 2.62 mmol) was added to a solution of compound 32a (1 g, 2.62 mmol) in toluene (20 mL) at room temperature. The reaction mixture was stirred at 30°C for 16 hours. After the reaction was completed, the reaction mixture was quenched with saturated sodium bicarbonate solution and extracted with ethyl acetate (50 mL × 3). The combined organic phases were concentrated under reduced pressure, and the residue was purified by silica gel column chromatography (PE/EA = 5/1 to 2/1) to obtain compound 32b (0.81 g, yield: 81.0%). MS m/z (ESI): 382.2 [M+1]⁺.

### Step 3: Synthesis of Compound 32c

(Triphenylphosphonio)difluoroacetate (1.50 g, 4.20 mmol) was added to a solution of compound 32b (800 mg, 2.10 mmol) in NMP (15 mL) at room temperature. The reaction mixture was stirred at 40°C for 4 hours. After the reaction was completed, the reaction mixture was diluted with water (100 mL) and extracted with ethyl acetate (100 mL × 3). The combined organic phases were washed with saturated brine (50 mL) and concentrated under reduced pressure. The residue was purified by silica gel column chromatography (PE/EA = 40/1 to 5/1) to obtain compound 32c (570 mg, yield: 65.4%). MS m/z (ESI): 416.4 [M+1]⁺.

### Step 4: Synthesis of Compound 32d

TMSI (1.30 g, 6.51 mmol) was added to a solution of compound 32c (540 mg, 1.30 mmol) in DCM (10 mL) at room temperature. The reaction mixture was stirred at room temperature for 2 hours. After the reaction was completed, the reaction mixture was added with saturated sodium bicarbonate solution to adjust the pH to more than 7, and extracted with DCM (30 mL × 3). The crude product was purified by silica gel column chromatography (DCM/MeOH = 50/1 to 10/1) to obtain compound 32d (340 mg, yield: 93.2%). MS m/z (ESI): 282.2 [M+1]⁺.

### Step 5: Synthesis of Compound 32e

Sodium triacetoxyborohydride (905 mg, 4.27 mmol) was added to a solution of compound 32d (300 mg, 1.07 mmol) and compound A1 (463 mg, 1.60 mmol) in THF (5 mL) at room temperature. The reaction mixture was stirred at 40°C for 16 hours. After the reaction was completed, the reaction mixture was quenched with saturated sodium bicarbonate solution and extracted with ethyl acetate (30 mL × 3). The crude product was purified by silica gel column chromatography (PE/EA = 20/1 to 5/1) to obtain compound 32e (440 mg, yield: 74.5%). MS m/z (ESI): 555.5 [M+1]⁺.

### Step 6: Synthesis of Compound 32-P1

NaOH (289 mg, 7.22 mmol) was added to a mixed solution of compound 32e (400 mg, 0.72 mmol) in methanol/tetrahydrofuran/water (4 mL/4 mL/4 mL). The reaction mixture was stirred at 40°C for 16 hours. After the reaction was completed, the reaction mixture was added with glacial acetic acid to adjust the pH to 5, and concentrated under reduced pressure. The crude product was purified by preparative high-performance liquid chromatography (chromatographic column: Xbridge-C18; 19 × 150 mm, 5 µm; mobile phase: acetonitrile-water (0.1% ammonium bicarbonate); gradient: 20 to 70%; column temperature: 25°C; flow rate: 15 mL/min; wavelength: 214 nm; column pressure: 80 bar) to obtain compound 32-P1 (211.3 mg, yield: 66.4%). MS m/z (ESI): 441.1 [M+1]⁺. ¹H NMR (400 MHz, DMSO-*d₆*): δ 10.81 (s, 1H), 7.95 (d, *J* = 8.4 Hz, 2H), 7.65 *(d, J =* 8.0 Hz, 2H), 7.27-7.23 (m, 1H), 6.65 (s, 1H), 6.51-6.47 (m, 1H), 5.03-4.90 (m, 1H), 3.71 (s, 3H), 3.61-3.48 (m, 3H), 2.69-2.53 (m, 2H), 2.42 (s, 3H), 2.35-2.20 (m, 1H), 1.89-1.81 (m, 1H), 1.70-1.58 (m, 2H), 1.46-1.41 (m, 1H).

### Example 11 (Compounds 33-P1 and 33-P2)

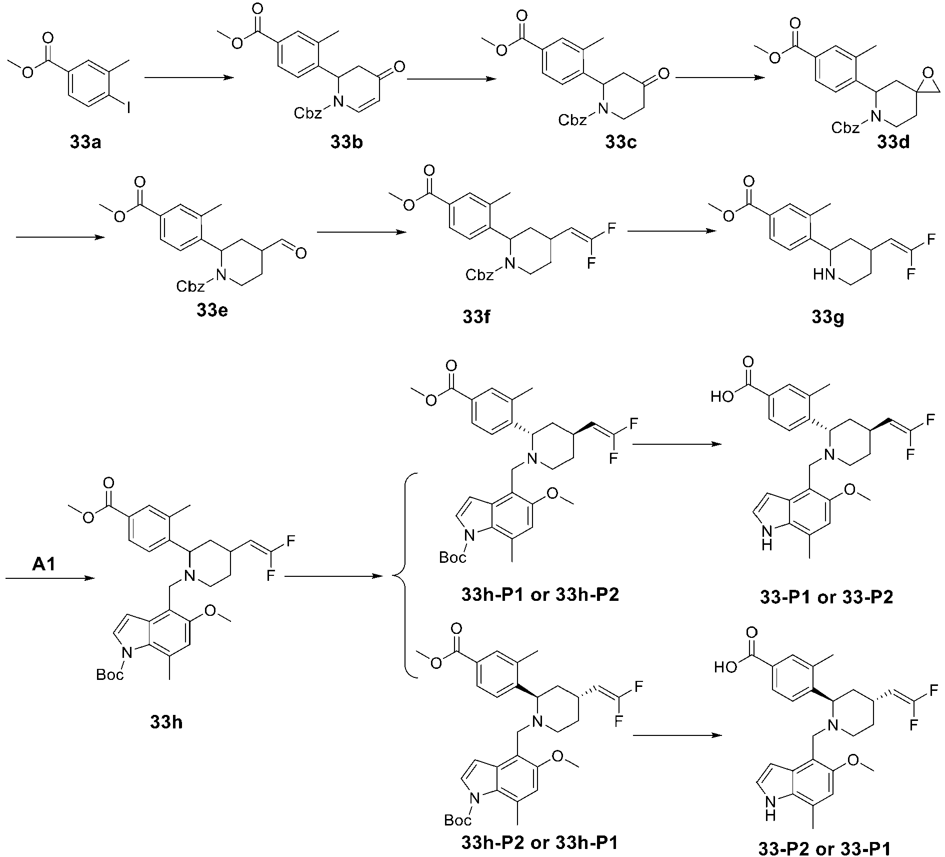

### Step 1: Synthesis of Compound 33b

i-PrMgCl·LiCl (104 mL, 135 mmol) was slowly added to a solution of compound 33a (25 g, 90.5 mmol) in THF (250 mL) at 0°C. The mixture was stirred at room temperature for 2 hours, followed by the addition of THF (250 mL). The mixture was cooled to -30°C, followed by the sequential addition of 4-methoxypyridine (14.8 g, 135 mmol) and Cbz-Cl (22.9 g, 135 mmol). The reaction mixture was stirred at -30°C for 1 hour. After the reaction was completed, the reaction mixture was quenched with water (100 mL), then added with 6 N hydrochloric acid to adjust the pH to 5, and extracted with ethyl acetate (200 mL × 3). The combined organic phases were concentrated under reduced pressure, and the residue was purified by silica gel column chromatography (PE/EA = 1/0 to 3/1) to obtain compound 33b (21.5 g, yield: 62.7%). MS m/z (ESI): 380.2 [M+1]⁺.

### Step 2: Synthesis of Compound 33c

Zinc powder (36 g, 0.55 mol) was added to a solution of compound 33b (21 g, 0.055 mol) in acetic acid (200 mL) at room temperature. The reaction mixture was stirred at 100°C for 16 hours. After the reaction was completed, the reaction mixture was filtered, and the filtrate was concentrated under reduced pressure. The residue was added with ethyl acetate (200 mL) and washed with saturated sodium bicarbonate solution (100 mL × 3). The organic phase was concentrated under reduced pressure, and the residue was purified by silica gel column chromatography (PE/EA = 1/0 to 3/1) to obtain compound 33c (16 g, yield: 76.2%). MS m/z (ESI): 382.3 [M+1]⁺.

### Step 3: Synthesis of Compound 33d

Potassium tert-butoxide solution (7.87 mL, 7.87 mmol) was slowly added to a mixed solution of trimethylsulfoxonium iodide (1.73 g, 7.87 mmol) in THF and DMSO (20 mL/20 mL) under a nitrogen atmosphere. The mixture was stirred at room temperature for 1 hour, followed by the addition of a solution of compound 33c (2 g, 5.25 mmol) in THF (10 mL). The reaction mixture was stirred at room temperature for 16 hours. After the reaction was completed, the reaction mixture was poured into ice water and extracted with ethyl acetate (50 mL × 3). The combined organic phases were concentrated under reduced pressure, and the residue was purified by silica gel column chromatography (petroleum ether/ethyl acetate = 1/0 to 3/1) to obtain compound 33d (1.1 g, yield: 53.1%). MS m/z (ESI): 396.4 [M+1]⁺.

### Step 4: Synthesis of Compound 33e

Tris(pentafluorophenyl)borane (3.44 g, 6.72 mmol) was added to a solution of compound 33d (2.41 g, 6.11 mmol) in toluene (50 mL) at room temperature. The reaction mixture was stirred at room temperature for 16 hours. After the reaction was completed, the reaction mixture was quenched with saturated sodium bicarbonate solution and extracted with ethyl acetate (50 mL × 3). The combined organic phases were concentrated under reduced pressure, and the residue was purified by silica gel column chromatography (PE/EA = 1/0 to 10/1) to obtain compound 33e (1.37 g, yield: 56.8%). MS m/z (ESI): 396.3 [M+1]⁺.

### Step 5: Synthesis of Compound 33f

Compound 33e (3.35 g, 8.48 mmol) was added to a solution of (triphenylphosphonio)difluoroacetate (6.04 g, 16.96 mmol) in NMP (35 mL). The reaction mixture was stirred at 80°C for 16 hours. After the reaction was completed, the reaction mixture was diluted with water (500 mL) and extracted with EA (100 mL × 3). The combined organic phases were concentrated under reduced pressure, and the crude product was purified by silica gel column chromatography (PE/EA = 1/0 to 10/1) to obtain compound 33f (1.23 g, yield: 33.8%, *trans* configuration). MS m/z (ESI): 430.4 [M+1]⁺.

### Step 6: Synthesis of Compound 33g

TMSI (1.17 g, 5.82 mmol) was added to a solution of compound 33f (500 mg, 1.17 mmol, *trans* configuration) in dichloromethane (20 mL) at 0°C. The reaction mixture was stirred at 0°C for 2 hours. After the reaction was completed, the reaction mixture was added with dilute hydrochloric acid to adjust the pH to acidic, and extracted with petroleum ether. The aqueous phase was added with saturated sodium bicarbonate solution to adjust the pH to alkaline, and extracted with DCM (30 mL × 3). The combined organic phases were concentrated under reduced pressure to obtain compound 33g (0.34 g, yield: 99%, *trans* configuration). MS m/z (ESI): 296.3 [M+1]⁺.

### Step 7: Synthesis of Compounds 33h, 33h-P1, and 33h-P2

Sodium triacetoxyborohydride (1.02 g, 4.83 mmol) was added to a solution of compound 33g (356 mg, 1.21 mmol, *trans* configuration) and compound A1 (523 mg, 1.81 mmol) in tetrahydrofuran (10 mL) at room temperature. The reaction mixture was stirred at 40°C for 16 hours. After the reaction was completed, the reaction mixture was added with ethyl acetate (30 mL) and washed with saturated sodium bicarbonate solution (10 mL × 3). The organic phase was concentrated under reduced pressure, and the crude product was purified by silica gel column chromatography (PE/EA = 10/1) to obtain compound 33h (0.42 g, yield: 61.6%, *trans* configuration). Compound 33h (150 mg, *trans* configuration) was resolved by preparative chiral chromatography [equipment: Gilson GX-281; column: Chiralpark IG 250 mm × 30 mm, 10 µm; mobile phase: Hex/EtOH = 90-10; flow rate: 25 mL/min] to obtain compound 33h-P1 (70 mg, yield: 46.7%) and compound 33h-P2 (65 mg, yield: 43.3%).

33h-P1: MS m/z (ESI): 569.5 [M+1]⁺. Chiral HPLC: retention time = 4.56 min, UV = 254 nm.

33h-P2: MS m/z (ESI): 569.5 [M+1]⁺. Chiral HPLC: retention time = 5.74 min, UV = 254 nm.

### Step 8: Synthesis of Compound 33-P1

NaOH (42 mg, 1.05 mmol) was added to a mixed solution of compound 33h-P1 (60 mg, 0.105 mmol) in methanol/tetrahydrofuran/water (0.6 mL/0.6 mL/0.6 mL). The reaction mixture was stirred at 40°C for 16 hours. After the reaction was completed, the reaction mixture was added with AcOH to adjust the pH to 5, and concentrated under reduced pressure. The crude product was purified by preparative high-performance liquid chromatography (chromatographic column: Xbridge-C18; 19 × 150 mm, 5 µm; mobile phase: acetonitrile-water (0.1% ammonium bicarbonate); gradient: 20 to 55%; column temperature: 25°C; flow rate: 15 mL/min; wavelength: 214 nm; column pressure: 80 bar) to obtain compound 33-P1 (39.4 mg, yield: 82.1%). MS m/z (ESI): 455.2 [M+1]⁺. ¹H NMR (400 MHz, DMSO-*d₆*): δ 10.81 (s, 1H), 7.84 (q, *J =* 18.8 Hz, 2H), 7.76 (s, 1H), 7.26 (t, *J =* 2.8 Hz, 1H), 6.65 (s, 1H), 6.49 (t, *J =* 2.8 Hz, 1H), 5.07-4.97 (m, 1H), 3.71 (s, 3H), 3.70 (d, *J =* 2.0 Hz, 1H), 3.57 (*d, J =* 11.6 Hz, 1H), 3.22 (d, *J =* 11.6 Hz, 1H), 2.71 (s, 1H), 2.62-2.59 (m, 1H), 2.42 (d, *J =* 6.8Hz, 6H), 2.26 (t, *J* = 12.4 Hz, 1H), 1.76-1.69 (m, 2H), 1.55 (d, *J =* 12.4 Hz, 1H), 1.43 (d, *J =* 13.2 Hz, 1H).

### Step 9: Synthesis of Compound 33-P2

NaOH (46 mg, 1.14 mmol) was added to a mixed solution of compound 33h-P2 (65 mg, 0.11 mmol) in methanol/tetrahydrofuran/water (0.6 mL/0.6 mL/0.6 mL). The reaction mixture was stirred at 40°C for 16 hours. After the reaction was completed, the reaction mixture was added with AcOH to adjust the pH to 5, and concentrated under reduced pressure. The crude product was purified by preparative high-performance liquid chromatography (chromatographic column: Xbridge-C18; 19 × 150 mm, 5 µm; mobile phase: acetonitrile-water (0.1% ammonium bicarbonate); gradient: 20 to 55%; column temperature: 25°C; flow rate: 15 mL/min; wavelength: 214 nm; column pressure: 80 bar) to obtain compound 33-P2 (34.3 mg, yield: 65.9%). MS m/z (ESI): 455.2 [M+1]⁺. ¹H NMR (400 MHz, DMSO-*d₆*): δ 10.81 (s, 1H), 7.84 (q, *J =* 18.8 Hz, 2H), 7.76 (s, 1H), 7.26 (t, *J =* 2.8 Hz, 1H), 6.65 (s, 1H), 6.49 (t, *J =* 2.8 Hz, 1H), 5.07-4.97 (m, 1H), 3.71 (s, 3H), 3.70 (d, *J =* 2.0 Hz, 1H), 3.57 (*d, J =* 11.6 Hz, 1H), 3.22 (d, *J =* 11.6 Hz, 1H), 2.71 (s, 1H), 2.62-2.59 (m, 1H), 2.42 (d, *J =* 6.8Hz, 6H), 2.26 (t, *J* = 12.4 Hz, 1H), 1.76-1.69 (m, 2H), 1.55 (d, *J* = 12.4 Hz, 1H), 1.43 (d, *J* = 13.2 Hz, 1H).

### Example 12 (Compound 34-1)

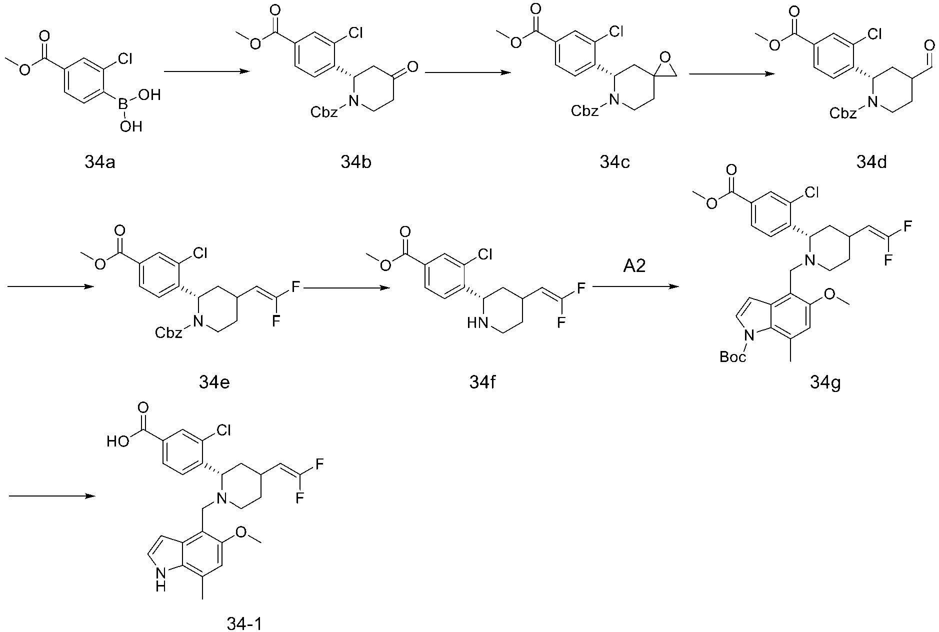

### Step 1: Synthesis of Compound 34b

(-)-1,2-Bis((2*R*,5*R*)-2,5-diphenylphospholano)ethane (0.20 g, 0.39 mmol) and acetylacetonatobis(ethylene)rhodium(I) (0.10 g, 0.39 mmol) were sequentially added to a mixed solution of compound 34a (5.02 g, 23.36 mmol) and benzyl 4-oxo-3,4-dihydropyridine-1(2*H*)-carboxylate (1.80 g, 7.79 mmol) in *tert-amyl* alcohol and water (30 mL/3 mL). The reaction mixture was stirred at 80°C for 16 hours. After the reaction was completed, the reaction mixture was added with ethyl acetate (50 mL) and washed with water (100 mL × 3). The organic phase was dried over anhydrous sodium sulfate, filtered, and the filtrate was concentrated under reduced pressure. The residue was purified by silica gel column chromatography (petroleum ether/ethyl acetate = 1/0 to 4/1) to obtain compound 34b (2.90 g). MS m/z (ESI): 402.1 [M+1]⁺.

### Step 2: Synthesis of Compound 34c

Potassium *tert*-butoxide (1.21 g, 10.85 mmol) was slowly added to a mixed solution of trimethylsulfoxonium iodide (2.38 g, 10.85 mmol) in tetrahydrofuran and dimethyl sulfoxide (15 mL/15 mL) under a nitrogen atmosphere. The mixture was stirred at room temperature for 0.5 hours, followed by the addition of a solution of compound 34b (2.90 g, 7.23 mmol) in DMSO (5 mL). The reaction mixture was stirred at 26°C for 16 hours. After the reaction was completed, the reaction mixture was poured into ice water (150 mL) and extracted with ethyl acetate (50 mL × 3). The combined organic phases were dried over anhydrous sodium sulfate, filtered, and the filtrate was concentrated under reduced pressure. The residue was purified by silica gel column chromatography (petroleum ether/ethyl acetate = 1/0 to 3/1) to obtain compound 34c (1.20 g). MS m/z (ESI): 416.1 [M+1]⁺.

### Step 3: Synthesis of Compound 34d

Tris(pentafluorophenyl)borane (1.48 g, 2.89 mmol) was added to a solution of compound 34c (1.20 g, 2.89 mmol) in toluene (20 mL) at room temperature. The reaction mixture was stirred at room temperature for 16 hours. After the reaction was completed, the reaction mixture was quenched with saturated sodium bicarbonate solution (20 mL) and extracted with ethyl acetate (50 mL × 3). The combined organic phases were dried over anhydrous sodium sulfate, filtered, and the filtrate was concentrated under reduced pressure. The residue was purified by silica gel column chromatography (petroleum ether/ethyl acetate = 5/1 to 2/1) to obtain compound 34d (1.50 g, crude product). MS m/z (ESI): 416.1 [M+1]⁺.

### Step 4: Synthesis of Compound 34e

(Triphenylphosphonio)difluoroacetate (1.85 g, 5.20 mmol) was added to a solution of compound 34d (1.08 g, 2.60 mmol) in *N*-methylpyrrolidone (30 mL). The reaction mixture was stirred at 80°C for 16 hours. After the reaction was completed, the reaction mixture was diluted with water (50 mL) and extracted with ethyl acetate (50 mL × 3). The combined organic phases were dried over anhydrous sodium sulfate, filtered, and the filtrate was concentrated under reduced pressure. The residue was purified by silica gel column chromatography (petroleum ether/ethyl acetate = 1/0 to 10/1) to obtain compound 34e (250 mg). MS m/z (ESI): 450.1 [M+1]⁺.

### Step 5: Synthesis of Compound 34f

Iodotrimethylsilane (444.7 mg, 2.22 mmol) was added to a solution of compound 34e (250 mg, 0.56 mmol) in dichloromethane (5 mL) at 0°C. The reaction mixture was stirred at 0°C for 2 hours. After the reaction was completed, the reaction mixture was concentrated under reduced pressure, then added with dilute hydrochloric acid to adjust the pH to 4 to 5, and washed with petroleum ether (50 mL). The aqueous phase was added with saturated sodium bicarbonate solution to adjust the pH to 8 to 9, and extracted with dichloromethane (30 mL × 3). The combined organic phases were dried over anhydrous sodium sulfate, filtered, and the filtrate was concentrated under reduced pressure to obtain compound 34f (70 mg). MS m/z (ESI): 316.0 [M+1]⁺.

### Step 6: Synthesis of Compound 34g

Compound 34f (60 mg, 0.19 mmol), compound A2 (236.2 mg, 0.76 mmol), cesium carbonate (185.7 mg, 0.57 mmol), and potassium iodide (94.6 mg, 0.57 mmol) were sequentially dissolved in N,N-dimethylformamide (5 mL) at room temperature. The reaction mixture was stirred at room temperature for 2 hours. After the reaction was completed, the reaction mixture was quenched with water (5 mL) and extracted with ethyl acetate (20 mL × 2). The combined organic phases were then washed with saturated brine (20 mL × 3), dried over anhydrous sodium sulfate, filtered, and the filtrate was concentrated under reduced pressure. The residue was purified by silica gel column chromatography (petroleum ether/ethyl acetate = 1:0 to 8:1) to obtain compound 34-1 (50 mg, yield: 44.7%). MS m/z (ESI): 589.2 [M+1]⁺.

### Step 7: Synthesis of Compound 34-1

Sodium hydroxide (47.8 mg, 1.19 mmol) was added to a mixed solution of compound 34g (47 mg, 0.079 mmol) in methanol/tetrahydrofuran/water (1 mL/1 mL/1 mL). The reaction mixture was stirred at 40°C for 16 hours. After the reaction was completed, the reaction mixture was added with acetic acid to adjust the pH to 5, and concentrated under reduced pressure. The residue was purified by preparative high-performance liquid chromatography (chromatographic column: Gemini 5 µm C18 150 × 21.2 mm; 150 × 21.2 mm, 5 µm; mobile phase: acetonitrile-water (0.1% ammonia); gradient: 30 to 60%; column temperature: 25°C; flow rate: 20 mL/min; wavelength: 214 nm; column pressure: 70 bar) to obtain compound 34-1 (5.68 mg). MS m/z (ESI): 475.35 [M+1]⁺. ¹H NMR (400 MHz, DMSO-*d₆*) δ 10.84 (s, 1H), 7.98-7.86 (m, 3H), 7.26-7.25 (m, 1H), 6.66 (s, 1H), 6.51- 6.44 (m, 1H), 4.99-4.37 (m, 1H), 3.92-3.75 (m, 1H), 3.72 (s, 3H), 3.61-3.56 (m, 1H), 3.24 *(d, J=* 11.6 Hz, 1H), 2.79-2.60 (m, 2H), 2.42 (s, 3H), 2.33-2.27 (m, 1H), 1.71-1.28 (m, 4H).

### Example 13 (Compound 35-P1)

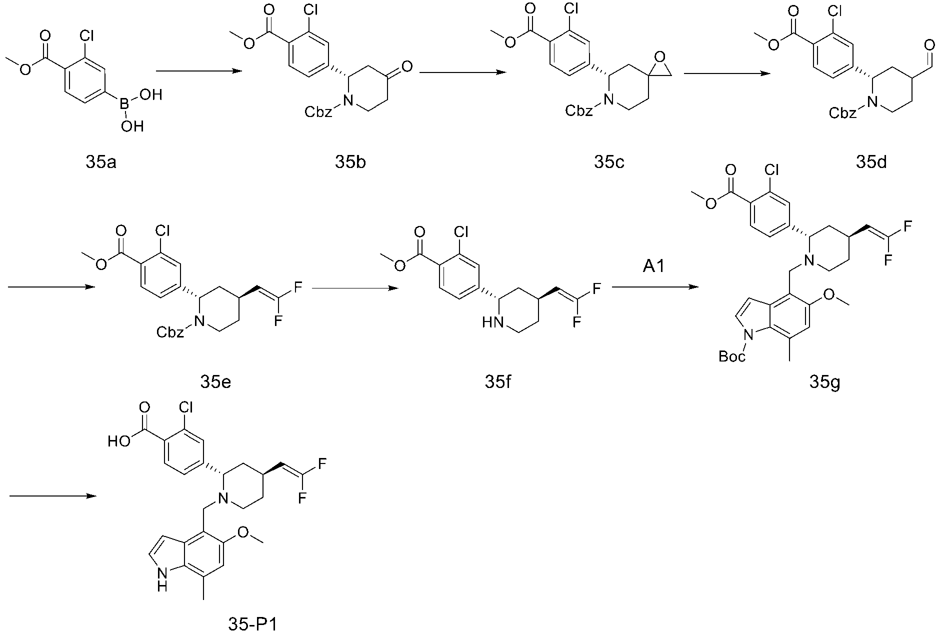

### Step 1: Synthesis of Compound 35b

(-)-1,2-Bis((2*R*,5*R*)-2,5-diphenylphospholano)ethane (0.17 g, 0.32 mmol) and acetylacetonatobis(ethylene)rhodium(I) (0.08 g, 0.32 mmol) were sequentially added to a mixed solution of compound 35a (4.18 g, 19.47 mmol), benzyl 4-oxo-3,4-dihydropyridine-1(2*H*)-carboxylate (1.5 g, 6.49 mmol), and triethylamine (1.97 g, 19.47 mmol) in *tert-amyl* alcohol and water (50 mL/5 mL). The reaction mixture was stirred at 80°C for 16 hours. After the reaction was completed, the reaction mixture was extracted with ethyl acetate (150 mL). The combined organic phases were dried over anhydrous sodium sulfate, filtered, and the filtrate was concentrated under reduced pressure. The residue was purified by silica gel column chromatography (PE/EA = 1/0 to 5/1) to obtain compound 35b (2.80 g, crude product). MS m/z (ESI): 402.2 [M+1]⁺.

### Step 2: Synthesis of Compound 35c

Potassium *tert*-butoxide (0.94 g, 8.37 mmol) was slowly added to a mixed solution of trimethylsulfoxonium iodide (1.85 g, 8.37 mmol) in THF and DMSO (20 mL/20 mL) under a nitrogen atmosphere. The mixture was stirred at room temperature for 0.5 hours, followed by the addition of a solution of compound 35b (2.80 g, 6.98 mmol) in DMSO (5 mL). The reaction mixture was stirred at room temperature for 16 hours. After the reaction was completed, the reaction mixture was poured into ice water (40 mL) and extracted with ethyl acetate (80 mL × 3). The combined organic phases were dried over anhydrous sodium sulfate, filtered, and the filtrate was concentrated under reduced pressure. The residue was purified by silica gel column chromatography (PE/EA = 20/1 to 3/1) to obtain compound 35c (1.80 g). MS m/z (ESI): 416.2 [M+1]⁺.

### Step 3: Synthesis of Compound 35d

Tris(pentafluorophenyl)borane (2.20 g, 4.33 mmol) was added to a solution of compound 35c (1.80 g, 4.33 mmol) in toluene (20 mL) at room temperature. The reaction mixture was stirred at room temperature for 16 hours. After the reaction was completed, the reaction mixture was quenched with saturated sodium bicarbonate solution (20 mL) and extracted with ethyl acetate (50 mL × 3). The combined organic phases were dried over anhydrous sodium sulfate, filtered, and the filtrate was concentrated under reduced pressure. The residue was purified by silica gel column chromatography (PE/EA = 20/1 to 3/1) to obtain compound 35d (0.90 g). MS m/z (ESI): 416.2 [M+1]⁺.

### Step 4: Synthesis of Compound 35e

Compound 35d (900 mg, 2.16 mmol) was added to a solution of (triphenylphosphonio)difluoroacetate (1.54 g, 4.32 mmol) in NMP (20 mL). The reaction mixture was stirred at 80°C for 16 hours. After the reaction was completed, the reaction mixture was diluted with water (50 mL) and extracted with ethyl acetate (50 mL × 3). The combined organic phases were dried over anhydrous sodium sulfate, filtered, and the filtrate was concentrated under reduced pressure. The residue was purified by silica gel column chromatography (PE/EA = 1/0 to 10/1) to obtain compound 35e (400 mg). MS m/z (ESI): 450.2 [M+1]⁺.

### Step 5: Synthesis of Compound 35f

Iodotrimethylsilane (498.8 mg, 3.55 mmol) was added to a solution of compound 35e (400 mg, 0.89 mmol) in dichloromethane (10 mL) at 0°C. The reaction mixture was stirred at 0°C for 2 hours. After the reaction was completed, the reaction mixture was concentrated under reduced pressure, then added with dilute hydrochloric acid to adjust the pH to 4 to 5, and washed with petroleum ether (10 mL × 2). The aqueous phase was added with saturated sodium bicarbonate solution to adjust the pH to 8 to 9, and extracted with dichloromethane (20 mL × 3). The combined organic phases were washed with saturated brine (20 mL × 3), dried over anhydrous sodium sulfate, filtered, and the filtrate was concentrated under reduced pressure to obtain compound 35f (200 mg). The crude product was directly used in the next step without further purification. MS m/z (ESI): 316.1 [M+1]⁺.

### Step 6: Synthesis of Compound 35g

Sodium triacetoxyborohydride (402.6 mg, 1.90 mmol) was added to a solution of compound 35f (200 mg, 0.63 mmol) and compound A1 (from MeiYue) (275.8 mg, 0.95 mmol) in tetrahydrofuran (10 mL) at room temperature. The reaction mixture was stirred at 40°C for 16 hours. After the reaction was completed, the reaction mixture was quenched with water (10 mL) and extracted with ethyl acetate (20 mL × 3). The combined organic phases were washed with saturated brine (20 mL × 3), dried over anhydrous sodium sulfate, filtered, and the filtrate was concentrated under reduced pressure. The residue was purified by silica gel column chromatography (PE/EA = 1/0 to 3/1) to obtain compound 35g (95 mg). MS m/z (ESI): 589.3 [M+1]⁺.

### Step 7: Synthesis of Compound 35-P1

Sodium hydroxide (54.2 mg, 1.35 mmol) was added to a mixed solution of compound 35g (80 mg, 0.13 mmol) in methanol/tetrahydrofuran/water (3 mL/3 mL/3 mL). The reaction mixture was stirred at 40°C for 16 hours. After the reaction was completed, the reaction mixture was added with AcOH to adjust the pH to 5, and concentrated under reduced pressure. The residue was purified by preparative high-performance liquid chromatography (chromatographic column: Xbridge-C18; 19 × 150 mm, 5 µm; mobile phase: acetonitrile-water (0.1% ammonium bicarbonate); gradient: 5% to 45%; column temperature: 25°C; flow rate: 20 mL/min; wavelength: 214 nm; column pressure: 80 bar) to obtain compound 35-P1 (42.5 mg). MS m/z (ESI): 475.3 [M+1]⁺. ¹H NMR (400 MHz, DMSO-*d₆*) δ 13.25 (s, 1H), 10.84 (s, 1H), 7.79 (*d, J =* 8.0 Hz, 1H), 7.72 (s, 1H), 7.53 (*d, J =* 8.4 Hz, 1H), 7.33-7.20 (m, 1H), 6.66 (s, 1H), 6.51-6.38 (m, 1H), 5.06-4.86 (m, 1H), 3.74 (s, 3H), 3.63 (d, *J =* 11.2 Hz, 1H), 3.57-3.44 (m, 1H), 3.30-3.23 (m, 1H), 2.72-2.62 (m, 1H), 2.61-2.53 (m, 1H), 2.42 (s, 3H), 2.33-2.20 (m, 1H), 1.86-1.72 (m, 1H), 1.71-1.53 (m, 2H), 1.49-1.37 (m, 1H).

### Example 14 (Compound 36-P1)

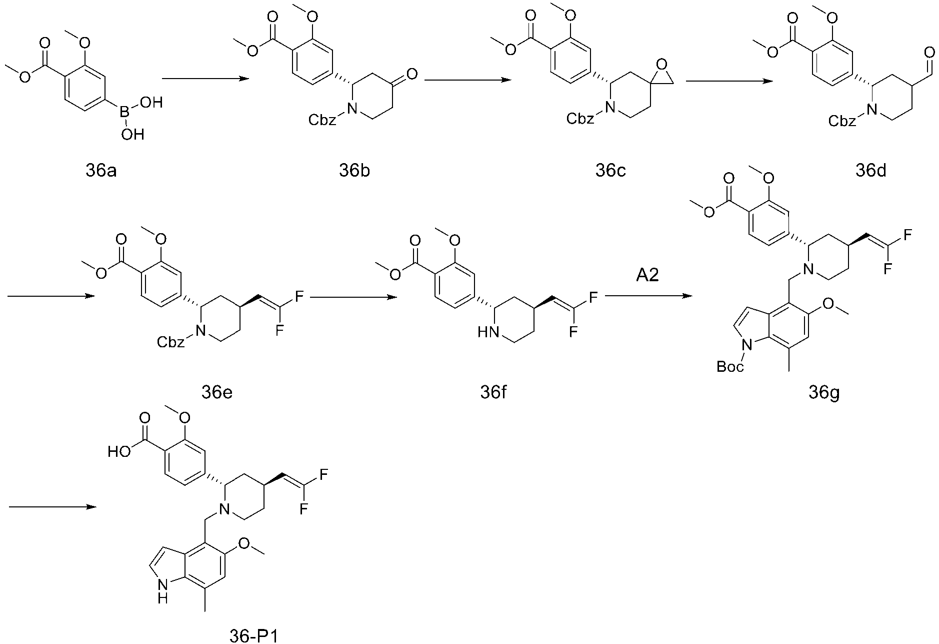

### Step 1: Synthesis of Compound 36b

(-)-1,2-Bis((2*R*,5*R*)-2,5-diphenylphospholano)ethane (0.27 g, 0.54 mmol) and acetylacetonatobis(ethylene)rhodium(I) (0.14 g, 0.54 mmol) were sequentially added to a mixed solution of benzyl 4-oxo-3,4-dihydropyridine-1(2*H*)-carboxylate (2.50 g, 10.81 mmol), compound 36a (5.67 g, 27.03 mmol), and triethylamine (3.28 g, 32.43 mmol) in *tert-amyl* alcohol and water (25 mL/2.5 mL). The reaction mixture was stirred at 80°C for 10 hours. After the reaction was completed, the reaction mixture was added with ethyl acetate (50 mL) and washed with water (30 mL × 3). The combined organic phases were dried over anhydrous sodium sulfate, filtered, and the filtrate was concentrated under reduced pressure. The residue was purified by silica gel column chromatography (PE/EA = 1/0 to 3/1) to obtain compound 36b (4.50 g, crude product). MS m/z (ESI): 397.8 [M+1]⁺.

### Step 2: Synthesis of Compound 36c

Potassium *tert*-butoxide (1.87 g, 16.65 mmol) was slowly added to a mixed solution of trimethylsulfoxonium iodide (3.66 g, 16.65 mmol) in tetrahydrofuran and dimethyl sulfoxide (20 mL/20 mL) under a nitrogen atmosphere. The mixture was stirred at room temperature for 1 hour, followed by the addition of a solution of compound 36b (4.40 g, 11.08 mmol) in DMSO (10 mL). The reaction mixture was stirred at room temperature for another 4 hours. After the reaction was completed, the reaction mixture was poured into ice water and extracted with ethyl acetate (50 mL × 3). The combined organic phases were dried over anhydrous sodium sulfate, filtered, and the filtrate was concentrated under reduced pressure. The residue was purified by silica gel column chromatography (petroleum ether/ethyl acetate = 1/0 to 3/1) to obtain compound 36c (3.70 g). MS m/z (ESI): 411.9 [M+1]⁺.

### Step 3: Synthesis of Compound 36d

Tris(pentafluorophenyl)borane (4.90 g, 9.57 mmol) was added to a solution of compound 36c (3.60 g, 8.76 mmol) in toluene (36 mL) at room temperature. The reaction mixture was stirred at room temperature for 16 hours. After the reaction was completed, the reaction mixture was quenched with saturated sodium bicarbonate solution (20 mL) and extracted with ethyl acetate (50 mL × 3). The combined organic phases were dried over anhydrous sodium sulfate, filtered, and the filtrate was concentrated under reduced pressure. The residue was purified by silica gel column chromatography (PE/EA = 5/1 to 2/1) to obtain compound 36d (2.45 g). MS m/z (ESI): 411.9 [M+1]⁺.

### Step 4: Synthesis of Compound 36e

Compound 36d (2.40 g, 5.83 mmol) was added to a solution of (triphenylphosphonio)difluoroacetate (4.13 g, 11.66 mmol) in N-methylpyrrolidone (24 mL). The reaction mixture was stirred at 80°C for 16 hours. After the reaction was completed, the reaction mixture was diluted with water (50 mL) and extracted with ethyl acetate (50 mL × 3). The combined organic phases were dried over anhydrous sodium sulfate, filtered, and the filtrate was concentrated under reduced pressure. The residue was purified by silica gel column chromatography (PE/EA = 1/0 to 10/1) to obtain compound 36e (1.60 g). MS m/z (ESI): 445.9 [M+1]⁺.

### Step 5: Synthesis of Compound 36f

Iodotrimethylsilane (2.38 g, 11.90 mmol) was added to a solution of compound 36e (1.50 g, 3.37 mmol) in dichloromethane (16 mL) at 0°C. The reaction mixture was stirred at 0°C for 1 hour. The reaction mixture was concentrated under reduced pressure at room temperature, and added with dilute hydrochloric acid to adjust the pH to 4 to 5. The aqueous phase was washed with petroleum ether (30 mL × 2), then added with saturated sodium bicarbonate solution to adjust the pH to 8 to 9, and extracted with ethyl acetate (30 mL × 3). The combined organic phases were dried over anhydrous sodium sulfate, filtered, and the filtrate was concentrated under reduced pressure to obtain compound 36f (0.86 g). MS m/z (ESI): 311.7 [M+1]⁺.

### Step 6: Synthesis of Compound 36g

Cesium carbonate (1.26 g, 3.85 mmol) and potassium iodide (319.9 mg, 1.92 mmol) were added to a solution of compound 36f (300 mg, 0.96 mmol) and compound A2 (598.9 mg, 1.92 mmol) in N,N-dimethylformamide (3 mL) at room temperature. The reaction mixture was stirred at room temperature for 2 hours. After the reaction was completed, the reaction mixture was extracted with ethyl acetate (30 mL × 3). The combined organic phases were dried over anhydrous sodium sulfate, filtered, and the filtrate was concentrated under reduced pressure. The residue was purified by silica gel column chromatography (PE/EA = 1/0 to 10/1) to obtain compound 36g (150 mg). MS m/z (ESI): 585.1 [M+1]⁺.

### Step 7: Synthesis of Compound 36-P1

Sodium hydroxide (95.6 mg, 2.39 mmol) was added to a mixed solution of compound 36g (140 mg, 0.24 mmol) in methanol/tetrahydrofuran/water (1 mL/1 mL/1 mL). The reaction mixture was stirred at 40°C for 16 hours. After the reaction was completed, the reaction mixture was added with AcOH to adjust the pH to 5, and concentrated under reduced pressure. The residue was purified by preparative high-performance liquid chromatography (chromatographic column: Xbridge-C18; 19 × 150 mm, 5 µm; mobile phase: acetonitrile-water (0.1% ammonium bicarbonate); gradient: 20 to 30%; column temperature: 25°C; flow rate: 25 mL/min; wavelength: 214 nm; column pressure: 50 bar) to obtain compound 36-P1 (50.4 mg). MS m/z (ESI): 471.0 [M+1]⁺. ¹H NMR (400 MHz, DMSO-*d₆*) δ 12.41 (s, 1H), 10.82 (s, 1H), 7.65 (d, *J =* 7.6 Hz, 1H), 7.30-7.23 (m, 2H), 7.15 (d, *J =* 7.6 Hz, 1H), 6.65 (s, 1H), 6.56-6.51 (m, 1H), 5.03-4.90 (m, 1H), 3.85 (s, 3H), 3.70 (s, 3H), 3.63 (d, *J=* 12.0 Hz, 1H), 3.49-3.44 (m, 1H), 3.38-3.34 (m, 1H), 2.61-2.54 (m, 2H), 2.41 (s, 3H), 2.27-2.21 (m, 1H), 1.96-1.82 (m, 1H), 1.71-1.59 (m, 2H), 1.48-1.40 (m, 1H).

### Example 15 (Compound 37-P1)

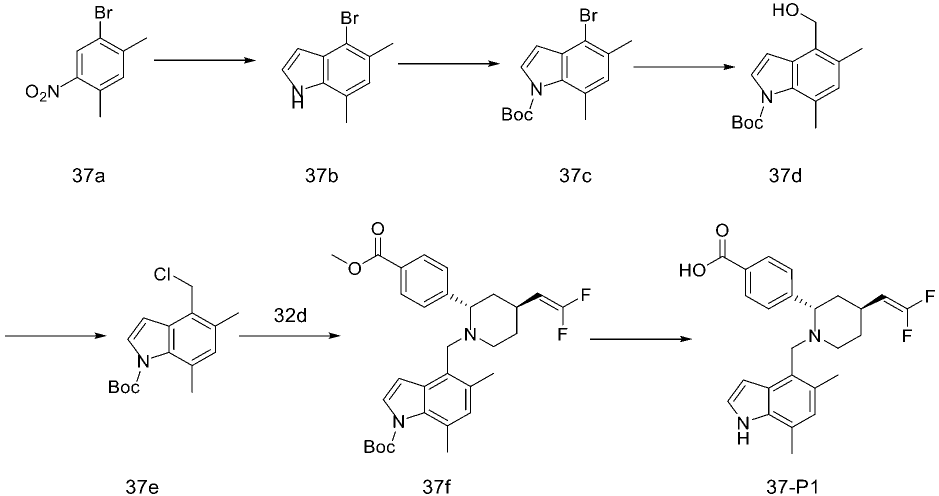

### Step 1: Synthesis of Compound 37b

Vinylmagnesium bromide (1.0 M, 86.9 mL, 86.93 mmol) was slowly added dropwise to a solution of compound 37a (5 g, 21.73 mmol) in tetrahydrofuran (100 mL) at -78°C. The reaction mixture was stirred at 0°C for 2 hours. After the reaction was completed, the reaction mixture was quenched with water (100 mL) and extracted with ethyl acetate (100 mL × 3). The combined organic phases were then washed with saturated brine (200 mL × 1), dried over anhydrous sodium sulfate, filtered, and the filtrate was concentrated under reduced pressure. The residue was purified by silica gel column chromatography (petroleum ether/ethyl acetate = 100/0 to 80/20) to obtain compound 37b (2 g). MS m/z (ESI): 223.9 [M+1]⁺.

### Step 2: Synthesis of Compound 37c

Di-*tert*-butyl dicarbonate (7.30 g, 33.47 mmol) and 4-dimethylaminopyridine (0.27 g, 2.23 mmol) were sequentially added to a solution of compound 37b (5 g, 22.31 mmol) in dichloromethane (50 mL) at room temperature. The reaction mixture was stirred at room temperature for 2 hours. After the reaction was completed, the reaction mixture was concentrated under reduced pressure. The residue was purified by silica gel column chromatography (petroleum ether/ethyl acetate = 1/0 to 10/1) to obtain compound 37c (4.50 g). ¹H NMR (400 MHz, CDCl₃) δ 7.51 (d, *J =* 4.0 Hz, 1H), 6.71 (s, 1H), 6.56 (d, *J =* 4.0 Hz, 1H), 3.89 (s, 3H), 2.57 (s, 3H), 1.59 (s, 9H).

### Step 3: Synthesis of Compound 37d

Tributyltin methoxide (1.48 g, 4.61 mmol) and X-Phos Pd G2 (241.6 mg, 0.30 mmol, CAS: 1310584-14-5) were added to a solution of compound 37c (1 g, 3.07 mmol) in 1,4-dioxane (15 mL) under a nitrogen atmosphere. The reaction mixture was stirred at 80°C for 16 hours under a nitrogen atmosphere. After the reaction was completed, the reaction mixture was concentrated under reduced pressure. The residue was purified by silica gel column chromatography (petroleum ether/ethyl acetate = 1:0 to 3:1) to obtain compound 37d (0.40 g). MS m/z (ESI): 258.1 [M-OH]⁺.

### Step 4: Synthesis of Compound 37e

(Chloromethylene)dimethylammonium chloride (370.5 mg, 2.89 mmol) was added to a solution of compound 37d (400 mg, 1.45 mmol) in dichloromethane (10 mL) at 0°C. The reaction mixture was stirred at room temperature for 2 hours. After the reaction was completed, the reaction mixture was quenched with saturated sodium bicarbonate (10 mL) and extracted with ethyl acetate (20 mL × 2). The combined organic phases were washed with saturated brine (20 mL × 1), dried over anhydrous sodium sulfate, filtered, and the filtrate was concentrated under reduced pressure to obtain compound 37e (300 mg, crude product). MS m/z (ESI): 258.1 [M-Cl]⁺.

### Step 5: Synthesis of Compound 37f

Compound 32d (150 mg, 0.53 mmol), cesium carbonate (694.9 mg, 2.13 mmol), and potassium iodide (177.0 mg, 1.06 mmol) were added to a solution of compound 37e (314.4 mg, 1.06 mmol) in N,N-dimethylformamide (3 mL). The reaction mixture was stirred at room temperature for 2 hours. After the reaction was completed, the reaction mixture was quenched with water (10 mL) and extracted with ethyl acetate (20 mL × 2). The combined organic phases were then washed with saturated brine (20 mL × 3), dried over anhydrous sodium sulfate, filtered, and the filtrate was concentrated under reduced pressure. The residue was purified by silica gel column chromatography (petroleum ether/ethyl acetate = 1:0 to 10:1) to obtain compound 37f (100 mg). MS m/z (ESI): 539.2 [M+1]⁺.

### Step 6: Synthesis of Compound 37-P1

Sodium hydroxide (37.1 mg, 0.93 mmol) was added to a mixed solution of compound 37f (50 mg, 0.093 mmol) in methanol/tetrahydrofuran/water (1 mL/1 mL/1 mL). The reaction mixture was stirred at 40°C for 16 hours. After the reaction was completed, the reaction mixture was added with acetic acid to adjust the pH to 5, and concentrated under reduced pressure. The residue was purified by Biotage flash chromatography (column: C18 reverse phase column; acetonitrile-water (0.1% ammonium bicarbonate); gradient: 5% to 35%) to obtain product 37-P1 (25.7 mg). MS m/z (ESI): 425.1 [M+1]⁺. Supercritical fluid chromatography (SFC): retention time = 4.451 min, UV = 254 nm. ¹HNMR (400 MHz, DMSO-*d₆*) δ 12.79 (s, 1H), 10.84 (s, 1H), 7.95 (d, *J =* 8.8 Hz, 2H), 7.63 (d, *J* = 8.0 Hz, 2H), 7.22 (t, *J =* 3.0 Hz, 1H), 6.64 (s, 1H), 6.58-6.50 (m, 1H), 5.07-4.88 (m, 1H), 3.59 (d, *J =* 12.0 Hz, 1H), 3.55-3.48 (m, 1H), 3.29-3.22 (m, 1H), 2.73-2.60 (m, 1H), 2.48-2.41 (m, 1H), 2.36 (s, 3H), 2.30-2.19 (m, 4H), 2.00-1.87 (m, 1H), 1.69-1.55 (m, 2H), 1.49-1.36 (m, 1H).

### Example 16 (Compound 38-P1)

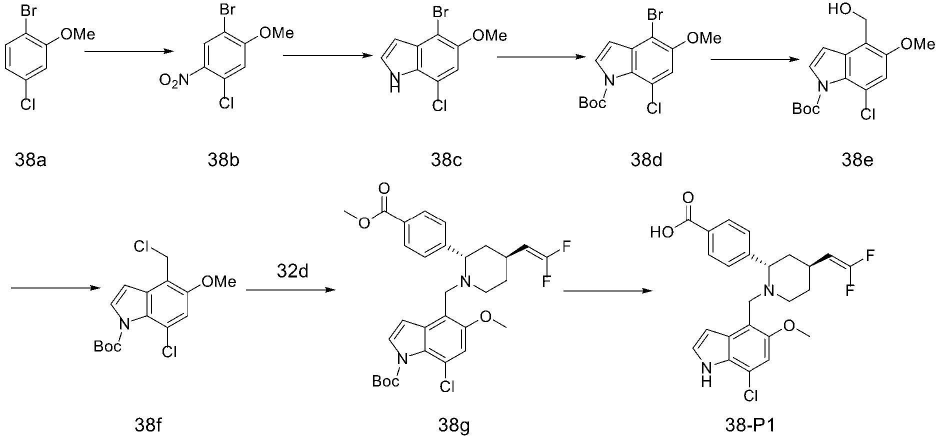

### Step 1: Synthesis of Compound 38b

Potassium nitrate (7.99 g, 79.57 mmol) was slowly added in batches to a solution of compound 38a (25 g, 113.76 mmol) in concentrated sulfuric acid (120 mL) at 0°C. The reaction mixture was stirred at 0°C for 1 hour. After the reaction was completed, the reaction mixture was poured into ice water (200 mL), resulting in the formation of a precipitate. The mixture was filtered, and the filter cake was washed with water and dried to obtain compound 38b (12 g, crude product). ¹H NMR (400 MHz, CDCl₃) δ 8.26 (s, 1H), 6.98 (s, 1H), 3.99 (s, 3H).

### Step 2: Synthesis of Compound 38c

Vinylmagnesium bromide (1.0 M, 108.7 mL, 108.71 mmol) was slowly added dropwise to a solution of compound 38b (7.20 g, 27.17 mmol) in tetrahydrofuran (240 mL) at -78°C. The reaction mixture was stirred at 0°C for 2 hours. After the reaction was completed, the reaction mixture was quenched with water (300 mL) and extracted with ethyl acetate (100 mL × 3). The combined organic phases were then washed with saturated brine (200 mL × 1), dried over anhydrous sodium sulfate, filtered, and the filtrate was concentrated under reduced pressure. The residue was purified by silica gel column chromatography (petroleum ether/ethyl acetate = 100/0 to 85/15) to obtain compound 38c (1.40 g). MS m/z (ESI): 259.9 [M+1]⁺.

### Step 3: Synthesis of Compound 38d

Di-tert-butyl dicarbonate (1.77 g, 8.10 mmol) and 4-dimethylaminopyridine (0.07 g, 0.54 mmol) were sequentially added to a solution of compound 38c (1.40 g, 5.40 mmol) in dichloromethane (20 mL) at room temperature. The reaction mixture was stirred at room temperature for 2 hours. After the reaction was completed, the reaction mixture was concentrated under reduced pressure. The residue was purified by silica gel column chromatography (petroleum ether/ethyl acetate = 1/0 to 9/1) to obtain compound 38d (1.80 g). ¹H NMR (400 MHz, CDCl₃) δ 7.58 (d, *J =* 3.6 Hz, 1H), 6.97 (s, 1H), 6.63 (d, *J =* 3.6 Hz, 1H), 3.93 (s, 3H), 1.64 (s, 9H).

### Step 4: Synthesis of Compound 38e

Tributyltin methoxide (2.26 g, 7.10 mmol) and tetrakis(triphenylphosphine)palladium (1.09 g, 0.94 mmol) were added to a solution of compound 38d (1.70 g, 4.73 mmol) in 1,4-dioxane (30 mL) under a nitrogen atmosphere. The reaction mixture was stirred at 100°C for 16 hours under a nitrogen atmosphere. After the reaction was completed, the reaction mixture was concentrated under reduced pressure. The residue was purified by silica gel column chromatography (petroleum ether/ethyl acetate = 1:0 to 4:1) to obtain compound 38e (0.65 g). MS m/z (ESI): 294.0 [M-OH]⁺.

### Step 5: Synthesis of Compound 38f

(Chloromethylene)dimethylammonium chloride (392.8 mg, 3.07 mmol) was added to a solution of compound 38e (640 mg, 2.05 mmol) in dichloromethane (15 mL) at 0°C under a nitrogen atmosphere. The reaction mixture was stirred at room temperature for 2 hours. After the reaction was completed, the reaction mixture was quenched with saturated sodium bicarbonate (10 mL) and extracted with ethyl acetate (20 mL × 2). The combined organic phases were washed with saturated brine (20 mL × 1), dried over anhydrous sodium sulfate, filtered, and the filtrate was concentrated under reduced pressure to obtain compound 38f (600 mg, crude product). MS m/z (ESI): 293.9 [M-Cl]⁺.

### Step 6: Synthesis of Compound 38g

Compound 32d (180 mg, 0.64 mmol), cesium carbonate (416.9 mg, 1.28 mmol), and potassium iodide (212.4 mg, 1.28 mmol) were added to a solution of compound 38f (423.9 mg, 1.28 mmol) in N,N-dimethylformamide (15 mL). The reaction mixture was stirred at room temperature for 2 hours. After the reaction was completed, the reaction mixture was quenched with water (10 mL) and extracted with ethyl acetate (20 mL × 2). The combined organic phases were then washed with saturated brine (20 mL × 3), dried over anhydrous sodium sulfate, filtered, and the filtrate was concentrated under reduced pressure. The residue was purified by Biotage flash chromatography (column: C18 reverse phase column; acetonitrile-water (0.1% formic acid); gradient: 5 to 50%) to obtain compound 38g (190 mg). MS m/z (ESI): 575.2 [M+1]⁺.

### Step 7: Synthesis of Compound 38-P1

Sodium hydroxide (128.4 mg, 3.21 mmol) was added to a mixed solution of compound 38g (185 mg, 0.32 mmol) in methanol/tetrahydrofuran/water (3 mL/3 mL/3 mL). The reaction mixture was stirred at 40°C for 16 hours. After the reaction was completed, the reaction mixture was added with acetic acid to adjust the pH to 5, and concentrated under reduced pressure. The residue was purified by Biotage flash chromatography (column: C18 reverse phase column; acetonitrile-water (0.1% ammonium bicarbonate); gradient: 5% to 35%) to obtain compound 38-P1 (133.7 mg). MS m/z (ESI): 461.0 [M+1]⁺. Supercritical fluid chromatography (SFC): retention time = 4.337 min, UV = 214 nm. ¹H NMR (400 MHz, DMSO-*d₆*) δ 12.83 (s, 1H), 11.20 (s, 1H), 7.95 (d, *J =* 8.4 Hz, 2H), 7.63 (d, *J =* 7.9 Hz, 2H), 7.35 (*t, J =* 2.8 Hz, 1H), 6.94 (s, 1H), 6.61-6.60 (m, 1H), 5.04-4.94 (m, 1H), 3.74 (s, 3H), 3.59-3.50 (m, 2H), 3.34-3.31 (m, 2H), 2.72-2.64 (m, 1H), 2.30-2.24 (m, 1H), 1.89-1.83 (m, 1H), 1.67-1.59 (m, 2H), 1.46-1.42 (m, 1H).

### Example 17 (Compound 39-P1)

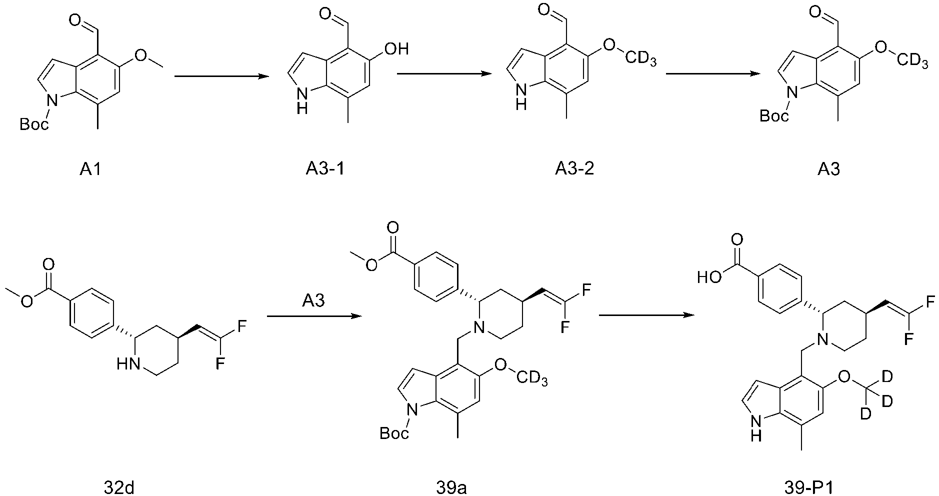

### Step 1: Synthesis of Compound A3-1

Boron tribromide (12.08 g, 48.22 mmol) was added to a solution of compound A3 (7 g, 24.11 mmol) in dichloromethane (140 mL) at 0°C. The reaction mixture was stirred at room temperature for 2 hours. After the reaction was completed, the reaction mixture was quenched with saturated sodium bicarbonate solution (50 mL) and extracted with dichloromethane (100 mL × 2). The combined organic phases were concentrated under reduced pressure, and the residue was purified by silica gel column chromatography (PE/EA = 1/0 to 2/1) to obtain compound A3-1 (1 g). MS m/z (ESI): 176.2 [M+1]⁺.

### Step 2: Synthesis of Compound A3-2

Deuterated iodomethane (893.6 mg, 6.16 mmol) and potassium carbonate (1.06 g, 7.70 mmol) were added to a solution of compound A3-1 (900 mg, 5.13 mmol) in DMF (18 mL) at room temperature. The reaction mixture was stirred at room temperature for 2 hours. After the reaction was completed, the reaction mixture was diluted with water (20 mL) and extracted with ethyl acetate (20 mL × 3). The combined organic phases were dried over anhydrous sodium sulfate, filtered, and the filtrate was concentrated under reduced pressure. The residue was purified by silica gel column chromatography (PE/EA = 1/0 to 5/1) to obtain compound A3-2 (950 mg). MS m/z (ESI): 193.1 [M+1]⁺.

### Step 3: Synthesis of Compound A3

Di-tert-butyl dicarbonate (1.18 g, 5.43 mmol), triethylamine (0.75 g, 7.41 mmol), and 4-dimethylaminopyridine (60.4 mg, 0.49 mmol) were sequentially added to a solution of compound A3-2 (0.95 g, 4.94 mmol) in dichloromethane (20 mL) at room temperature. The reaction mixture was stirred at room temperature for 2 hours. After the reaction was completed, the reaction mixture was concentrated under reduced pressure. The residue was purified by silica gel column chromatography (petroleum ether/ethyl acetate = 1/0 to 9/1) to obtain compound A3 (1.20 g). MS m/z (ESI): 293.3 [M+1]⁺.

### Step 4: Synthesis of Compound 39a

Sodium triacetoxyborohydride (1.20 g, 5.68 mmol) was added to a solution of compound 32d (400 mg, 1.42 mmol) and compound A3 (625.7 mg, 2.13 mmol) in tetrahydrofuran (10 mL) at room temperature. The reaction mixture was stirred at 40°C for 16 hours. After the reaction was completed, the reaction mixture was diluted with ethyl acetate (50 mL) and washed with saturated sodium bicarbonate solution (20 mL × 3). The organic phase was dried over anhydrous sodium sulfate, filtered, and the filtrate was concentrated under reduced pressure. The residue was purified by silica gel column chromatography (PE/EA= 1/0 to 10/1) to obtain compound 39a (0.60 g). MS m/z (ESI): 558.5 [M+1]⁺.

### Step 5: Synthesis of Compound 39-P1

Sodium hydroxide (143.2 mg, 3.58 mmol) was added to a mixed solution of compound 39a (200 mg, 0.36 mmol) in methanol/tetrahydrofuran/water (2 mL/2 mL/2 mL). The reaction mixture was stirred at 40°C for 16 hours. After the reaction was completed, the reaction mixture was added with acetic acid to adjust the pH to 5, and concentrated under reduced pressure. The residue was purified by Biotage flash chromatography (column: C18 reverse phase column; acetonitrile-water (0.1% ammonium bicarbonate); gradient: 5% to 35%) to obtain compound 39-P1 (94.6 mg). MS m/z (ESI): 444.4 [M+1]⁺. Supercritical fluid chromatography (SFC): retention time = 2.462 min, UV = 214 nm. ¹H NMR (400 MHz, DMSO-*d₆*) δ 10.81 (s, 1H), 7.95 (d, *J =* 8.0 Hz, 2H), 7.63 (d, *J =* 8.0 Hz, 2H), 7.25 (t, *J =* 2.6 Hz, 1H), 6.64 (s, 1H), 6.51-6.47 (m, 1H), 5.04-4.91 (m, 1H), 3.58 (d, *J =* 12.0 Hz, 1H), 3.51-3.47 (m, 1H), 3.29 (d, *J =* 11.6 Hz, 1H), 2.71-2.65 (m, 1H), 2.60-2.55 (m, 1H), 2.41 (s, 3H), 2.24 (t, *J* = 11.6 Hz, 1H), 1.90-1.80 (m, 1H), 1.72-1.57 (m, 2H), 1.48-1.39 (m, 1H).

### Example 18 (Compound 40-P1)

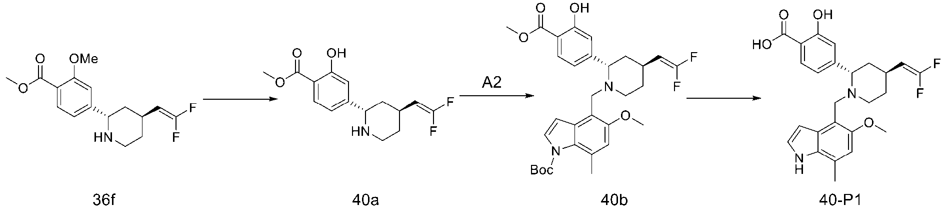

### Step 1: Synthesis of Compound 40a

Boron tribromide (1.0 M, 8.4 mL, 8.35 mmol) was added dropwise to a solution of compound 36f (260 mg, 0.83 mmol) in dichloromethane (3 mL) at 0°C. The reaction mixture was stirred at room temperature for 2 hours. After the reaction was completed, the reaction mixture was quenched with saturated sodium bicarbonate solution (50 mL) and extracted with dichloromethane (50 mL × 3). The combined organic phases were dried over anhydrous sodium sulfate, filtered, and the filtrate was concentrated under reduced pressure to obtain compound 40a (130 mg, crude product). MS m/z (ESI): 298.1 [M+1]⁺.

### Step 2: Synthesis of Compound 40b

Potassium carbonate (113.2 mg, 0.80 mmol) and potassium iodide (134 mg, 0.80 mmol) were added to a solution of compound 40a (120 mg, 0.40 mmol) and compound A2 (188.2 mg, 0.60 mmol) in DMF (2 mL). The reaction mixture was stirred at room temperature for 2 hours. After the reaction was completed, the reaction mixture was quenched with water (5 mL) and extracted with ethyl acetate (20 mL × 3). The combined organic phases were dried over anhydrous sodium sulfate, filtered, and the filtrate was concentrated under reduced pressure. The residue was purified by silica gel column chromatography (PE/EA = 1/0 to 10/1) to obtain compound 40b (120 mg). MS m/z (ESI): 571.0 [M+1]⁺.

### Step 3: Synthesis of Compound 40-P1

Sodium hydroxide (76.9 mg, 1.92 mmol) was added to a mixed solution of compound 40b (110 mg, 0.19 mmol) in methanol/tetrahydrofuran/water (1 mL/1 mL/1 mL). The reaction mixture was stirred at 40°C for 16 hours. After the reaction was completed, the reaction mixture was added with acetic acid to adjust the pH to 5, and concentrated under reduced pressure. The residue was purified by preparative high-performance liquid chromatography (chromatographic column: Gemini-C18; 150 × 21.2 mm, 5 µm; mobile phase: acetonitrile-water (0.1% trifluoroacetic acid); gradient: 20 to 40%; column temperature: 25°C; flow rate: 20 mL/min; wavelength: 214 nm; column pressure: 50 bar) to obtain compound 40-P1 (41.3 mg). MS m/z (ESI): 456.9 [M+1]⁺. Supercritical fluid chromatography (SFC): retention time = 1.933 min, UV = 214 nm. ¹H NMR (400 MHz, DMSO-*d₆*) δ 11.20 (s, 1H), 8.96 (s, 1H), 7.97 (d, *J* = 8.0 Hz, 1H), 7.48-7.40 (m, 1H), 7.36 (s, 1H), 7.21 (d, *J* = 8.4 Hz, 1H), 6.79 (s, 1H), 6.40 (s, 1H), 5.31-5.19 (m, 1H), 4.68 (t, *J =* 10.6 Hz, 1H), 4.28-4.13 (m, 2H), 3.75 (s, 3H), 3.53-3.45 (m, 1H), 3.16 (d, *J =* 12.0 Hz, 1H), 2.92-2.86 (m, 1H), 2.40 (s, 3H), 2.32-2.22 (m, 1H), 2.21-2.08 (m, 1H), 1.84 (d, *J =* 15.2 Hz, 1H), 1.65 (d, *J =* 14.8 Hz, 1H).

### Example 19 (Compound 41-P1)

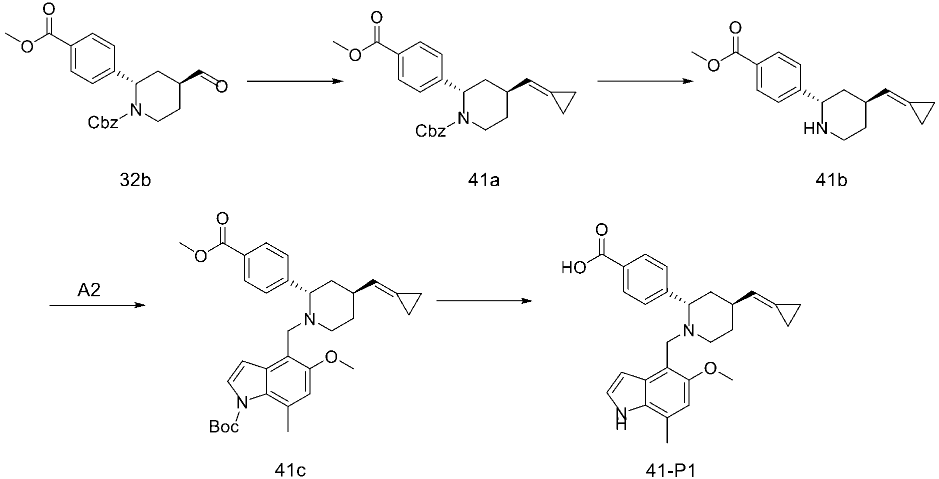

### Step 1: Synthesis of Compound 41a

Potassium tert-butoxide solution (1.0 M, 7.9 mL, 7.86 mmol) was slowly added to a solution of (3-bromopropyl)triphenylphosphonium bromide (1.83 g, 3.93 mmol) in THF (10 mL) under a nitrogen atmosphere. The mixture was stirred at 70°C for 1 hour, followed by the addition of a solution of compound 32b (1 g, 2.62 mmol) in THF (10 mL). The reaction mixture was stirred at 70°C for 3 hours. After the reaction was completed, the reaction mixture was poured into ice water (100 mL) and extracted with ethyl acetate (100 mL × 3). The combined organic phases were dried over anhydrous sodium sulfate, filtered, and the filtrate was concentrated under reduced pressure. The residue was purified by silica gel column chromatography (petroleum ether/ethyl acetate = 1/0 to 3/1) to obtain compound 41a (0.60 g). MS m/z (ESI): 406.3 [M+1]⁺.

### Step 2: Synthesis of Compound 41b

Iodotrimethylsilane (814.2 mg, 4.07 mmol) was added to a solution of compound 41a (550 mg, 1.35 mmol) in dichloromethane (10 mL) at 0°C. The reaction mixture was stirred at 0°C for 2 hours. After the reaction was completed, the reaction mixture was concentrated under reduced pressure at room temperature, and added with 1 N dilute hydrochloric acid to adjust the pH to 4 to 5. The aqueous phase was washed with petroleum ether (20 mL × 2), then added with saturated sodium bicarbonate solution to adjust the pH to 8 to 9, and extracted with dichloromethane (20 mL × 3). The combined organic phases were dried over anhydrous sodium sulfate, filtered, and the filtrate was concentrated under reduced pressure. The residue was purified by silica gel column chromatography (DCM/MeOH = 10/1) to obtain compound 41b (350 mg). MS m/z (ESI): 272.2 [M+1]⁺.

### Step 3: Synthesis of Compound 41c

Cesium carbonate (1.20 g, 3.68 mmol) and potassium iodide (305.8 mg, 1.84 mmol) were added to a solution of compound 41b (250 mg, 0.92 mmol) and compound A2 (572.6 mg, 1.84 mmol) in DMF (5 mL) at room temperature. The reaction mixture was stirred at room temperature for 2 hours. After the reaction was completed, the reaction mixture was extracted with ethyl acetate (50 mL × 3). The combined organic phases were dried over anhydrous sodium sulfate, filtered, and the filtrate was concentrated under reduced pressure. The residue was purified by silica gel column chromatography (PE/EA= 1/0 to 10/1) to obtain compound 41c (350 mg). MS m/z (ESI): 545.5 [M+1]⁺.

### Step 4: Synthesis of Compound 41-P1

Sodium hydroxide (183.2 mg, 4.58 mmol) was added to a mixed solution of compound 41c (250 mg, 0.46 mmol) in methanol/tetrahydrofuran/water (2.5 mL/2.5 mL/2.5 mL). The reaction mixture was stirred at 40°C for 16 hours. After the reaction was completed, the reaction mixture was added with acetic acid to adjust the pH to 5, and concentrated under reduced pressure. The residue was purified by Biotage flash chromatography (column: C18 reverse phase column; acetonitrile-water (0.1% ammonium bicarbonate); gradient: 10% to 30%) to obtain compound 41-P1 (152.8 mg). MS m/z (ESI): 431.4 [M+1]⁺. Supercritical fluid chromatography (SFC): retention time = 3.101 min, UV = 214 nm. ¹HNMR (400 MHz, DMSO-*d₆*) δ 12.80 (s, 1H), 10.80 (s, 1H), 7.95 (d, *J =* 8.4 Hz, 2H), 7.65 (d, *J =* 8.0 Hz, 2H), 7.25 (t, *J =* 2.8 Hz, 1H), 6.64 (s, 1H), 6.48-6.44 (m, 1H), 5.99-5.94 (m, 1H), 3.70 (s, 3H), 3.55 (d, *J =* 12.0 Hz, 1H), 3.47-3.40 (m, 1H), 3.22 (d, *J* = 12.0 Hz, 1H), 2.70-2.63 (m, 1H), 2.61-2.54 (m, 1H), 2.41 (s, 3H), 2.26-2.16 (m, 1H), 1.95-1.80 (m, 2H), 1.73-1.58 (m, 2H), 1.22-1.13 (m, 1H), 1.12-1.02 (m, 3H).

### Examples 20 (Comparative Example 1)

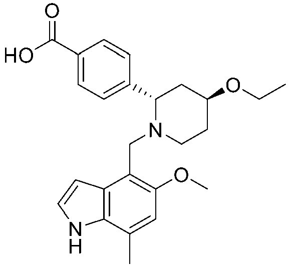

The preparation of Comparative Example 1 refers to the preparation of the control compound in WO2022028527A1. MS m/z (ESI): 423.1 [M+1]⁺. ¹H NMR (400 MHz, DMSO) δ 10.74 (s, 1H), 7.89 (d, *J =* 8.4 Hz, 2H), 7.58 (d, *J =* 7.9 Hz, 2H), 7.18 (t, *J =* 2.7 Hz, 1H), 6.58 (s, 1H), 6.41-6.35 (m, 1H), 3.64 (s, 3H), 3.55-3.42 (m, 3H), 3.36 (t, *J =* 7.0 Hz, 2H), 3.16 (d, *J =* 11.8 Hz, 1H), 2.41-2.38 (m, 1H), 2.35 (s, 3H), 2.29-2.19 (m, 1H), 1.81-1.71 (m, 1H), 1.68-1.58 (m, 2H), 1.46-1.35 (m, 1H), 1.10 (t, *J =* 7.0 **Hz, 3H).**

### Example 21 (Compound 60-P1)

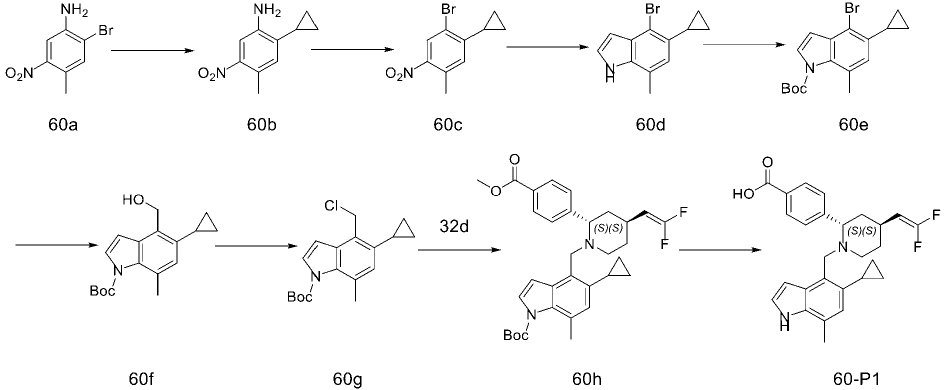

### Step 1: Synthesis of Compound 60b

[1,1'-Bis(diphenylphosphino)ferrocene]dichloropalladium(II) dichloromethane complex (8.12 g, 9.95 mmol) was added to a mixed solution of compound 60a (23 g, 99.50 mmol), cyclopropylboronic acid (12.82 g, 149.25 mmol), and potassium phosphate (63.36 g, 298.50 mmol) in 1,4-dioxane and water (300 mL/100 mL). The reaction mixture was stirred at 90°C for 16 hours. After the reaction was completed, the reaction mixture was extracted with ethyl acetate (500 mL). The combined organic phases were washed with saturated brine (200 mL × 1), dried over anhydrous sodium sulfate, filtered, and the filtrate was concentrated under reduced pressure. The residue was purified by silica gel column chromatography (petroleum ether/ethyl acetate = 1/0 to 3/1) to obtain compound 60b (15 g). MS m/z (ESI): 193.2 [M+1]⁺.

### Step 2: Synthesis of Compound 60c

tert-Butyl nitrite (16.09 g, 156.17 mmol) was slowly added to a solution of compound 60b (15 g, 78.09 mmol) in acetonitrile (300 mL) at 0°C. The mixture was stirred at 0°C for 0.5 hours, followed by the addition of copper(II) bromide (34.84 g, 156.17 mmol). The reaction mixture was stirred at room temperature for 2 hours. After the reaction was completed, the reaction mixture was diluted with water (200 mL) and extracted with ethyl acetate (500 mL × 3). The combined organic phases were concentrated under reduced pressure, and the residue was purified by silica gel column chromatography (petroleum ether/ethyl acetate = 1/0 to 10/1) to obtain compound 60c (15 g). ¹HNMR (400 MHz, CDCl₃) δ 8.22 (s, 1H), 6.79 (s, 1H), 2.53 (s, 3H), 2.24-2.18 (m, 1H), 1.17-1.11 (m, 2H), 0.79-0.74 (m, 2H).

### Step 3: Synthesis of Compound 60d

Vinylmagnesium bromide (1.0 M, 312.4 mL, 312.4 mmol) was slowly added dropwise to a solution of compound 60c (20 g, 78.43 mmol) in tetrahydrofuran (400 mL) at -78°C. The reaction mixture was stirred at 0°C for 2 hours. After the reaction was completed, the reaction mixture was quenched with water (100 mL) and extracted with ethyl acetate (200 mL × 3). The combined organic phases were then washed with saturated brine (100 mL × 1), dried over anhydrous sodium sulfate, filtered, and the filtrate was concentrated under reduced pressure. The residue was purified by silica gel column chromatography (petroleum ether/ethyl acetate = 100/0 to 80/20) to obtain compound 60d (5 g). MS m/z (ESI): 248.1 [M-1]⁺.

### Step 4: Synthesis of Compound 60e

Di-tert-butyl dicarbonate (6.54 g, 29.98 mmol) and 4-dimethylaminopyridine (0.24 g, 1.99 mmol) were sequentially added to a solution of compound 60d (5 g, 19.98 mmol) in dichloromethane (100 mL) at room temperature. The reaction mixture was stirred at room temperature for 2 hours. After the reaction was completed, the reaction mixture was concentrated under reduced pressure. The residue was purified by silica gel column chromatography (petroleum ether/ethyl acetate = 1/0 to 10/1) to obtain compound 60e (6.50 g). ¹H NMR (400 MHz, CDCl₃) δ 7.52 (d, *J* = 3.6 Hz, 1H), 6.66 (s, 1H), 6.63 (d, *J* = 3.6 Hz, 1H), 2.55 (s, 3H), 2.24-2.16 (m, 1H), 1.62 (s, 9H), 1.03-0.98 (m, 2H), 0.71-0.67 (m, 2H).

### Step 5: Synthesis of Compound 60f

Tributyltin methoxide (548.4 mg, 1.71 mmol) and methanesulfonato(2-dicyclohexylphosphino-2',4',6'-tri-i-propyl-1,1'-biphenyl)(2'-amino-1,1'-biphenyl-2-yl)palladium(II) (96.3 mg, 0.11 mmol) were added to a solution of compound 60e (400 mg, 1.13 mmol) in 1,4-dioxane (8 mL) under a nitrogen atmosphere. The reaction mixture was stirred at 80°C for 16 hours. After the reaction was completed, the reaction mixture was concentrated under reduced pressure. The residue was purified by silica gel column chromatography (petroleum ether/ethyl acetate = 1:0 to 3:1) to obtain compound 60f (200 mg). MS m/z (ESI): 284.3 [M-OH]⁺.

### Step 6: Synthesis of Compound 60g

(Chloromethylene)dimethylammonium chloride (127.0 mg, 0.99 mmol) was added to a solution of compound 60f (200 mg, 0.66 mmol) in dichloromethane (4 mL) at 0°C. The reaction mixture was stirred at room temperature for 2 hours. After the reaction was completed, the reaction mixture was quenched with saturated sodium bicarbonate (10 mL) and extracted with ethyl acetate (20 mL × 2). The combined organic phases were washed with saturated brine (20 mL × 1), dried over anhydrous sodium sulfate, filtered, and the filtrate was concentrated under reduced pressure to obtain compound 60g (200 mg, crude product). MS m/z (ESI): 284.2 [M-Cl]⁺.

### Step 7: Synthesis of Compound 60h

Compound 60g (200 mg, 0.71 mmol), cesium carbonate (926.6 mg, 2.84 mmol), and potassium iodide (236.1 mg, 1.42 mmol) were added to a solution of compound 32d (456.2 mg, 1.42 mmol) in N,N-dimethylformamide (10 mL). The reaction mixture was stirred at room temperature for 2 hours. After the reaction was completed, the reaction mixture was quenched with water (50 mL) and extracted with ethyl acetate (50 mL × 2). The combined organic phases were then washed with saturated brine (50 mL × 3), dried over anhydrous sodium sulfate, filtered, and the filtrate was concentrated under reduced pressure. The residue was purified by silica gel column chromatography (petroleum ether/ethyl acetate = 1:0 to 10:1) to obtain compound 60h (200 mg). MS m/z (ESI): 565.5 [M+1]⁺.

### Step 8: Synthesis of Compound 60-P1

Sodium hydroxide (127.3 mg, 3.18 mmol) was added to a mixed solution of compound 60h (180 mg, 0.32 mmol) in methanol, tetrahydrofuran, and water (3 mL/3 mL/3 mL). The reaction mixture was stirred at 40°C for 16 hours. After the reaction was completed, the reaction mixture was added with AcOH to adjust the pH to 5, and concentrated under reduced pressure. The residue was purified by preparative high-performance liquid chromatography (chromatographic column: Xbridge-C18; 19 × 150 mm, 5 µm; mobile phase: acetonitrile-water (0.05% ammonia); gradient: 50 to 90%; column temperature: 25°C; flow rate: 20 mL/min; wavelength: 214 nm; column pressure: 60 bar) to obtain compound 60-P1 (44.3 mg). MS m/z (ESI): 451.4 [M+1]⁺. Supercritical fluid chromatography (SFC): retention time = 3.164 min, UV = 254 nm. ¹H NMR (400 MHz, DMSO-*d₆*) δ 10.83 (s, 1H), 7.91 (d, *J* = 8.0 Hz, 2H), 7.57 (d, *J* = 8.0 Hz, 2H), 7.23 (t, *J* = 2.8 Hz, 1H), 6.64-6.54 (m, 1H), 6.44 (s, 1H), 5.06-4.89 (m, 1H), 3.79 (d, *J* = 12.0 Hz, 1H), 3.54-3.44 (m, 2H), 2.72-2.65 (m, 1H), 2.57-2.52 (m, 1H), 2.41-2.25 (m, 4H), 2.24-2.15 (m, 1H), 2.02-1.90 (m, 1H), 1.73-1.56 (m, 2H), 1.50-1.38 (m, 1H), 0.89-0.77 (m, 1H), 0.73-0.61 (m, 1H), 0.60-0.50 (m, 1H), 0.25-0.14 (m, 1H).

### Example 22 (Compound 61-P1)

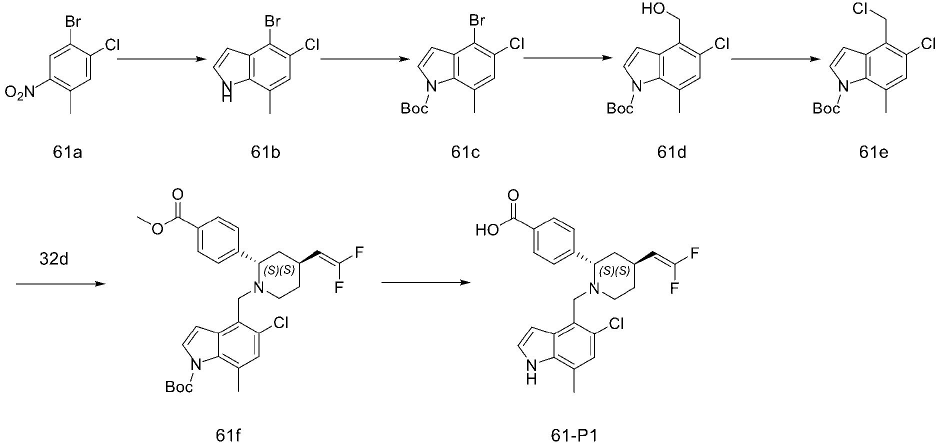

### Step 1: Synthesis of Compound 61b

Vinylmagnesium bromide (1.0 M, 143 mL, 143.28 mmol) was slowly added dropwise to a solution of compound 61a (9.70 g, 38.72 mmol) in tetrahydrofuran (200 mL) at -78°C. The reaction mixture was stirred at 0°C for 2 hours. After the reaction was completed, the reaction mixture was quenched with water (100 mL) and extracted with ethyl acetate (100 mL × 3). The combined organic phases were then washed with saturated brine (200 mL × 1), dried over anhydrous sodium sulfate, filtered, and the filtrate was concentrated under reduced pressure. The residue was purified by silica gel column chromatography (petroleum ether/ethyl acetate = 100/0 to 80/20) to obtain compound 61b (4.55 g). MS m/z (ESI): 244.0 [M+1]⁺.

### Step 2: Synthesis of Compound 61c

Di-tert-butyl dicarbonate (6.09 g, 27.91 mmol) and 4-dimethylaminopyridine (0.23 g, 1.86 mmol) were sequentially added to a solution of compound 61b (4.55 g, 18.60 mmol) in dichloromethane (50 mL) at room temperature. The reaction mixture was stirred at room temperature for 2 hours. After the reaction was completed, the reaction mixture was concentrated under reduced pressure. The residue was purified by silica gel column chromatography (petroleum ether/ethyl acetate = 1/0 to 10/1) to obtain compound 61c (3.60 g). MS m/z (ESI): 243.6 [M-100]⁺.

### Step 3: Synthesis of Compound 61d

Tributyltin methoxide (4.87 g, 15.19 mmol) and tetrakis(triphenylphosphine)palladium (2.34 g, 2.02 mmol) were added to a solution of compound 61c (3.50 g, 10.12 mmol) in 1,4-dioxane (30 mL) under a nitrogen atmosphere. The reaction mixture was stirred at 100°C for 16 hours under a nitrogen atmosphere. After the reaction was completed, the reaction mixture was concentrated under reduced pressure. The residue was purified by silica gel column chromatography (petroleum ether/ethyl acetate = 1:0 to 3:1) to obtain compound 61d (0.63 g). MS m/z (ESI): 278.0 [M-OH]⁺.

### Step 4: Synthesis of Compound 61e

(Chloromethylene)dimethylammonium chloride (355.8 mg, 2.78 mmol) was added to a solution of compound 61d (550 mg, 1.85 mmol) in dichloromethane (10 mL) at 0°C. The reaction mixture was stirred at room temperature for 2 hours. After the reaction was completed, the reaction mixture was quenched with saturated sodium bicarbonate (10 mL) and extracted with ethyl acetate (20 mL × 2). The combined organic phases were washed with saturated brine (20 mL × 1), dried over anhydrous sodium sulfate, filtered, and the filtrate was concentrated under reduced pressure to obtain compound 61e (500 mg, crude product). MS m/z (ESI): 278.0 [M-Cl]⁺.

### Step 5: Synthesis of Compound 61f

Compound 32d (200.8 mg, 0.71 mmol), cesium carbonate (930.3 mg, 2.85 mmol), and potassium iodide (473.9 mg, 2.85 mmol) were added to a solution of compound 61e (450 mg, 1.43 mmol) in N,N-dimethylformamide (6 mL). The reaction mixture was stirred at room temperature for 2 hours. After the reaction was completed, the reaction mixture was quenched with water (10 mL) and extracted with ethyl acetate (20 mL × 2). The combined organic phases were then washed with saturated brine (20 mL × 3), dried over anhydrous sodium sulfate, filtered, and the filtrate was concentrated under reduced pressure. The residue was purified by silica gel column chromatography (petroleum ether/ethyl acetate = 1:0 to 10:1) to obtain compound 61f(165 mg). MS m/z (ESI): 559.3 [M+1]⁺.

### Step 6: Synthesis of Compound 61-P1

Sodium hydroxide (110.7 mg, 2.77 mmol) was added to a mixed solution of compound 61f (155 mg, 0.28 mmol) in methanol/tetrahydrofuran/water (1 mL/1 mL/1 mL). The reaction mixture was stirred at 40°C for 16 hours. After the reaction was completed, the reaction mixture was added with AcOH to adjust the pH to 5, and concentrated under reduced pressure. The residue was purified by preparative high-performance liquid chromatography (chromatographic column: Gemini-C18; 150 × 21.2 mm, 5 µm; mobile phase: acetonitrile-water (0.1% formic acid); gradient: 25 to 50%; column temperature: 25°C; flow rate: 15 mL/min; wavelength: 214 nm; column pressure: 80 bar) to obtain compound 61-P1 (75 mg). MS m/z (ESI): 445.1 [M+1]⁺. Supercritical fluid chromatography (SFC): retention time = 2.824 min, UV = 254 nm. ¹HNMR (400 MHz, DMSO-*d₆*) δ 12.83 (s, 1H), 11.20 (s, 1H), 8.14 (s, 0.25H), 7.94 (d, *J* = 8.4 Hz, 2H), 7.66 (d, *J* = 8.0 Hz, 2H), 7.36 (t, *J* = 2.6 Hz, 1H), 6.87 (s, 1H), 6.73-6.60 (m, 1H), 5.07-4.87 (m, 1H), 3.68 (d, *J* = 12.0 Hz, 1H), 3.63-3.55 (m, 1H), 3.50 (d, *J* = 12.4 Hz, 1H), 2.74-2.63 (m, 1H), 2.57-2.52 (m, 1H), 2.41 (s, 3H), 2.37-2.29 (m, 1H), 2.03-1.87 (m, 1H), 1.73-1.58 (m, 2H), 1.54-1.41 (m, 1H).

### Biological Examples

### 1. Complement-mediated Hemolytic Activity Assay

The hemolysis assay was performed with reference to the method described in Xuan Yuan et al., Haematologica. (2017) 102: 466-475. Prior to the assay, the optimal concentration of normal human serum (NHS) required to achieve 100% lysis of rabbit erythrocytes (RE) was determined by titration. In this assay, NHS (collected from healthy volunteers) was diluted in GVB⁰ buffer containing 10 mM Mg-EGTA (0.1% gelatin, 5 mM Veronal, 145 mM NaCl, 0.025% NaN₃, pH 7.3, Complement Technology) and incubated with various concentrations of test compounds at 37°C for 15 minutes. RE (collected from healthy Japanese white rabbits), freshly suspended in GVB⁰ buffer containing 10 mM Mg-EGTA, were then added to achieve a final concentration of 1 × 10⁸ cells/mL and incubated at 37°C for 30 minutes. The positive control group (100% lysis) consisted of GVB⁰ buffer containing 10 mM Mg-EGTA with NHS and RE but without test compounds. The negative control group (0% lysis) consisted of GVB⁰ buffer containing 10 mM Mg-EGTA with inactivated NHS (heated at 56°C for 30 minutes or 65°C for 5 minutes) and RE but without test compounds. After centrifugation of the samples at 2000 g for 5 minutes, the supernatant was collected. Absorbance at 415 nm (A415) was measured using a microplate reader (Molecular Devices, SpectraMax i3X). IC₅₀ values were calculated by nonlinear regression from the percentage of hemolysis as a function of test compound concentration. The test results are shown in Table 1.

**Table 1**

| Example No. | Compound No. | Hemolysis IC₅₀ (nM) |
|---|---|---|
| Example 1 | Compound 1 | 86.16 |
| Example 10 | Compound 32-P1 | 94.25 |
| Example 11 | Compound 33-P1 | 82.25 |
| Example 12 | Compound 34-1 | 61.62 |
| Example 13 | Compound 35-P1 | 61.49 |
| Example 14 | Compound 36-P1 | 43.98 |
| Example 15 | Compound 37-P1 | 20.74 |
| Example 16 | Compound 38-P1 | 139.30 |
| Example 17 | Compound 39-P1 | 113.40 |
| Example 18 | Compound 40-P1 | 21.82 |
| Example 19 | Compound 41-P1 | 34.79 |
| Example 2 | Compound 19 | 2787 |
| Example 21 | Compound 60-P1 | 115.5 |

### 2. Wieslab Alternative Complement Pathway Activation and Inhibition Assay

The activity of the alternative complement pathway in monkey serum was tested using a Wieslab assay kit (WIESLAB^{®} Complement System Alternative Pathway, AP330RUO). A 96-well plate was removed from the kit as needed and equilibrated at room temperature for 30 minutes, while the remaining plates were sealed and stored at 4°C. The positive control (untreated monkey serum), negative control (100 µL of untreated monkey serum, heated in a water bath at 56°C for 30 minutes), and test samples were diluted 18-fold with Diluent AP provided in the kit and added to the plate at 100 µL/well. 100 µL of Diluent AP was used as a blank control. The plate was sealed with a transparent sealing film, covered with a lid, and incubated at 37°C for 65 minutes. After incubation, the liquid in the wells was discarded, and the plate was washed with 1 × wash buffer (300 µL/well) three times, with the liquid being removed as thoroughly as possible from the plate after the final wash. 100 µL of conjugate was added to each well, and the plate was incubated at room temperature (20 to 25°C) for 30 minutes, followed by repetition of the washing step. 100 µL of substrate solution was then added to each well, and the plate was incubated at room temperature (20 to 25°C) for 30 minutes in the dark. The OD405 value was read immediately, or the reaction was terminated by addition of 5 mM EDTA, and the OD405 value was read within 60 minutes. Complement activity was calculated according to the formula: % complement activity = (Sample - NC) / (PC - NC) × 100.

### 3. TR-FRET Binding Assay

A competitive binding assay using a Cy5 fluorescently labeled small molecule inhibitor as a probe was employed to screen compounds for their inhibitory effect on binding to human complement factor B. Complement factor B was incubated with EZ-Link^{™} Sulfo-NHS-LC-LC-Biotin at a 1:2 ratio on ice for 1 hour, after which the reaction was terminated by addition of 1 M Tris (pH 7.5). Subsequently, the reaction mixture was purified twice using a 2 mL Zeba^{™} desalt spin column to obtain biotin-labeled complement factor B (according to the EZ-Link^{™} Sulfo-NHS-LC-Biotin instructions). During the assay, biotin-labeled complement factor B at a final concentration of 10 nM was pre-incubated with various concentrations of the compounds in buffer at room temperature for 1 hour. The reaction was initiated by addition of a Cy5 fluorescently labeled probe and europium chelate-labeled streptavidin (PerkinElmer, #AD0060) at final concentrations of 75 nM and 5 nM, respectively. Kinetic readings were performed on a microplate reader (337 nm excitation, 665 nm emission, 70 µs time-gated) to obtain time-resolved fluorescence energy transfer (TR-FRET) data for determining IC₅₀ values.

### 4. Optical Surface Plasmon Resonance (SPR) Binding Assay

An SPR assay was conducted at 25°C using PBS buffer supplemented with 0.05% (v/v) P20 and 5% DMSO as the running buffer. The analysis was performed on a Biacore 8K instrument (GE Healthcare). A CM7 chip (GE Healthcare) was activated with 400 mM EDC and 100 mM NHS at a flow rate of 30 µL/min for 420 seconds. Complement factor B was diluted to 50 µg/mL in 10 mM sodium acetate (pH 4.0) and covalently immobilized onto the chip at a flow rate of 10 µL/min for 1200 seconds (resulting in a protein immobilization level of 25000 RU). The chip was then blocked with 1 M ethanolamine hydrochloride at a flow rate of 10 µL/min for 300 seconds. Test compounds were injected at a concentration of 500 µM, with an association time of 120 seconds and a dissociation time of 300 seconds. Data analysis was performed using a 1:1 binding model (Biacore Insight Evaluation Software, Version 2.0.15.12933).

Conclusion: Based on the optical surface plasmon resonance binding assay, the compounds of the present disclosure exhibit significantly superior binding affinity compared to the comparative example.

### 5. Evaluation of the Inhibitory Effect of Test Compounds on the Alternative Pathway of the Human Serum Complement System

Test compounds were evaluated starting at a concentration of 10 µM, with 3-fold serial dilutions across 8 concentration points. The final concentration of DMSO was 0.1%, and all measurements were performed in duplicate. A vial of human serum (Complement Technology, NHS) was removed from a -80°C ultra-low temperature freezer and allowed to thaw completely on ice. The samples were gently mixed by pipetting and diluted with Diluent AP (WIESLAB^{®} Complement System Alternative Pathway, AP330RUO) to prepare a 1.25× human serum working solution. In parallel, an aliquot of human serum was inactivated by heating in a water bath at 56°C for 30 minutes and diluted in the same manner to prepare a 1.25× inactivated working solution to serve as the NC control. In a 96-well plate, 25 µL of compound working solution and 100 µL of the 1.25× human serum working solution were sequentially added to each well. For the PC control wells, 25 µL of 5‰ DMSO and 100 µL of the 1.25× human serum working solution were added. For the negative control wells, 25 µL of 5‰ DMSO and 100 µL of the 1.25× inactivated human serum working solution were added. The plate was gently mixed on a microplate thermoshaker and incubated at room temperature for 15 minutes. Subsequently, 100 µL of the above mixture was carefully transferred to the pre-coated (LPS-containing) plate provided in the kit (WIESLAB^{®} Complement System Alternative Pathway, AP330RUO). Additionally, 100 µL of Diluent AP was used as a blank control to avoid bubble formation. The plate was incubated at 37°C for 65 minutes. After incubation, the liquid in the wells was discarded, and the plate was washed with 1× wash buffer (300 µL/well) four times, with the liquid being removed as thoroughly as possible from the plate after the final wash. 100 µL of conjugate was added to each well, and the plate was incubated at room temperature (20 to 25°C) for 30 minutes, followed by repetition of the washing step. 100 µL of substrate solution was then added to each well, and the plate was incubated at room temperature (20 to 25°C) for 30 minutes in the dark. The OD405 value was read immediately, or the reaction was terminated by addition of 5 mM EDTA, and the OD405 value was read within 60 minutes. Complement activity was calculated according to the formula: % complement activity = (Sample - NC) / (PC - NC) × 100, and data analysis was performed. The test results are shown in Table 2.

**Table 2**

| Example No. | Compound No. | IC₅₀ (nM) |
|---|---|---|
| Example 1 | Compound 1 | 53.22 |
| Example 10 | Compound 32-P1 | 47.27 |
| Example 11 | Compound 33-P1 | 50.37 |
| Example 20 | Comparative Example 1 | 142.00 |

### 6. Liver Microsomal Stability Assay

### (1) Preparation of Buffer

A 0.1 M dipotassium hydrogen phosphate aqueous solution (containing 1 mM ethylenediaminetetraacetic acid) was prepared, and the pH was adjusted to 7.4 using a 0.1 M potassium dihydrogen phosphate aqueous solution (containing 1 mM ethylenediaminetetraacetic acid).

### (2) Microsome Sources and Preparation of Working Solutions

Microsome Sources:
Mouse: ICR/CD-1 Liver Microsomes, Cat. No.: M00501, BioreclamationIVT.
Rat: SD Rat Liver Microsomes, Cat. No.: LM-DS-02M, RILD Research Institute for Liver Diseases (Shanghai) Co., Ltd.
Dog: Beagle Dog Liver Microsomes, Cat. No.: M00201, BioreclamationIVT.
Monkey: Cynomolgus Monkey Liver Microsomes, Cat. No.: LM-SXH-02M, RILD Research Institute for Liver Diseases (Shanghai) Co., Ltd.
Human: Pooled Human Liver Microsomes (Mongolian), Cat. No.: LM-R-02M, RILD Research Institute for Liver Diseases (Shanghai) Co., Ltd.

### Preparation of Working Solutions

Each test compound was dissolved in DMSO to prepare a 10 mM solution, 10 µL of which was then added to 190 µL of acetonitrile to prepare a 0.5 mM stock solution. 1.5 µL of the 0.5 mM compound stock solution was mixed with 20 mg/mL liver microsomes at a concentration of 18.75 µM, followed by the addition of 479.75 µL of buffer (the actual preparation volume may be adjusted as required).

### (3) Experimental Procedure

NADPH was prepared at a concentration of 10 mg/mL in buffer. A 96-well plate was placed on ice. For each compound, wells corresponding to different time points (0, 10, 30, 60, and 90 minutes, as well as a non-NADPH control) were set up, with 30 µL of the working solution added to each well. For the 0-minute wells, 155 µL of ice-cold acetonitrile solution (containing an internal standard at a concentration of 1 µM) was added first, mixed by pipetting, followed by the addition of 15 µL of NADPH (10 mg/mL). Prior to initiating the reaction, the 96-well plate was pre-incubated on a microplate thermoshaker at 37°C for 5 minutes. The metabolic reaction was then initiated by adding 15 µL of NADPH (10 mg/mL) to each well. After 10, 30, 60, and 90 minutes, the reaction was terminated by adding 155 µL of ice-cold acetonitrile solution (containing an internal standard at a concentration of 1 µM) to the corresponding wells. For the Non-NADPH system, the reaction was terminated after 90 minutes by adding 155 µL of ice-cold acetonitrile solution (containing an internal standard at a concentration of 1 µM). After the reaction was completed, the 96-well plate was shaken on a microplate shaker (600 rpm) for 10 minutes, followed by centrifugation at 4°C and 4000 g for 15 minutes. 50 µL of the supernatant was transferred to a new 2 mL 96-well plate, followed by the addition of 300 µL of deionized water. The samples were analyzed using an AB SCIEX ExionLC-Triple Quad 5500 high-performance liquid chromatography-mass spectrometry system by Analyst 1.6.3 software.

### 7. Hepatocyte Stability Assay

**(1) Reagent Information**
   Williams E Medium: Gibco, Lot No.: 2336217.
   GlutaMAX (100×): Gibco, Lot No.: 22777239.
   Dexamethasone: NICPBP.
   Human insulin: Gibco, 12585-014.
   FBS: Corning, 35-076-CV.
   DPBS: Gibco, 2224922.
   HEPES: Sigma, RNBH8969.
   Isotonic Percoll: Cytiva, 10303640.
**(2) Cell Sources and Preparation of Working Solutions**
   Cell Sources
   Human: BioIVT, Product No.: X008001;
   Monkey: Cynomolgus, RILD, Product No.: HP-SXH-02M;
   Dog: Beagle, TPCS, Product No.: CDH-100BE-SQ;
   Rat: SD, BioIVT, Product No.: M00005;
   Mouse: CD-1, TPCS, Product No.: CMH-100CD-SQ.
   Preparation of Working Solutions
   Compounds were diluted with DMSO to 10 mM and further diluted with 50% acetonitrile in water to 100 µM.
**(3) Experimental Procedure**
   Incubation medium and thawing medium were pre-warmed at 37°C for 15 minutes. Hepatocytes were removed from a liquid nitrogen tank, thawed at 37°C, and transferred to thawing medium. After brief centrifugation, the cells were transferred to a biosafety cabinet. Cell count and viability were determined by AO/PI staining. The cells were diluted with incubation medium to a working concentration of 0.5 × 10⁶ cells/mL. 2 µL of compound working solution was added to 198 µL of hepatocyte suspension. After mixing, the cells were incubated at 37°C under 5% CO₂. Samples were collected at reaction time points of 0.5, 15, 30, 60, 90, and 120 minutes. At each time point, 25 µL of the reaction mixture was added to 150 µL of ACN containing internal standards (100 nM alprazolam, 200 nM caffeine, and 100 nM tolbutamide) and vortexed for 5 minutes to quench. All samples were centrifuged at 3220 g for 45 minutes. 100 µL of the supernatant was mixed with 100 µL of deionized water and analyzed by LC/MS/MS using a Triple Quad^{™} 5500 high-performance liquid chromatography-mass spectrometry system by Analyst 1.6.3 software.

### 8. Protein Binding Assay

### Preparation of Buffer

Phosphate-buffered saline: Cat. No.: E607008-0500, BBI.

### (1) Plasma Sources and Preparation of Working Solutions

Plasma Sources:
Human plasma, purchased from Wuhan Chundu Biotechnology Co., Ltd.
Animal plasma, collected in-house or sourced from CRO.

### (2) Preparation of Working Solutions:

Preparation of compound working solution 1: 1 µL of compound DMSO stock solution (10 mM) was transferred to 99 µL of ACN to prepare compound working solution 1 with a concentration of 200 µM. An appropriate amount of compound working solution 1 was then added to an appropriate volume of plasma (or serum) and mixed to obtain working solution 2 with a final concentration of 1 µM.

### (3) Experimental Procedure

The equilibrium dialysis system was inserted into the corresponding wells of a RED Devices plate. 100 µL of working solution 2 was added to the red plasma chamber, and 350 µL of PBS was added to the buffer chamber. The equilibrium dialysis plate was sealed with a sealing film and incubated at 37°C in a thermoshaker at 250 rpm for approximately 4 hours. After incubation, a certain volume (typically a multiple of 25 µL) of samples were transferred from both the buffer chamber and the plasma chamber to centrifuge tubes or deep-well plates (stored at -20°C) for later use. 25 µL each of the dialyzed plasma samples and buffer samples were added to a 96-well plate. 25 µL of PBS was added to the plasma samples while 25 µL of plasma was added to the buffer samples. 300 µL of pre-prepared ice-cold precipitation solution containing an internal standard (tolbutamide, with a final concentration of 1 µM) was then added, and the mixture was shaken at 400 rpm for 5 minutes. Preparation of T0 samples: 25 µL of working solution 2 prepared in 2.2.2 was added to a 96-well plate, followed by the addition of 25 µL of PBS. After mixing, 300 µL of ice-cold precipitation solution containing an internal standard (tolbutamide) was added, and the mixture was shaken at 400 rpm for 5 minutes. All samples were prepared in duplicate. The precipitated samples were centrifuged at 4°C and 4000 rpm for 15 minutes. After centrifugation, the supernatant was diluted 4-fold with ultrapure water. The samples were analyzed using an AB SCIEX ExionLC-Triple Quad 5500 high-performance liquid chromatography-mass spectrometry system by Analyst 1.6.3 software.

### 9. Caco-2 Permeability Assay

### (1) Sources and Preparation of Buffers and Cell Culture Media

HBSS: Sigma-Aldrich, Cat#: H8264;
FBS: Gibco, Cat#: 10091148;
HEPES: Gibco, Cat#: 15630106;
DMEM: Gibco, Cat#: 11995-065;
Trypsin: Gibco, Cat#: 25200-056;
DPBS: Gibco, Cat#: 14190-144;
MEM NEAA: Gibco, Cat#: 11140050;
Pen Strep: Gibco, Cat#: 15140-122;
24-Multiwell insert system: Corning, Cat#: 351181;
24-Multiwell cell plate: Corning, Cat#: 353047.
Preparation of cell culture medium: 50 mL of FBS, 5 mL of Pen Strep, and 5 mL of MEM NEAA were added to DMEM (500 mL per culture flask), followed by thorough mixing.
Preparation of experimental HBSS (containing HEPES): 12.5 mL of HEPES was added to 487.5 mL of HBSS, and the pH was adjusted to 7.4.

### (2) Cell Culture and Preparation of Working Solutions

The culture medium was aspirated from the culture flask using a sterile pipette tip. 10 mL of DPBS was added to the flask for rinsing the cells. The rinsing step was repeated once with DPBS. The cells were rinsed once with 1 mL of 0.25% Trypsin, followed by the addition of 2 mL of 0.25% Trypsin. The flask was gently shaken, covered with a lid, and placed in an incubator for digestion until the cells detached. 8 mL of culture medium was aspirated and mixed with the cells, and the cell suspension was transferred to a 15 mL centrifuge tube, followed by centrifugation at 1000 rpm for approximately 5 minutes. The supernatant medium was discarded, and 10 mL of culture medium was added to resuspend the cells. Approximately 1 mL of the digested cell suspension was transferred to a 1.5 mL centrifuge tube. 20 µL of the cell suspension was mixed with 20 µL of trypan blue solution, and 10 µL of the mixture was loaded onto a cell counting chamber. Data provided by the cell counter were recorded. Based on the number of viable cells, the mixture was diluted with DMEM to a concentration of 3.2 × 10⁴ cells/mL, and 400 µL of the cell suspension was added to each well. The plate was gently tapped around the edges. 30 mL of MEM was added to the lower chamber. The edges of the cell plate were wiped with alcohol swabs and transferred to an incubator for culture at 37°C under 5% CO₂. The culture medium was replaced every 3 to 4 days. Transepithelial electrical resistance was measured using a Millipore resistance meter to assess cell status. When the resistance exceeded 600 Ω, the cells were used for subsequent experiments. Cell passage number, seeding date, and cell count for each experiment were recorded for future reference.

Compounds were diluted with DMSO to 10 mM. 5 µL of the 10 mM compound solution was added to 5 mL of HBSS (containing HEPES) to prepare a compound working solution.

### (3) Experimental Procedure

HBSS (containing HEPES) was pre-warmed to 37°C. Transepithelial electrical resistance was measured and recorded. The culture medium was removed from the cell plate, and the cells were rinsed once with HBSS (containing HEPES). The compound working solution was added to the donor compartment and HBSS (containing HEPES) solution was added to the receiver compartment. The cell plate was equilibrated at 37°C under 5% CO₂ for 10 minutes. After equilibration, 100 µL of samples were collected from both the donor and receiver compartments as 0-minute samples. The cell plate was further equilibrated at 37°C under 5% CO₂ for 90 minutes. Samples were collected from both the donor and receiver compartments as 90-minute samples. 50 µL each of the 0-minute and 90-minute samples were added to 300 µL of ACN solution (containing 10 ng/mL tolbutamide as an internal standard) and vortexed thoroughly. The samples were centrifuged at 4000 rpm and 4°C for 10 minutes. 100 µL of the supernatant was mixed with 100 µL of deionized water and subjected to analysis. The samples were analyzed using an AB SCIEX ExionLC-Triple Quad 5500 high-performance liquid chromatography-mass spectrometry system by Analyst 1.6.3 software.

Conclusion: The compounds of the present disclosure exhibit significantly superior permeability compared to the comparative example.

### 10. Single-Dose PK Study in Mice

Male ICR mice (Shanghai Bikai Keyi Biotechnology Co., Ltd.) weighing approximately 25 g were fasted overnight. Three mice per group were administered the test compounds by oral gavage at a dosing volume of 10 mL/kg. Blood samples (30 µL per time point) were collected via the orbital sinus into EDTA-K2 anticoagulant tubes and immediately centrifuged at 4000 rpm for 5 minutes at 4°C. The supernatants were collected. Blood samples were collected at the following time points: 15 minutes, 30 minutes, 1 hour, 2 hours, 4 hours, 7 hours, and 24 hours. The animals were continuously monitored for general condition after dosing. After completion of blood collection at all time points, the animals were euthanized. Plasma samples were analyzed by LC-MS/MS, and pharmacokinetic parameters were calculated using WinNonlin software. The results are shown in Table 3.

**Table 3**

| Example No. | Compound No. | Route of administration | Maximum plasma concentration Cₘₐₓ (ng/mL) | Half-life T_{1/2} (h) | Area under the curve AUC₀₋₂₄ₕ (hr·ng/mL) | Clearance CL (mL/hr/kg) |
|---|---|---|---|---|---|---|
| Example 1 | Compound 1 | Oral gavage 10 mg/kg | 1532.97 | 13.07 | 9229.18 | 841.87 |

### 11. Single-Dose PK Study in Rats

### Experimental Method:

Male SD rats (Shanghai Bikai Keyi Biotechnology Co. Ltd.) aged 6 to 9 weeks were fasted overnight. Three mice per group were administered the test compounds by oral gavage at a dosing volume of 10 mL/kg. Blood samples (0.2 mL per time point) were collected via the jugular vein into EDTA-K2 anticoagulant tubes and immediately centrifuged at 4000 rpm for 5 minutes at 4°C. The supernatants were collected. Blood samples were collected at the following time points: 15 minutes, 30 minutes, 1 hour, 2 hours, 4 hours, 7 hours, and 24 hours. The animals were continuously monitored for general condition after dosing. After completion of blood collection at all time points, the animals were euthanized. Plasma samples were analyzed by LC-MS/MS, and pharmacokinetic parameters were calculated using WinNonlin software. The results are shown in Table 4.

| Example No. | Compound No. | Route of administration | Maximum plasma concentration Cₘₐₓ (ng/mL) | Half-life T_{1/2} (h) | Area under the curve AUC₀₋₂₄ₕ (hr·ng/mL) | Clearance CL (mL/hr/kg) |
|---|---|---|---|---|---|---|
| Example 1 | Compound 1 | Oral gavage 3 mg/kg | 719.73 | 11.26 | 8317.76 | 281.58 |
| Example 10 | Compound 32-P1 | Oral gavage 3 mg/kg | 1285.10 | 13.21 | 17617.73 | 124.49 |
| Example 11 | Compound 33-P1 | Oral gavage 3 mg/kg | 1235.23 | 11.21 | 10737.25 | 229.69 |
| Example 14 | Compound 36-P1 | Oral gavage 3 mg/kg | 666.67 | 8.29 | 4501.70 | 615.71 |
| Example 17 | Compound 39-P1 | Oral gavage 3 mg/kg | 1279.37 | 9.44 | 13340.26 | 177.16 |
| Example 20 | Comparative Example 1 | Oral gavage 3 mg/kg | 713.73 | 3.38 | 3661.44 | 812.92 |

Conclusion: Compared to the comparative example, the compounds of the present disclosure exhibit significantly superior pharmacokinetic properties in single-dose studies in rats. Furthermore, the compounds of the present disclosure also exhibit significantly superior pharmacokinetic properties in other species, such as dogs.

The embodiments of the technical solutions of the present disclosure have been illustratively described above. It should be understood that the scope of protection of the present disclosure is not limited to the embodiments described above. Any modifications, equivalent substitutions, or improvements made by those skilled in the art within the spirit and principles of the present disclosure shall fall within the scope of protection of the claims of the present disclosure.

## Claims

1. A compound represented by formula (IE) or a pharmaceutically acceptable salt thereof, 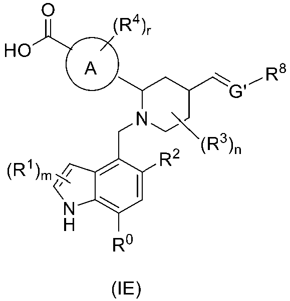 wherein
ring A is selected from the group consisting of phenyl, 5- to 10-membered heteroaryl, and 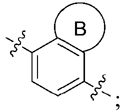
ring B is selected from the group consisting of 3- to 8-membered cycloalkyl, 3- to 8-membered heterocycloalkyl, phenyl, and 5- to 6-membered heteroaryl;
G' is CR⁷ or N;
R⁰ is selected from the group consisting of H, halogen, CN, C₁₋₆ alkyl, C₁₋₆ haloalkyl, C₁₋₆ deuteroalkyl, C₁₋₆ hydroxyalkyl, and 3- to 8-membered cycloalkyl;
each R¹ is the same or different, and independently selected from the group consisting of H, D, halogen, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
R² is selected from the group consisting of H, D, halogen, CN, C₁₋₆ alkyl, C₁₋₆ alkoxy, C₁₋₆ deuteroalkoxy, C₂₋₆ alkynyl, and 3- to 8-membered cycloalkyl;
each R³ is the same or different, and independently selected from the group consisting of H, D, halogen, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
each R⁴ is the same or different, and independently selected from the group consisting of H, halogen, OH, C₁₋₆ alkyl, C₁₋₆ haloalkyl, C₁₋₆ alkoxy, C₁₋₆ haloalkoxy, C₂₋₆ alkynyl, and 3- to 8-membered cycloalkyl;
R⁷ and R⁸ are the same or different, and each independently selected from the group consisting of H, halogen, CN, C₁₋₆ alkyl, C₁₋₆ haloalkyl, C₁₋₆ alkoxy, and C₁₋₆ haloalkoxy;
alternatively, R⁷ and R⁸ together with the carbon atom to which they are attached form a 3- to 8-membered cycloalkyl or 3- to 8-membered heterocycloalkyl ring, wherein the 3- to 8-membered cycloalkyl or 3- to 8-membered heterocycloalkyl is optionally substituted by one or more substituents selected from the group consisting of halogen, C₁₋₆ alkyl, C₁₋₆ haloalkyl, C₁₋₆ alkoxy, and C₁₋₆ haloalkoxy;
m is 0, 1, or 2;
n is 0, 1, 2, or 3;
r is 0, 1, 2, 3, or 4.

2. The compound or the pharmaceutically acceptable salt thereof according to claim 1, wherein 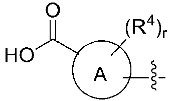 is selected from the group consisting of: 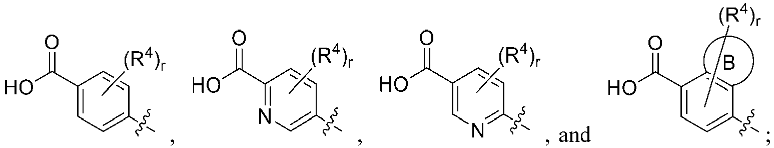 ring B, R⁴, and r are as defined in claim 1;
preferably, ring B is selected from the group consisting of 5- to 6-membered cycloalkyl, 5- to 6-membered heterocycloalkyl, phenyl, and 6-membered heteroaryl;
preferably, ring B is selected from the group consisting of phenyl, pyridinyl, cyclopentenyl, and dihydrofuranyl.

3. The compound or the pharmaceutically acceptable salt thereof according to claim 1 or 2, which is a compound represented by formula (IIG) or a pharmaceutically acceptable salt thereof: 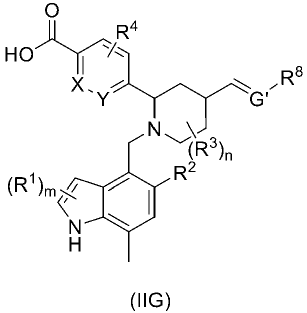 wherein
X and Y are the same or different, and each independently selected from the group consisting of CH and N;
G', R¹, R², R³, R⁴, R⁸, m, and n are as defined in claim 1 or 2;
preferably,
R⁷ and R⁸ are the same or different, and each independently selected from the group consisting of H, halogen, CN, C₁₋₆ alkyl, C₁₋₆ haloalkyl, C₁₋₆ alkoxy, and C₁₋₆ haloalkoxy;
each R¹ is the same or different, and independently selected from the group consisting of H, D, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
R² is selected from the group consisting of C₁₋₆ alkyl, C₁₋₆ alkoxy, C₁₋₆ deuteroalkoxy, and 3- to 8-membered cycloalkyl;
each R³ is the same or different, and independently selected from the group consisting of H, D, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
each R⁴ is the same or different, and independently selected from the group consisting of H, halogen, C₁₋₆ alkyl, C₁₋₆ haloalkyl, C₁₋₆ alkoxy, and C₁₋₆ haloalkoxy.

4. A compound represented by formula (I) or formula (III) or a pharmaceutically acceptable salt thereof, 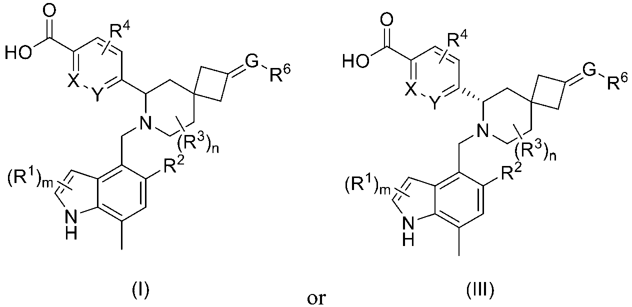 wherein
X and Y are the same or different, and each independently selected from the group consisting of CH and N;
G is CR⁵ or N;
R⁵ and R⁶ are the same or different, and each independently selected from the group consisting of H, halogen, CN, C₁₋₆ alkyl, C₁₋₆ haloalkyl, C₁₋₆ alkoxy, and C₁₋₆ haloalkoxy;
each R³ is the same or different, and independently selected from the group consisting of H, D, CN, NH₂, OH, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, two R³ together with the carbon atom to which they are attached form a C₃₋₈ cycloalkyl or 3- to 8-membered heterocycloalkyl ring, wherein the C₃₋₈ cycloalkyl or 3- to 8-membered heterocycloalkyl is optionally substituted by halogen, C₁₋₆ alkyl, C₁₋₆ haloalkyl, C₁₋₆ alkoxy, and C₁₋₆ haloalkoxy;
R¹, R², R⁴, m, and n are as defined in any one of claims 1 to 3.

5. The compound or the pharmaceutically acceptable salt thereof according to claim 4, which is a compound represented by formula (III-1) or formula (III-2) or a pharmaceutically acceptable salt thereof: 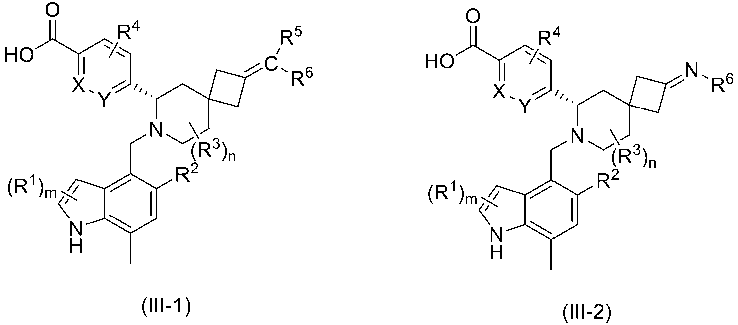
wherein X, Y, R¹, R², R³, R⁴, R⁵, R⁶, m, and n are as defined in claim 4;
preferably, R⁵ and R⁶ are the same or different, and each independently selected from the group consisting of H, halogen, CN, C₁₋₆ alkyl, and C₁₋₆ haloalkyl; preferably, R⁵ and R⁶ are the same or different, and each independently selected from the group consisting of H, F, CN, and methyl;
preferably, R⁶ is C₁₋₆ alkoxy.

6. The compound or the pharmaceutically acceptable salt thereof according to any one of claims 1 to 3, which is a compound represented by formula (IVG) or a pharmaceutically acceptable salt thereof: 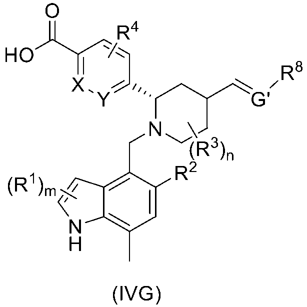 wherein X, Y, G', R¹, R², R³, R⁴, R⁸, m, and n are as defined in any one of claims 1 to 3.

7. The compound or the pharmaceutically acceptable salt thereof according to any one of claims 1 to 3 or 6, which is a compound represented by formula (IVG-1) or formula (IVG-2) or a pharmaceutically acceptable salt thereof: 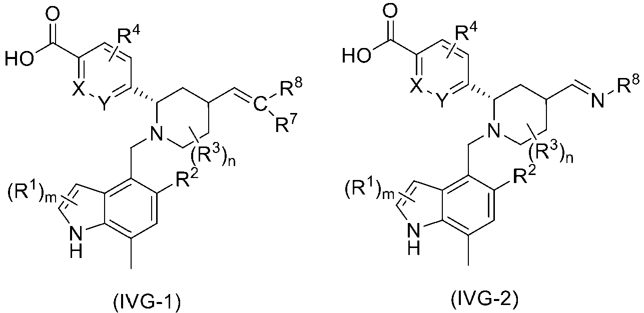 wherein X, Y, R¹, R², R³, R⁴, R⁷, R⁸, m, and n are as defined in any one of claims 1 to 3 or 6.

8. The compound or the pharmaceutically acceptable salt thereof according to any one of claims 1 to 3, 6, or 7, which is a compound represented by formula (IVG-3) or a pharmaceutically acceptable salt thereof: 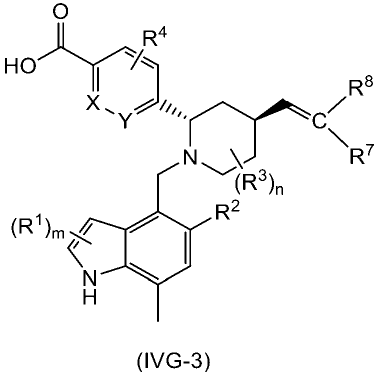 wherein X, Y, R¹, R², R³, R⁴, R⁷, R⁸, m, and n are as defined in any one of claims 1 to 3, 6, or 7.

9. The compound or the pharmaceutically acceptable salt thereof according to any one of claims 1 to 3 or 6 to 8, wherein R⁷ and R⁸ are the same or different, and each independently selected from the group consisting of H, halogen, CN, C₁₋₆ alkyl, and C₁₋₆ haloalkyl; preferably, R⁷ and R⁸ are the same or different, and each independently selected from the group consisting of H, F, CN, methyl, and -CHF₂;
alternatively, R⁷ and R⁸ together with the carbon atom to which they are attached form a 3- to 6-membered cycloalkyl ring;
preferably, R⁷ and R⁸ together with the carbon atom to which they are attached form a cyclopropyl ring.

10. The compound or the pharmaceutically acceptable salt thereof according to any one of claims 1 to 9, wherein both X and Y are CH;
alternatively, X is N and Y is CH;
and/or, R² is selected from the group consisting of methyl, methoxy, deuteromethoxy, cyclopropyl, halogen, and ethynyl; preferably, R² is selected from the group consisting of methyl, methoxy, deuteromethoxy, and cyclopropyl; preferably, R² is methoxy;
and/or, R⁴ is selected from the group consisting of H, OH, halogen, C₁₋₆ alkyl, C₁₋₆ haloalkyl, C₁₋₆ alkoxy, C₂₋₆ alkynyl, and 3- to 8-membered cycloalkyl; preferably, R⁴ is H, OH, halogen, C₁₋₆ alkyl, or C₁₋₆ alkoxy.

11. The compound or the pharmaceutically acceptable salt thereof according to any one of claims 1 to 10, selected from the group consisting of the following compounds: 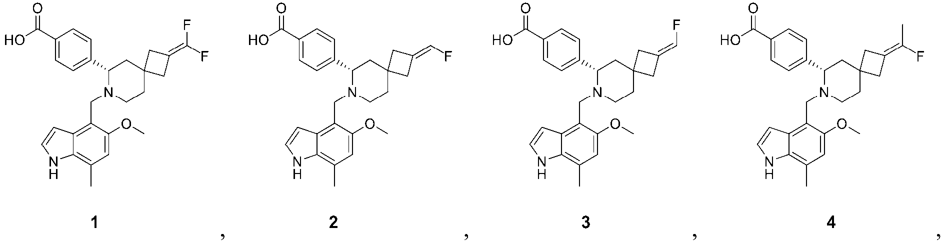 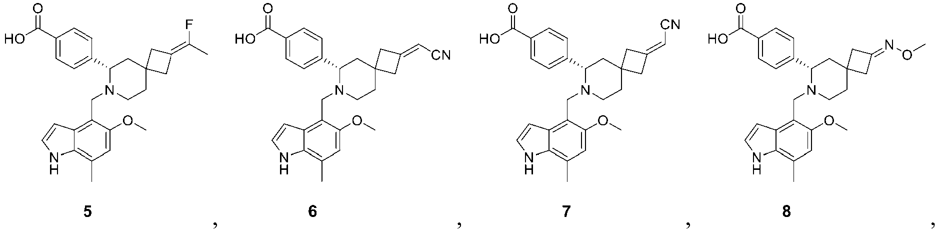 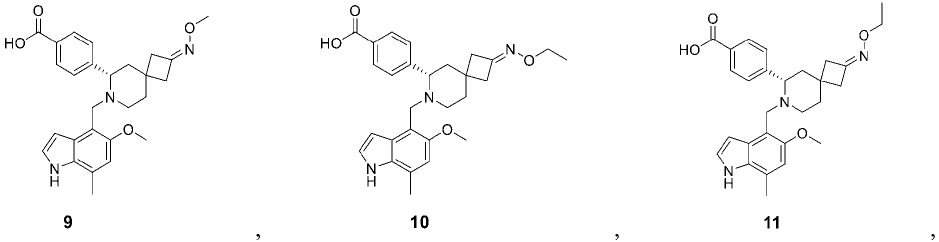 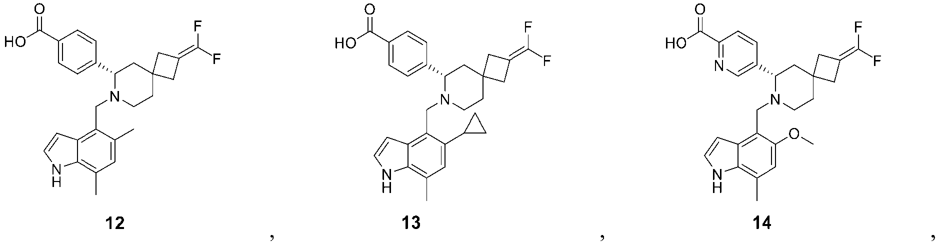 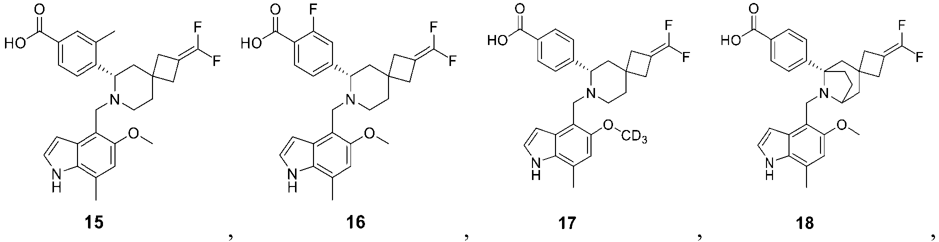 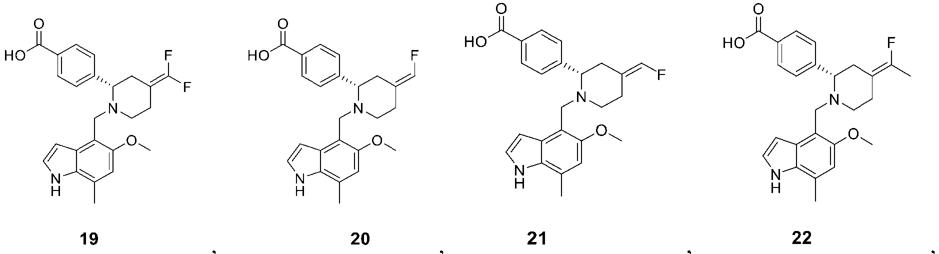 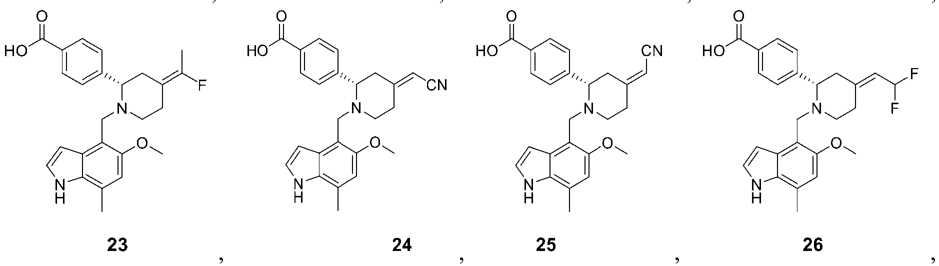 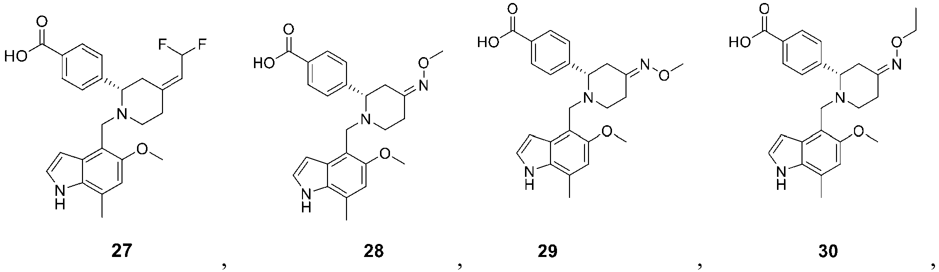 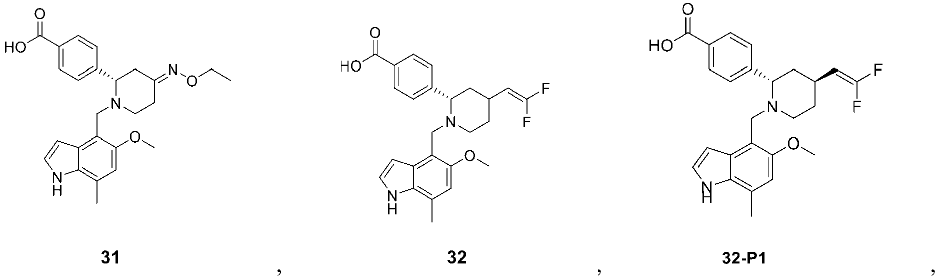 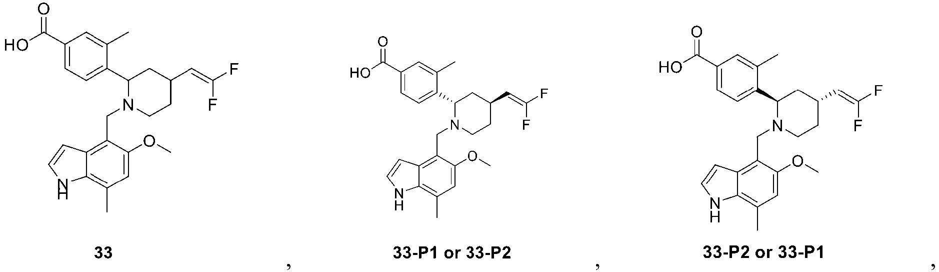 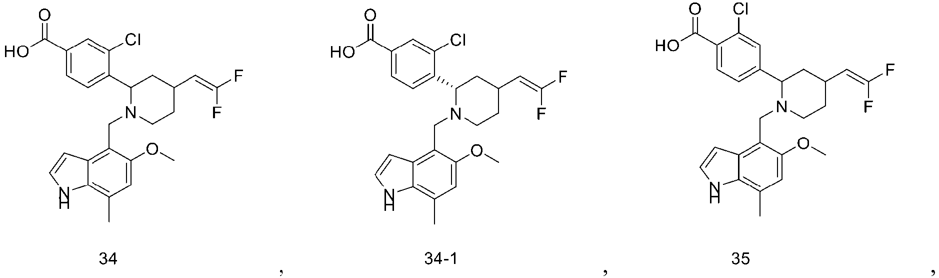 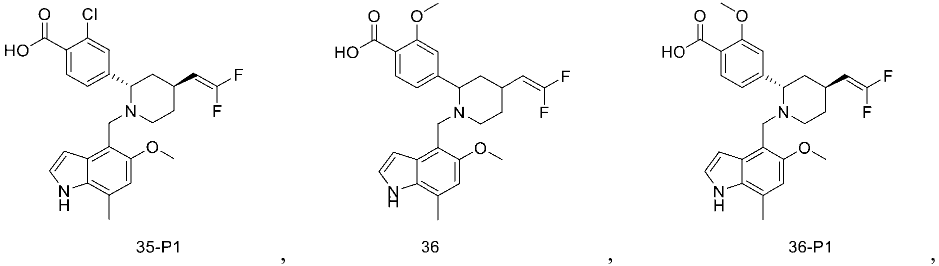 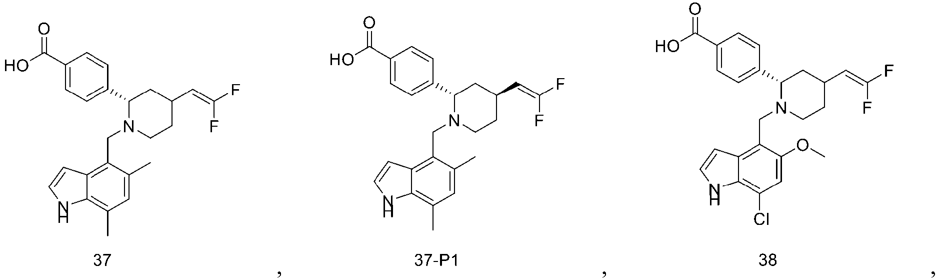 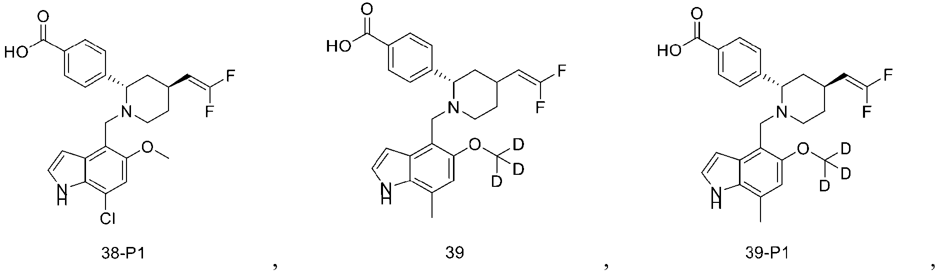 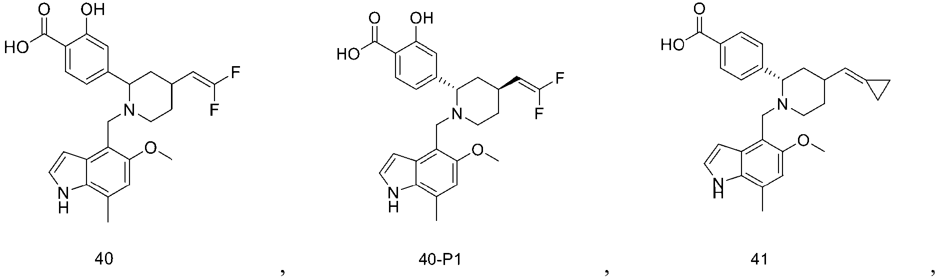 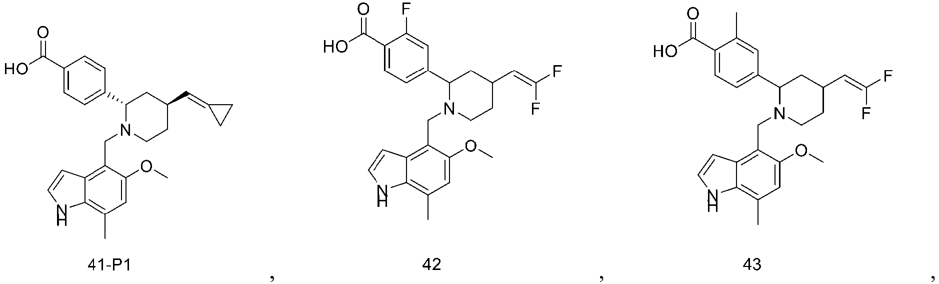 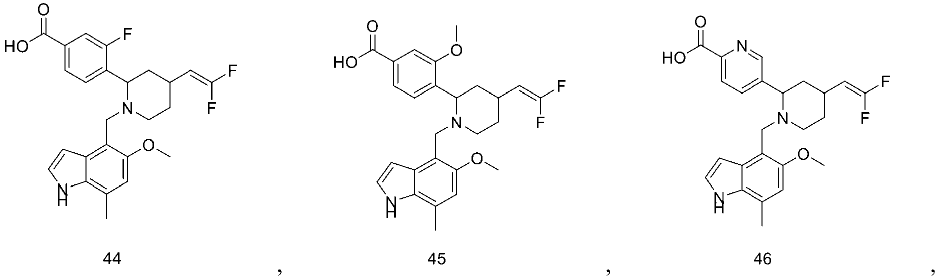 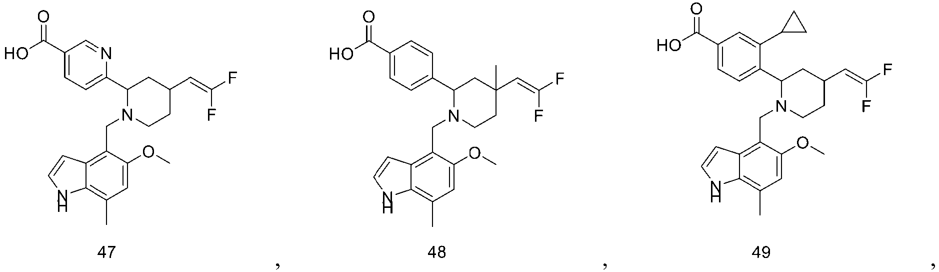 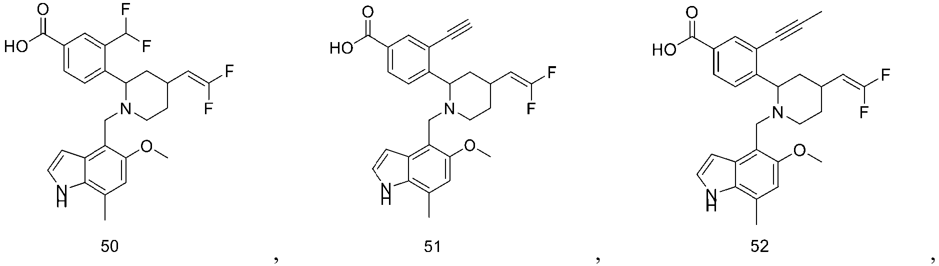 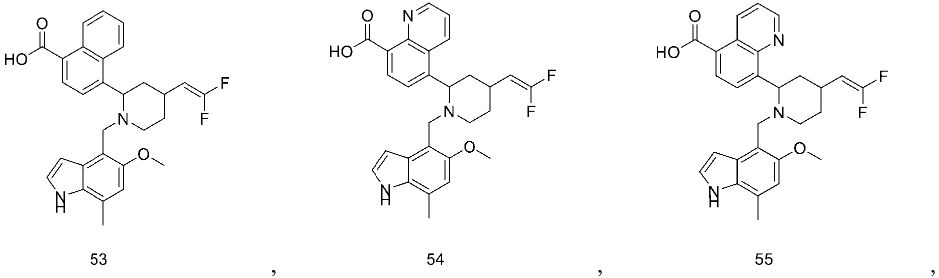 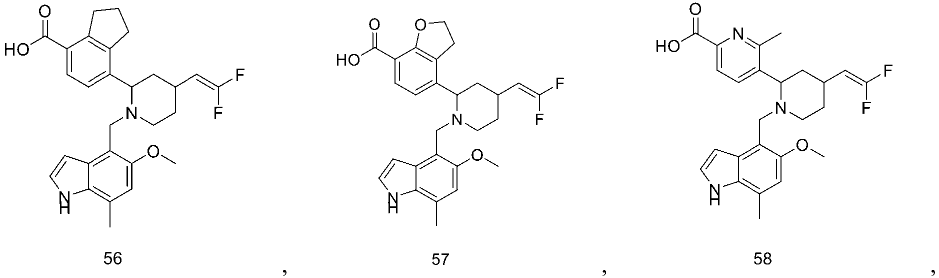 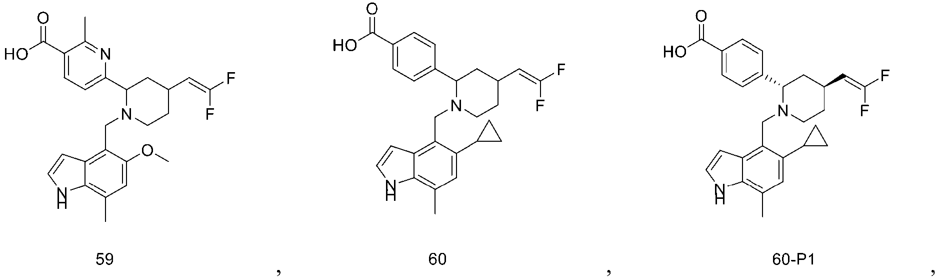 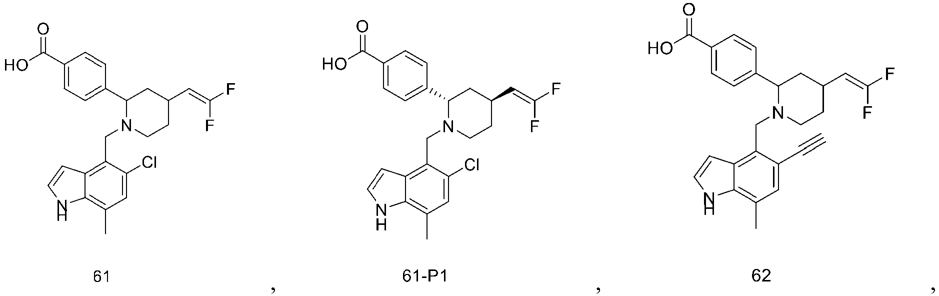 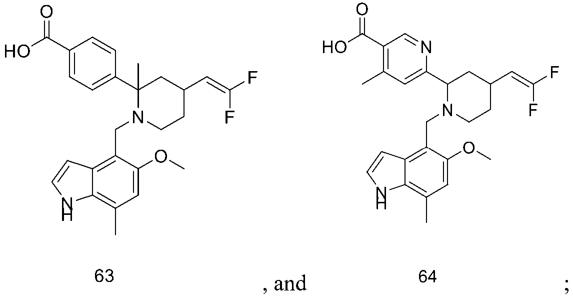 preferably, the compound or the pharmaceutically acceptable salt thereof is selected from the group consisting of the following compounds: 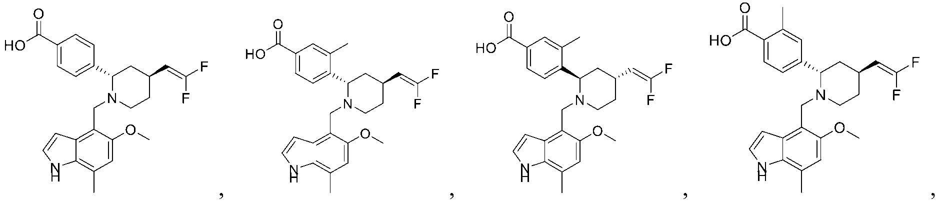 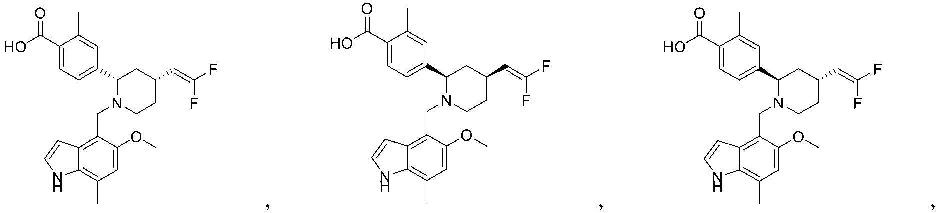 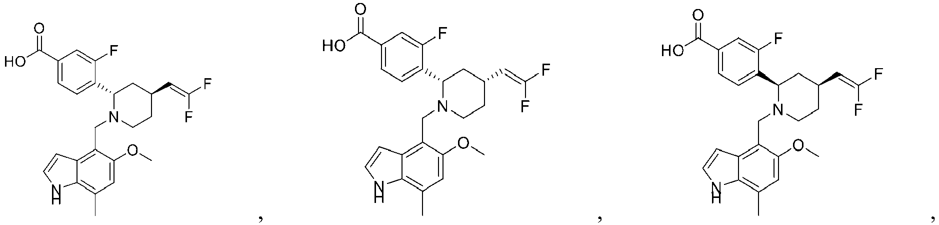 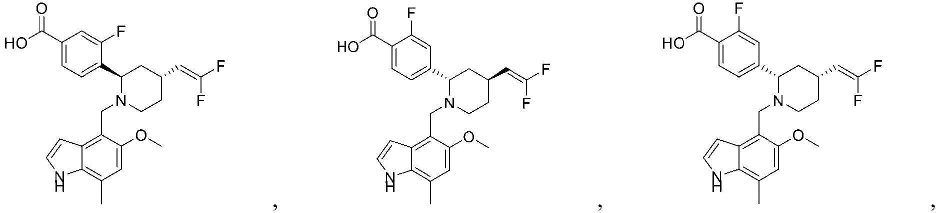 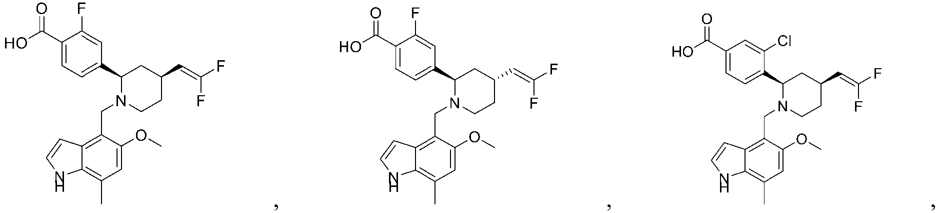 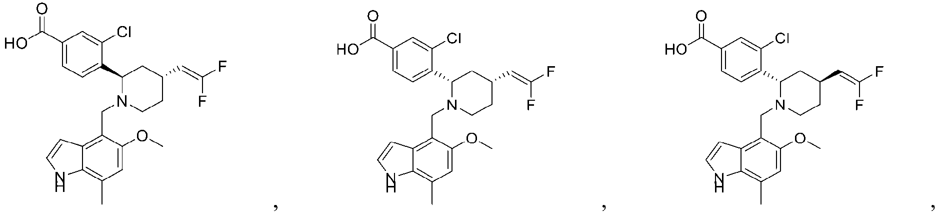 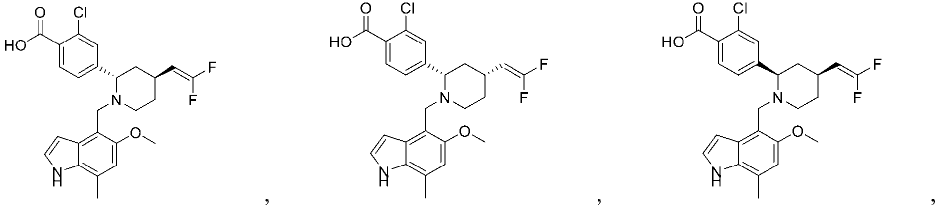 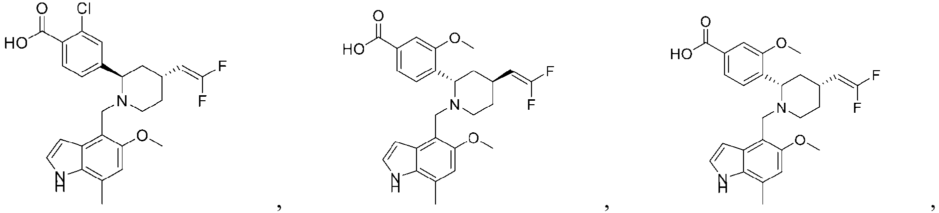 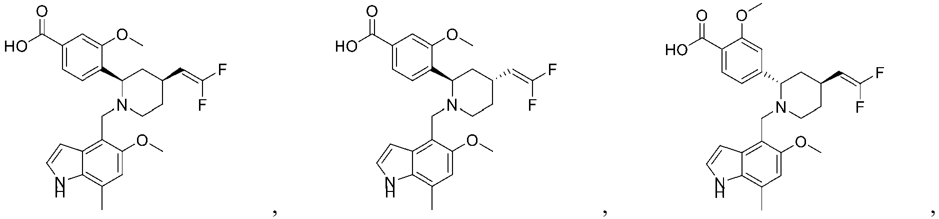 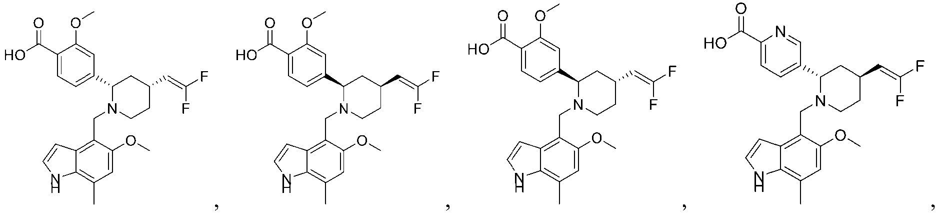 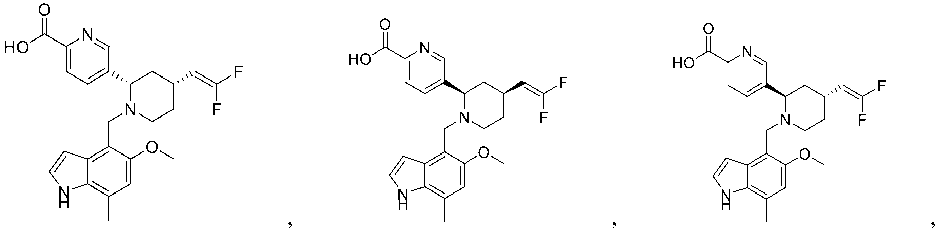 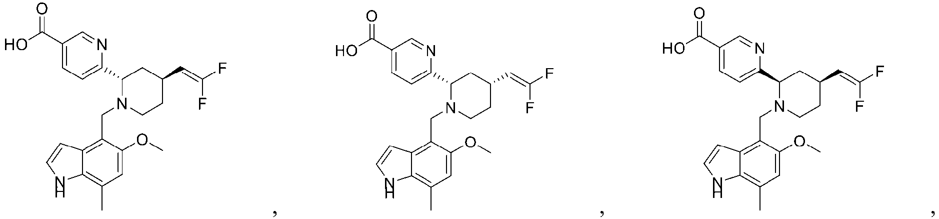 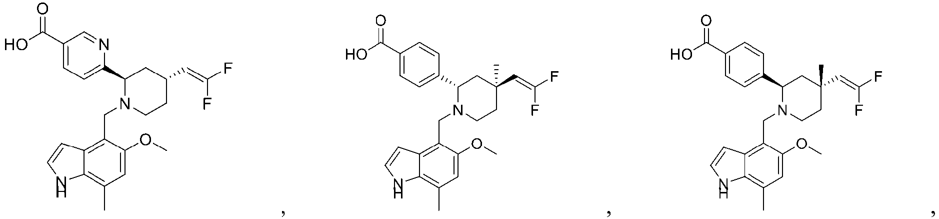 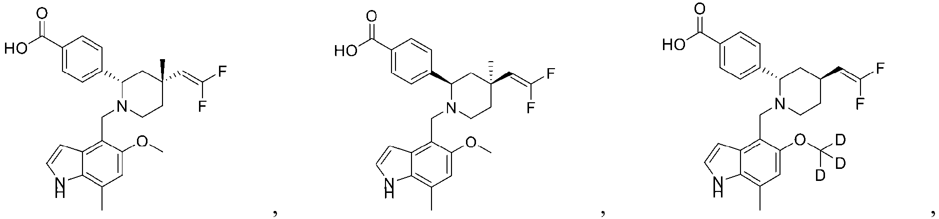 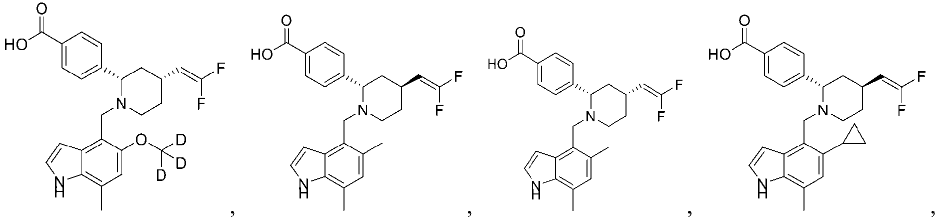 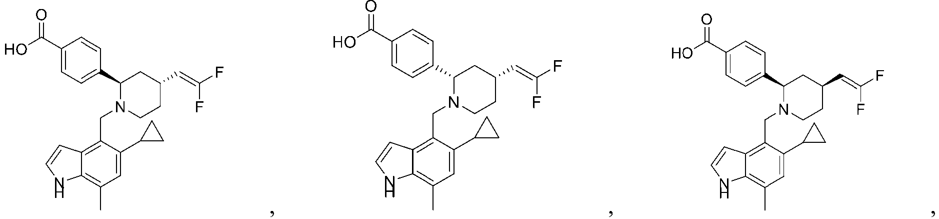 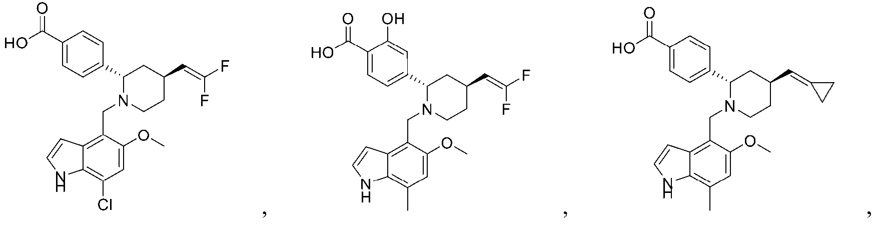 and

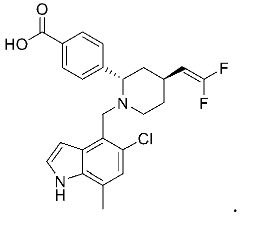

12. A pharmaceutical composition comprising at least one therapeutically effective amount of the compound or the pharmaceutically acceptable salt thereof as defined in any one of claims 1 to 11, and one or more pharmaceutically acceptable excipients.

13. Use of the compound or the pharmaceutically acceptable salt thereof as defined in any one of claims 1 to 11, or the pharmaceutical composition as defined in claim 12, in the manufacture of a medicament for modulating the activity of an alternative complement pathway in a subject; preferably, in the manufacture of a medicament for preventing and/or treating a disease or condition affected by modulation of the alternative complement pathway;
preferably, in the manufacture of a medicament for inhibiting complement factor B; preferably, in the manufacture of a medicament for preventing and/or treating a disease mediated by complement factor B;
preferably, in the manufacture of a medicament for preventing and/or treating an autoimmune disease, an inflammatory disease, a cardiovascular disease, or a respiratory disease; preferably, in the manufacture of a medicament for preventing and/or treating paroxysmal nocturnal hemoglobinuria (PNH), primary glomerulonephritis (IgAN), membranous nephropathy (MN), diabetic kidney disease (DKD), C3 glomerulonephritis (C3G), lupus nephritis (LN), proliferative nephritis, systemic lupus erythematosus (SLE), age-related macular degeneration (AMD), geographic atrophy (GA), atypical hemolytic uremic syndrome (aHUS), hemolytic uremic syndrome (HUS), diabetic retinopathy (DR), complications of hemodialysis, hemolytic anemia or hemodialysis, neuromyelitis optica (NMO), arthritis, rheumatoid arthritis, psoriatic arthritis, liver inflammation, dermatomyositis and amyotrophic lateral sclerosis, myasthenia gravis (MG), Guillain-Barré syndrome (GBS), neuromyelitis optica spectrum disorder (NMOSD), uveitis, retinitis pigmentosa, macular edema, Behçet's uveitis, multifocal choroiditis, Vogt-Koyanagi-Harada syndrome, intermediate uveitis, birdshot retinochoroiditis, sympathetic ophthalmia, ocular pemphigoid, ocular pemphigus, non-arteritic ischemic optic neuropathy, postoperative inflammation, retinal vein occlusion, glaucoma, Doyne honeycomb retinal dystrophy/Malattia Leventinese, Sorsby's fundus dystrophy, late-onset retinal macular dystrophy, North Carolina macular dystrophy, Stargardt disease, keratitis, anti-neutrophil cytoplasmic antibody-associated vasculitis (ANCA-AAV), Crohn's disease, adult respiratory distress syndrome, myocarditis, post-ischemia-reperfusion injury, myocardial infarction, balloon angioplasty, post-pump syndrome following cardiopulmonary bypass or renal bypass, atherosclerosis, hemodialysis, renal ischemia, acute kidney injury, mesenteric artery reperfusion after aortic reconstruction, and other diseases.

14. A compound represented by formula (IEA) or a salt thereof, 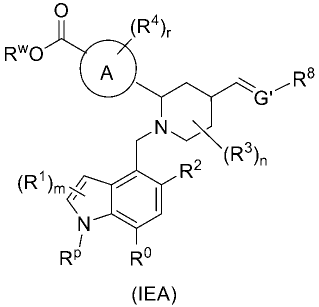
wherein R^{w} is C₁₋₆ alkyl;
R^{p} is an amino protecting group, preferably benzyl, tert-butoxycarbonyl (Boc), 2-biphenyl-2-propoxycarbonyl, benzyloxycarbonyl, fluorenylmethoxycarbonyl (Fmoc), or trifluoroacetyl;
ring A, G', R⁰, R¹, R², R³, R⁴, R⁸, r, m, and n are as defined in formula (IE);
preferably, the compound represented by formula (IEA) or the salt thereof is selected from the group consisting of the following compounds:

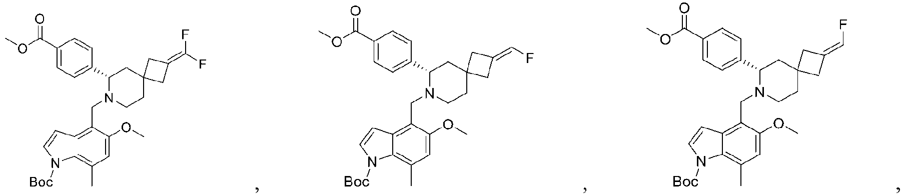

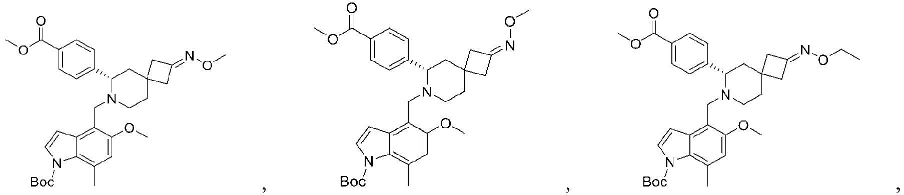

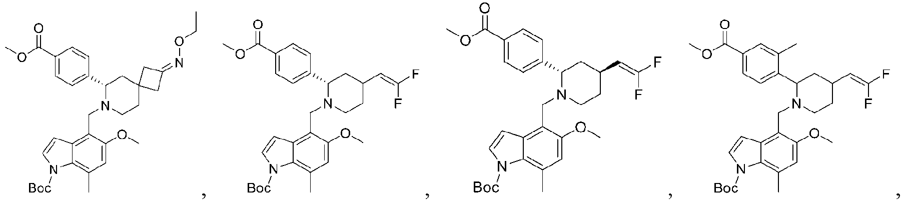

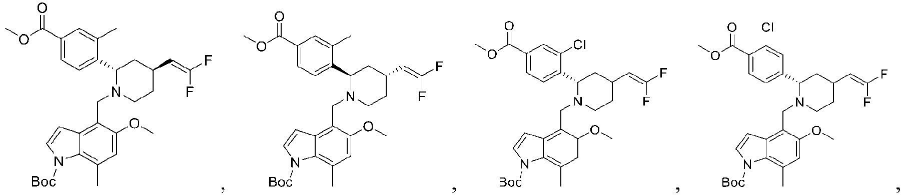

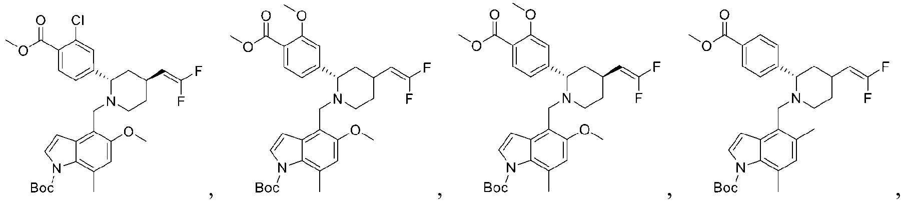

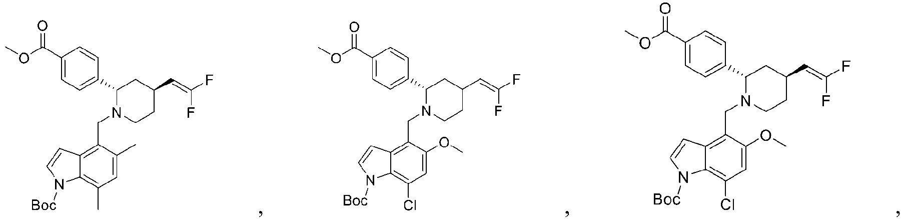

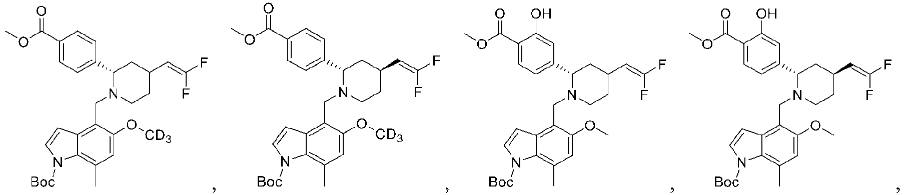

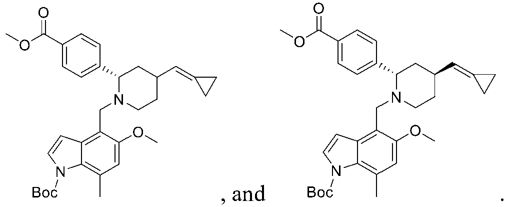

15. A method of preparing the compound represented by formula (IE) as defined in claim 1, comprising subjecting the compound represented by formula (IEA) as defined in claim 14 to hydrolysis and deprotection reactions to obtain the compound represented by formula (IE), 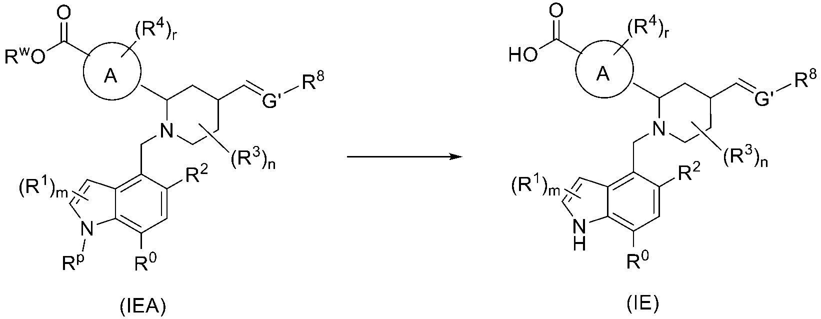
wherein R^{w} is C₁₋₆ alkyl;
R^{p} is an amino protecting group, preferably benzyl, tert-butoxycarbonyl (Boc), 2-biphenyl-2-propoxycarbonyl, benzyloxycarbonyl, fluorenylmethoxycarbonyl (Fmoc), or trifluoroacetyl;
ring A, G', R⁰, R¹, R², R³, R⁴, R⁸, r, m, and n are as defined in claim 1.
